# EUROPEAN PATENT APPLICATION

(11) **EP 2 199 282 A1**
(43) Date of publication of application: **23.06.2010**
(21) Application number: 08836955.8
(22) Date of filing: 09.10.2008
(51) Int. Cl.: C07D 213/75, A61K 31/4545, A61K 31/496, A61K 31/497, A61K 31/501, A61K 31/506, A61P 25/04, A61P 25/24, A61P 43/00, C07D 237/20, C07D 241/20, C07D 261/14, C07D 295/18, C07D 401/12, C07D 403/12, C07D 413/12

(54) **AMIDE COMPOUND**

(30) Priority: 10.10.2007 JP 2007264381
(71) Applicant: Takeda Pharmaceutical Company Limited, Osaka- shi, Osaka 541-0045 (JP)
(72) Inventor: KORI, Masakuni, Osaka-shi Osaka 532-8686 (JP); KOUNO, Mitsunori, Osaka-shi Osaka 532-8686 (JP)
(74) Representative: Jones, Nicholas Andrew
(86) International application number: PCT/JP2008/068369
(87) International publication number: WO 2009/048101

(57) **Abstract**

An object of the present invention is to provide a novel fused-ring compound which has a FAAH inhibitory effect and is useful as an analgesic.

The present invention relates to a compound represented by formula (I): 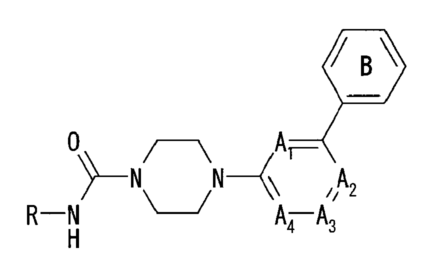 wherein symbols are as defined in the specification, or salt thereof.

## Description

### Technical Field

The present invention relates to a novel amide compound having a FAAH inhibitory effect.

### Background Art

Pain is disease which is serious for patients, lowers QOL, and also leads to difficulty in social life. Pain is classified into inflammatory pain, neuropathic pain, nociceptive pain, and psychogenic pain, etc. according to the cause. Inflammatory pain is pain associated with an inflammation being caused by nociceptive mechanical stimulus, heat stimulus or chemical stimulus arising from in vitro. It is known that not only inflammation site but also inflammatory cytokines and cyclooxygenase in spinal cord play an important role with respect to expression of inflammatory pain. Neuropathic pain is pathological pain generated by dysfunction of a peripheral or central nervous system itself. Nociceptive pain is pain generated when normal tissues are damaged or nociceptive stimulus as a causative is applied, and is classified into somatic pain and visceral pain.

A cyclooxygenase (COX) inhibitor such as indomethacin, a cyclooxygenase II (COX-II) inhibitor such as celecoxib, a central analgesic such as tramadol, and an antipyretic analgesic such as acetaminophen are used as a therapeutic agent for inflammatory pain. However, when the cyclooxygenase inhibitor is used for a long period of time, there is a problem that side effects such as gastrointestinal disturbance are caused. It is also reported that the cyclooxygenase II inhibitor causes gastric ulcer, and recently, side effects of a cardiocirculatory system such as myocardial infarction and cerebral infarction are also a problem.

An opioidic analgetic such as morphine and an anticonvulsant such as gabapentin or pregabalin are used as a therapeutic agent for neuropathic pain, but it is known that they can be required an increase in amount by long-term use and that they cause side effect such as sedation, and a agent which can be used without causing side effects and safely is not available yet.

Meanwhile, cannabinoid receptors have been identified since 1990's as receptors for Δ9-tetrahydrocannabinol (Δ9-THC), which is an active material obtained from the hemp plant. At present, the CB1 receptor (see Nature, Vol. 346, p. 561 (1990)), its splice variant CB1a (see J. Biol. Chem., Vol. 270, p. 3726 (1995)), and the CB2 receptor (see Eur. J. Biochem., Vol. 232, p. 54 (1995)) are known. Almost around the same time, N-arachidonoylethanolamine (anandamide) was found in the brain of a pig as an endogenous ligand for the CB1 receptor (see Science, Vol. 258, p. 1946 (1992)). Anandamide belongs to the family of N-acylated ethanolamine, as does N-palmitoylethanolamine or N-oleoylethanolamine. Fatty acid amides including these N-acylated ethanolamines are found to have effect on physiological functions such as pain (see Nature, Vol. 394, p. 277 (1998); and Pain, Vol. 76, p. 189 (1998)), dietary regulation (see Nature, Vol. 414, p. 209 (2001)) and promotion of sleep (see Science, Vol. 268, p. 1506 (1995)). The route for biosynthesis or decomposition of fatty acid amides has been investigated since 1980's. First, a calcium-dependent transacylase produces anandamide, which is N-acylphosphatidylethanolamine (see J. Neurochem., Vol. 41, p. 1303 (1983)), and then a fatty acid amide is released therefrom by the action of phospholipase D (see J. Neurochem., Vol. 42, p. 1613 (1984)). The existence of an enzymatic activity which hydrolyzes a fatty acid amide into the corresponding fatty acid, thereby eliminating its physiological activity, was suggested earlier but was confirmed only in the later half of 1990's. An active material hydrolyzing oleamide was isolated from a rat, and its cDNA was cloned (see Nature, Vol. 384, p. 83 (1996)). The enzyme produced by genetic recombination of the cDNA was able to hydrolyze various fatty acid amides including oleamide and anandamide, and was named as fatty acid amide hydrolase (hereinafter, sometimes abbreviated to "FAAH" in the present specification). Still, it is not sufficiently clear about the enzyme responsible for biosynthesis of fatty acid amides. However, the fact that fatty acid amides are produced from neuronal cells in a calcium-dependent, that is, neuronal activity-dependent manner (see Nature, Vol. 372, p. 686 (1994)) is highly meaningful for development of a therapeutic agent. Furthermore, an FAAH knockout mouse has been produced, and an FAAH inhibitory agent has been discovered, so that the physiological significance of FAAH inhibition is being revealed. In the FAAH knockout mouse, the content of fatty acid amides, including anandamide, in the brain increased by 10 to 15 times, but the mobility, body weight and body temperature of the mouse were normal. However, a decrease in the responsiveness to pain was observed, and this was correlated with the content of fatty acid amides in the brain (see Proc. Natl. Acad. Sci. USA, Vol. 98, p. 9371 (2001)). For the FAAH inhibitor, trifluoromethyl ketone derivatives (see J. Am. Chem. Soc., 118, 5938 (1996)), alpha-keto heterocyclic ring derivatives (see Proc. Natl. Acad. Sci. USA, Vol. 97, p. 5044 (2000)), sulfonylfluoride derivatives (see Biochem. Biophys. Res. Commun., Vol. 231, p. 217 (1997)), fluorophosphonate derivatives (see Biochem. Pharmacol., Vol. 53, p. 255 (1997)), and arylcarbamate derivatives (see Nat. Med., Vol. 9, p. 76 (2003)) are known.

In addition to this, FAAH or anandamide has been reported to be involved with various diseases. We have found that a FAAH inhibitor has a cerebro-neuroprotective effect and is useful as a therapeutic agent for cerebrovascular disorder. Also, it has been reported that large quantities of FAAH are found in the brain of Alzheimer's patients (see The Journal of Neuroscience, Vol. 23, p. 1136 (2003)). It has been also discovered by a test using rats that an increase in the amount of anandamide results in an antiparkinsonian activity (see Neuropsychopharmacology, Vol. 29, p. 1134 (2004)). It has also been reported that women having miscarriage show decreased levels of FAAH (see J. Clin. Endocrinol. Metab., 89, 5168 (2004)). Anandamide is reported to inhibit propagation of rectal cancer (see Gastroenterology, Vol. 125, p. 677 (2003)). It is reported that an FAAH knockout mouse is not susceptible to colonitis or colitis (see J. Clin. Invest., Vol. 113, p. 1202 (2004)). An FAAH inhibiting drug is reported to exhibit an anxiolytic effect (see Nature Medicine, Vol. 9, p. 76 (2003)). FAAH is reported to be a hydrolytic enzyme for oleylethanolamide, which is a satiety factor present in the small intestine (see Nature, Vol. 414, p. 209 (2001)). FAAH is a hydrolytic enzyme for stearoylethanolamide, and it is reported that administration of stearoylethanolamide to a mouse suppresses eating (see FASEB Journal, Vol. 18, p. 1580 (2004)). Since anandamide is an agonist of the vanilloid receptor, which is a nociceptor, the FAAH inhibitory agent is expected to have the same activity as that of the vanilloid receptor agonist (for example, prophylactic and/or therapeutic activity for frequent urination, urinary incontinence, interstitial cystitis) (see JP 2002-202204 A).

Since FAAH is an enzyme which hydrolyzes an endogenous sleep substance, oleamide, a FAAH inhibitor suppresses the decomposition of oleamide to induce sleep (US 2003/0092734 A).

International Publication No. WO 2007/020888 describes, as an amide compound having a FAAH inhibitory activity, a compound represented by the following formula: 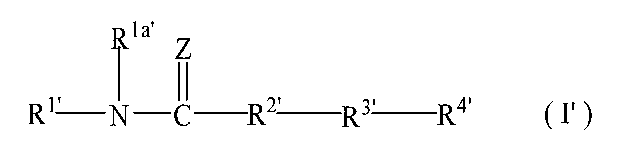 wherein Z is oxygen or sulfur; R^{1'} is an optionally substituted aryl or an optionally substituted heterocyclic group; R^{1a'} is a hydrogen atom, an optionally substituted hydrocarbon group, a hydroxy, an optionally substituted alkoxy, an optionally substituted aryloxy, an optionally substituted amino, or an optionally substituted 5- to 7-membered saturated cyclic amino; R^{2'} is an optionally substituted piperidine-1,4-diyl or an optionally substituted piperazine-1,4-diyl; R^{3'} is a divalent group formed by eliminating two hydrogen atoms from a benzene ring which may be further substituted or a 6-membered aromatic heterocycle containing 1 to 4 heteroatoms selected from oxygen, sulfur and nitrogen atoms in addition to carbon atoms which may be further substituted; and R^{4'} is a group formed by eliminating one hydrogen atom from an optionally substituted benzene ring or an optionally substituted 5- to 6-membered heterocycle containing 1 to 4 heteroatoms selected from oxygen, sulfur and nitrogen atoms in addition to carbon atoms, or a salt thereof.

### Disclosure of the Invention

### Problem to be Solved by the Invention

Currently, nonsteroidal anti-inflammatory drugs (NSAIDs) and narcotic analgesics, etc. are used as therapeutic agents for inflammatory pain and neuropathic pain, and there is a highly need for developing a more safe therapeutic agent for pain without causing side effects compared with these drugs. An object of the present invention is to provide a safe and excellent prophylactic or therapeutic agent for pain.

The present inventors have studied intensively so as to achieve the above-described object and have found that compounds represented by the following formula (I) or salts thereof (hereinafter, sometimes, referred to as Compound (I)) have a FAAH inhibitory activity and exert an excellent analgesic effect in various pain models, and thus completing the present invention.

That is, the present invention provides:
(1) A compound represented by formula (I): 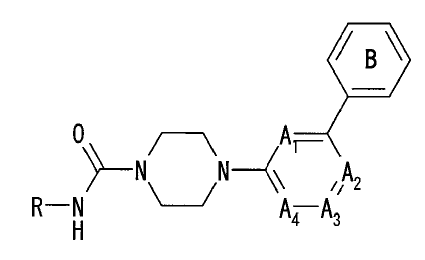 wherein R is an aromatic hydrocarbon or aromatic heterocyclic group each of which may be substituted by one or more substituents (excluding C₁₋₆ alkoxy, phenoxy, carboxyl and tetrazolyl);
   A₁, A₂, A₃ and A₄ are each independently CH or N;
   ring B is a phenyl group which may be substituted by one or more halogen atoms;
   provided that when the moiety represented by formula: 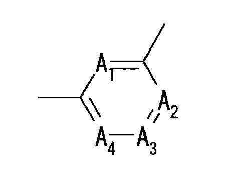 is 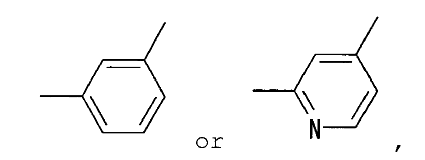 ring B is a phenyl group substituted by one or more halogen atoms, or a salt thereof;
(2) The compound according to (1), wherein R is an aromatic hydrocarbon or aromatic heterocyclic group which may be substituted by one or more substituents selected from halogen and a C₁₋₆ alkyl group which may be halogenated;
(3) The compound according to (1), wherein R is a phenyl or 5- to 10-membered aromatic heterocyclic group each of which may be substituted by one or more C₁₋₆ alkyl groups;
(4) The compound according to (1), wherein the moiety represented by the formula: 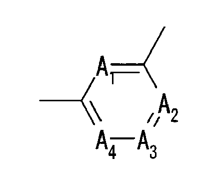 in formula (I) is 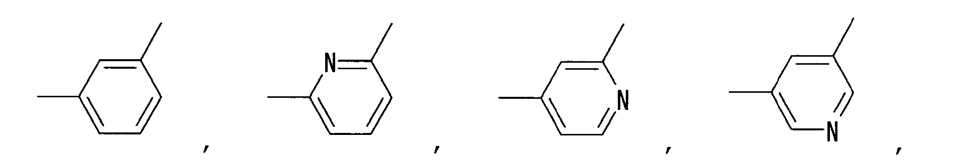 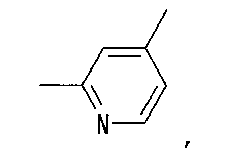 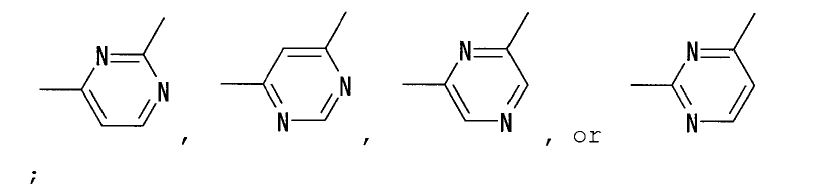
(5) The compound according to (1), wherein ring B is a phenyl group substituted by one or more halogen atoms;
(6) The compound according to (1), wherein R is a phenyl or 5- to 10-membered aromatic heterocyclic group each of which may be substituted by one or more C₁₋₆ alkyl groups, the moiety represented by the formula: 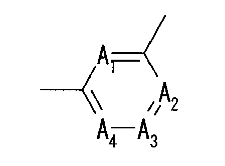 in formula (I) is 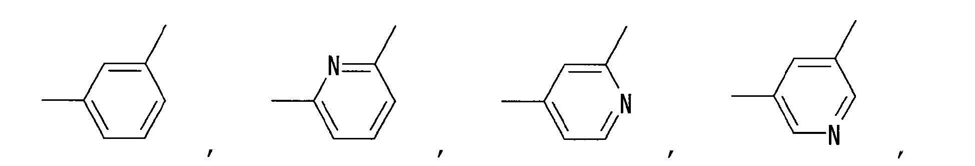 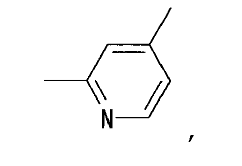 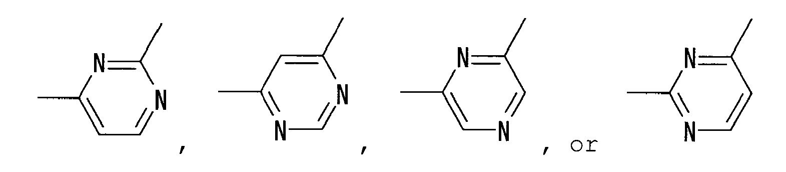 and ring B is a phenyl group substituted by one or more halogen atoms;
(7) The compound according to (1), wherein R is an isoxazolyl, pyridazinyl, pyridinyl, or phenyl group each of which may be substituted by one or more methyl groups, the moiety represented by the formula: 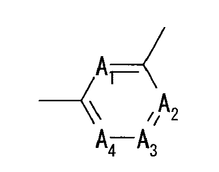 in formula (I) is 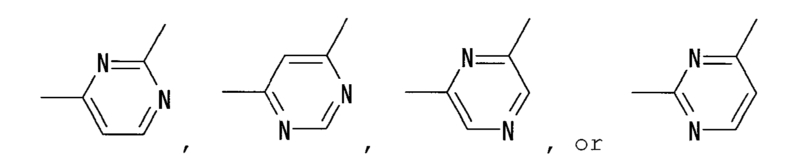 and ring B is a phenyl group substituted by one or more fluorine atoms;
(8) The compound according to (1), wherein R is an isoxazolyl group which may be substituted by one or more methyl groups, the moiety represented by the formula: 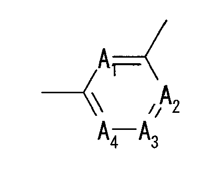 in formula (I) is 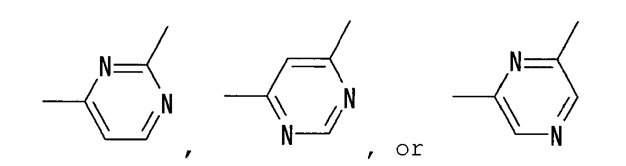 and ring B is a phenyl group substituted by two fluorine atoms;
(9) The compound according to (1), wherein the moiety represented by the formula: 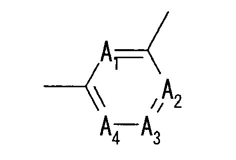 in formula (I) is 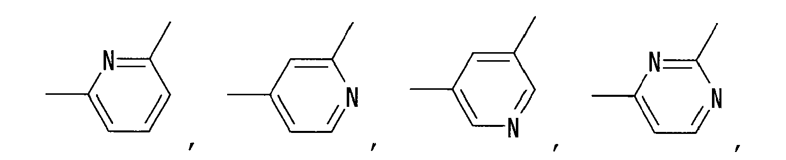 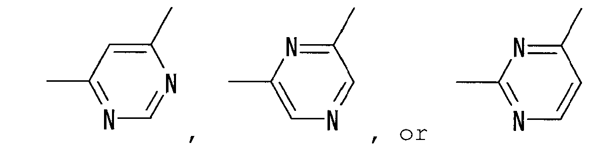 and ring B is a non-substituted phenyl group;
(10) The compound according to (1), wherein R is a phenyl or 5- to 10-membered aromatic heterocyclic group each of which may be substituted by one or more C₁₋₆ alkyl groups, the moiey represented by the formula: 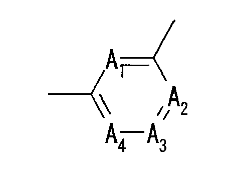 in formula (I) is 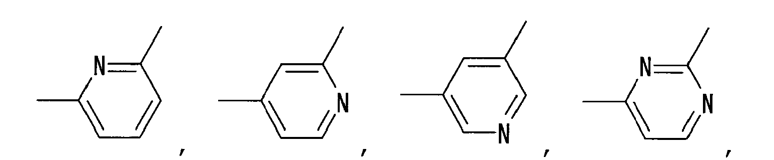 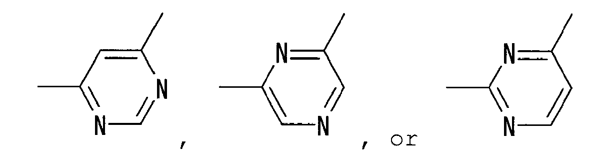 and ring B is a non-substituted phenyl group;
(11) The compound according to (1), wherein R is an isoxazolyl, pyridazinyl, pyridinyl, or phenyl group each of which may be substituted by one or more methyl groups, the moiety represented by the formula: 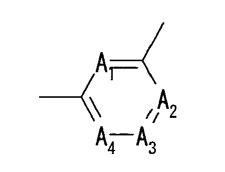 in formula (I) is 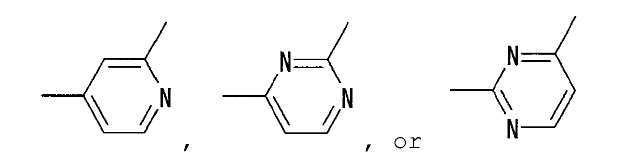 and ring B is a non-substituted phenyl group;
(12) The compound according to (1), wherein R is a pyridazinyl or pyridinyl group, the moiety represented by the formula: 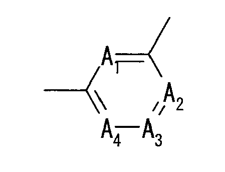 in formula (I) is 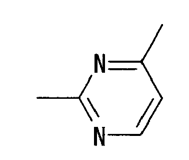 and ring B is a non-substituted phenyl group;
(13) 4-[2-(2,3-difluorophenyl)pyrimidin-4-yl]-N-(3,4-dimethylisoxazol-5-yl)piperazine-1-carboxamide or a salt thereof;
(14) 4-[6-(2,4-difluorophenyl)pyrazin-2-yl]-N-(3,4-dimethylisoxazol-5-yl)piperazine-1-carboxamide or a salt thereof;
(15) 4-[4-(2,4-difluorophenyl)pyrimidin-2-yl]-N-pyridin-3-ylpiperazine-1-carboxamide or a salt thereof;
(16) 4-[4-(2,4-difluorophenyl)pyrimidin-2-yl]-N-pyridazin-3-ylpiperazine-1-carboxamide or a salt thereof;
(17) 4-[6-(2,4-difluorophenyl)pyrimidin-4-yl]-N-(3,4-dimethylisoxazol-5-yl)piperazine-1-carboxamide or a salt thereof;
(18) 4-(4-phenylpyrimidin-2-yl)-N-pyridin-3-ylpiperazine-1-carboxamide or a salt thereof;
(19) 4-(4-phenylpyrimidin-2-yl)-N-pyridazin-3-ylpiperazine-1-carboxamide or a salt thereof;
(20) A prodrug of the compound according to (1);
(21) A medicine comprising the compound according to (1) or the prodrug according to (20);
(22) The medicine according to (21), which is a FAAH inhibitor;
(23) The medicine according to (21), which is a prophylatic or therapeutic agent for anxiety or depression, or an analgesic;
(24) The medicine according to (21), which is a prophylactic or therapeutic agent for inflammatory pain or neuropathic pain;
(25) A FAAH inhibitory method characterized by administering to a mammal the effective amount of compound according to (1), or a prodrug thereof;
(26) A method of prophylaxis or treatment for anxiety, or depression, or of pain relief characterized by administering to a mammal the effective amount of compound according to (1), or a prodrug thereof;
(27) A method of prophylaxis or treatment for inflammatory pain or neuropathic pain characterized by administering to a mammal the effective amount of compound according to (1), or a prodrug thereof.
(28) Use of the compound according to (1), or a prodrug thereof, for the manufacture of a FAAH inhibitor;
(29) Use of the compound according to (1), or a prodrug thereof, for the manufacture of a prophylactic or therapeutic agent for anxiety or depression, or an analgesic;
(30) Use of the compound according to (1), or a prodrug thereof, for the manufacture of a prophylactic or therapeutic agent for inflammatory pain or neuropathic pain.

According to the present invention, there can be provided a novel fused-ring compound which has a FAAH inhibitory effect and is useful as an analgesic.

### Best Mode for Carrying Out the Invention

As used herein, "having a FAAH inhibitory activity" refers to "having an activity which directly or indirectly lowers a fatty acid amide hydrolase activity".

As used herein, examples of "halogen (atom)" include fluorine (atom), chlorine (atom), bromine (atom), iodine (atom).

As used herein, examples of "C₁₋₆ alkyl group" include methyl, ethyl, propyl, isopropyl, butyl, isobutyl, sec-butyl, tert-butyl, pentyl, isopentyl, neopentyl, hexyl, and the like.

Definitions of each symbol in formula (I) will be described in detail below.

In the above-described formula (I), R represents an aromatic hydrocarbon or aromatic heterocyclic group which may be substituted with one or more substituents (excluding C₁₋₆alkoxy, phenoxy, carboxyl and tetrazolyl).

Examples of "aromatic hydrocarbon group" represented by R include C₆₋₁₄ aryl groups such as phenyl, 2-biphenylyl, 3-biphenylyl, 4-biphenylyl, cyclooctatetraenyl, and the like. Among these, phenyl is preferred.

Such "aromatic hydrocarbon group" may have 1 to 5, preferably 1 to 3, substituents on substitutable positions. A non-substituted aromatic hydrocarbon group is also preferred.

Examples of such substituent include a halogen atom (e.g., fluorine, chlorine, bromine, iodine, etc.); an optionally halogenated, hydroxylated or oxolated lower alkyl group (e.g., a C₁₋₆ alkyl group such as methyl, ethyl, propyl, isopropyl, butyl, isobutyl, sec-butyl, tert-butyl, pentyl, hexyl, etc.; an optionally halogenated C₁₋₆ alkyl group such as fluoromethyl, chloromethyl, difluoromethyl, dichloromethyl, trifluoromethyl, trichloromethyl, etc.; an optionally hydroxylated C₁₋₆ alkyl group such as hydroxymethyl, hydroxyethyl, etc.; an optionally oxolated C₁₋₆ alkyl group such as 2-oxopropyl, 2-oxobutyl, etc.); a cycloalkyl group (e.g., a C₃₋₆ cycloalkyl group such as cyclopropyl, cyclobutyl, cyclopentyl, cyclohexyl, etc.); a lower alkynyl group (e.g., a C₂₋₆ alkynyl group such as ethynyl, 1-propynyl, propargyl, etc.); a lower alkenyl group (e.g., a C₂₋₆ alkenyl group such as vinyl, allyl, isopropenyl, butenyl, isobutenyl, etc.); an aralkyl group (e.g., a C₇₋₁₁ aralkyl group such as benzyl, α-methylbenzyl, phenethyl, etc.), an aryl group (e.g., a C₆₋₁₀ aryl group such as phenyl, naphthyl, etc., preferably phenyl group); an aryloxy group (e.g., a C₆₋₁₀ aryloxy group (excluding phenoxy)); a lower alkanoyl group (e.g., a C₁₋₆ alkyl-carbonyl group such as formyl, acetyl, propionyl, butyryl, isobutyryl, etc.); an arylcarbonyl group (e.g., a C₆₋₁₀ aryl-carbonyl group such as benzoyl, naphthoyl, etc.); a lower alkanoyloxy group (e.g., a C₁₋₆ alkyl-carbonyloxy group such as formyloxy, acetyloxy, propionyloxy, butyryloxy, isobutyryloxy, etc.); an arylcarbonyloxy group (e.g., a C₆₋₁₀ aryl-carbonyloxy group such as benzoyloxy, naphthoyloxy, etc.); a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, tert-butoxycarbonyl, etc.); an aralkyloxycarbonyl group (e.g., a C₇₋₁₁ aralkyloxycarbonyl group such as benzyloxycarbonyl, etc.); a carbamoyl group; a mono-, di- or tri-halogeno-lower alkyl group (e.g., a mono-, di- or tri-halogeno-C₁₋₄ alkyl group such as chloromethyl, dichloromethyl, trifluoromethyl, 2,2,2-trifluoroethyl, etc.); an oxo group; an amidino group; an imino group, an amino group; a mono-lower alkylamino group (e.g., a mono-C₁₋₄ alkylamino group such as methylamino, ethylamino, propylamino, isopropylamino, butylamino, etc.); a di-lower alkylamino group (e.g., a di-C₁₋₄ alkylamino group such as dimethylamino, diethylamino, dipropylamino, diisopropylamino, dibutylamino, methylethylamino, etc.); a lower alkyl-lower alkylcarbonylamino group (e.g., N-methylacetyl group, etc.); an optionally halogenated lower alkylcarbonylamino group (e.g., acetylamino group, trifluoroacetylamino group, etc.); a 3- to 6-membered cyclic amino group which may contain, in addition to carbon atoms and one nitrogen atom, 1 to 3 heteroatoms selected from an oxygen atom, a sulfur atom and nitrogen atom (e.g., a 3- to 6-membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, N-ethylpiperazinyl, etc.); an alkylenedioxy group (e.g., a C₁₋₃ alkylenedioxy group such as methylenedioxy, ethylenedioxy, etc.); a hydroxy group; a nitro group; a cyano group; a mercapto group; a sulfo group; a sulfino group; a phosphono group; a sulfamoyl group; a monoalkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, N-butylsulfamoyl, etc.); a dialkylsulfamoyl group (e.g., a di-C₁₋₆ alkylsulfamoyl group such as N,N-dimethylsulfamoyl, N,N-diethylsulfamoyl, N,N-dipropylsulfamoyl, N,N-dibutylsulfamoyl, etc.); an alkylthio group (e.g., a C₁₋₆ alkylthio group such as methylthio, ethylthio, propylthio, isopropylthio, butylthio, sec-butylthio, tert-butylthio, etc.); an arylthio group (e.g., a C₆₋₁₀ arylthio group such as phenylthio, naphthylthio, etc.); a lower alkylsulfinyl group (e.g., a C₁₋₆ alkylsulfinyl group such as methylsulfinyl, ethylsulfinyl, propylsulfinyl, butylsulfinyl, etc.); an arylsulfinyl group (e.g., a C₆₋₁₀ arylsulfinyl group such as phenylsulfinyl, naphthylsulfinyl, etc.); a lower alkylsulfonyl group (e.g., a C₁₋₆ alkylsulfonyl group such as methylsulfonyl, ethylsulfonyl, propylsulfonyl, butylsulfonyl, etc.); and an arylsulfonyl group (e.g., a C₆₋₁₀ arylsulfonyl group such as phenylsulfonyl, naphthylsulfonyl, etc.).

Among these, a halogen and an optionally halogenated C₁₋₆ alkyl group are preferred, a C₁₋₆ alkyl group is more preferred, and methyl is particularly preferred.

The "aromatic hydrocarbon group which may be substituted with one or more substituents (excluding C₁₋₆alkoxy, phenoxy, carboxyl and tetrazolyl)" represented by R is also preferably a non-substituted aromatic hydrocarbon group.

Examples of "aromatic heterocyclic group" represented by R include a 5- to 14-membered, preferably a 5- to 10-membered, and more preferably a 5- or 6-membered aromatic heterocyclic group which contains 1 or 2 kinds of 1 to 4 heteroatoms selected from nitrogen, sulfur and oxygen atoms in addition to carbon atoms. Specific examples thereof include thienyl (e.g., 2-thienyl, 3-thienyl, etc.), furyl (e.g., 2-furyl, 3-furyl, etc.), pyridyl (e.g., 2-pyridyl, 3-pyridyl, 4-pyridyl, etc.), thiazolyl (e.g., 2-thiazolyl, 4-thiazolyl, 5-thiazolyl, etc.), oxazolyl (e.g., 2-oxazolyl, 4-oxazolyl, etc.), pyrazinyl, pyrimidinyl (e.g., 2-pyrimidinyl, 4-pyrimidinyl, etc.), pyrrolyl (e.g., 1-pyrrolyl, 2-pyrrolyl, 3-pyrrolyl, etc.), imidazolyl (e.g., 1-imidazolyl, 2-imidazolyl, 4-imidazolyl, etc.), pyrazolyl (e.g., 1-pyrazolyl, 3-pyrazolyl, 4-pyrazolyl, etc.), pyridazinyl (e.g., 3-pyridazinyl, 4-pyridazinyl, etc.), and isothiazolyl (e.g., 3-isothiazolyl, etc.), and isoxazolyl (e.g., 3-isoxazolyl). Among these, a 5- to 10-membered aromatic heterocyclic group (e.g., pyridyl, pyrazinyl, pyrazolyl, pyridazinyl, isoxazolyl, etc.) is preferred.

Such "aromatic heterocyclic group" may have 1 to 5, preferably 1 to 3, substituents on substitutable positions. A non-substituted aromatic heterocyclic group is also preferred.

Examples of such substituent include a halogen atom (e.g., fluorine, chlorine, bromine, iodine, etc.); an optionally halogenated, hydroxylated or oxolated lower alkyl group (e.g., a C₁₋₆ alkyl group such as methyl, ethyl, propyl, isopropyl, butyl, isobutyl, sec-butyl, tert-butyl, pentyl, hexyl, etc.; an optionally halogenated C₁₋₆ alkyl group such as fluoromethyl, chloromethyl, difluoromethyl, dichloromethyl, trifluoromethyl, trichloromethyl, etc.; an optionally hydroxylated C₁₋₆ alkyl group such as hydroxymethyl, hydroxyethyl, etc.; an optionally oxolated C₁₋₆ alkyl group such as 2-oxopropyl, 2-oxobutyl group, etc.); a cycloalkyl group (e.g., a C₃₋₆ cycloalkyl group such as cyclopropyl, cyclobutyl, cyclopentyl, cyclohexyl, etc.); a lower alkynyl group (e.g., a C₂₋₆ alkynyl group such as ethynyl, 1-propynyl, propargyl, etc.); a lower alkenyl group (e.g., a C₂₋₆ alkenyl group such as vinyl, allyl, isopropenyl, butenyl, isobutenyl, etc.); an aralkyl group (e.g., a C₇₋₁₁ aralkyl group such as benzyl, α-methylbenzyl, phenethyl, etc.); an aryl group (e.g., a C₆₋₁₀ aryl group such as phenyl, naphthyl, etc., preferably phenyl group); an aryloxy group (e.g., C₆₋₁₀ aryloxy group (excluding phenoxy)), a lower alkanoyl group (e.g., a C₁₋₆ alkyl-carbonyl group such as formyl, acetyl, propionyl, butyryl, isobutyryl, etc.), an arylcarbonyl group (e.g., a C₆₋₁₀ aryl-carbonyl group such as benzoyl, naphthoyl, etc.), a lower alkanoyloxy group (e.g., a C₁₋₆ alkyl-carbonyloxy group such as formyloxy, acetyloxy, propionyloxy, butyryloxy, isobutyryloxy, etc.); an arylcarbonyloxy group (e.g., a C₆₋₁₀ aryl-carbonyloxy group such as benzoyloxy, naphthoyloxy, etc.); a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, tert-butoxycarbonyl, etc.); an aralkyloxycarbonyl group (e.g., a C₇₋₁₁ aralkyloxycarbonyl group such as benzyloxycarbonyl, etc.); a carbamoyl group; a mono-, di- or tri-halogeno-lower alkyl group (e.g., a mono-, di- or tri-halogeno-C₁₋₄ alkyl group such as chloromethyl, dichloromethyl, trifluoromethyl, 2,2,2-trifluoroethyl, etc.); an oxo group; an amidino group; an imino group; an amino group; a mono-lower alkylamino group (e.g., a mono-C₁₋₄ alkylamino group such as methylamino, ethylamino, propylamino, isopropylamino, butylamino, etc.); a di-lower alkylamino group (e.g., a di-C₁₋₄ alkylamino group such as dimethylamino, diethylamino, dipropylamino, diisopropylamino, dibutylamino, methylethylamino, etc.); a lower alkyl-lower alkylcarbonylamino group (e.g., an N-methylacetyl group, etc.), an optionally halogenated lower alkylcarbonylamino group (e.g., acetylamino, trifluoroacetylamino, etc.); a 3-to 6-membered cyclic amino group which may contain, in addition to carbon atoms and one nitrogen atom, 1 to 3 heteroatoms selected from an oxygen atom, a sulfur atom and nitrogen atom (e.g., a 3- to 6-membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, N-ethylpiperazinyl, etc.); an alkylenedioxy group (e.g., a C₁₋₃ alkylenedioxy group such as methylenedioxy, ethylenedioxy, etc.); hydroxy group, nitro group, cyano group, mercapto group, sulfo group, sulfino group, phosphono group, sulfamoyl group, mono alkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, N-butylsulfamoyl, etc.); a dialkylsulfamoyl group (e.g., a di-C₁₋₆ alkylsulfamoyl group such as N,N-dimethylsulfamoyl, N,N-diethylsulfamoyl, N,N-dipropylsulfamoyl, N,N-dibutylsulfamoyl, etc.); an alkylthio group (e.g., a C₁₋₆ alkylthio group such as methylthio, ethylthio, propylthio, isopropylthio, butylthio, sec-butylthio, tert-butylthio, etc.); an arylthio group (e.g., a C₆₋₁₀ arylthio group such as phenylthio, naphthylthio, etc.); a lower alkylsulfinyl group (e.g., a C₁₋₆ alkylsulfinyl group such as methylsulfinyl, ethylsulfinyl, propylsulfinyl, butylsulfinyl, etc.); an arylsulfinyl group (e.g., a C₆₋₁₀ arylsulfinyl group such as phenylsulfinyl, naphthylsulfinyl, etc.); a lower alkylsulfonyl group (e.g., a C₁₋₆ alkylsulfonyl group such as methylsulfonyl, ethylsulfonyl, propylsulfonyl, butylsulfonyl, etc.); and an arylsulfonyl group (e.g., a C₆₋₁₀ arylsulfonyl group such as phenylsulfonyl, naphthylsulfonyl, etc.).

Among these, a halogen, and an optionally halogenated C₁₋₆ alkyl group are preferred, a C₁₋₆ alkyl group is more preferred, and methyl is particularly preferred.

In the above-described formula (I), A₁, A₂, A₃ and A₄ each independently represents CH or N.

Among A₁, A₂, A₃ and A₄, 0 to 2 substituents preferably represent N.

That is, the moiety represented by formula: 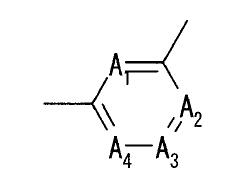 is preferably

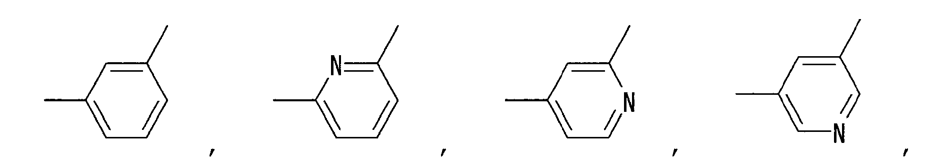

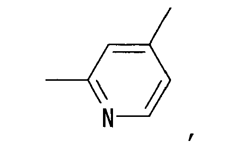

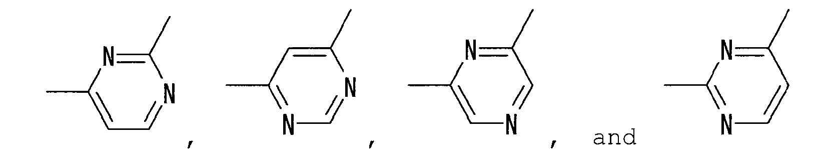

More preferably, the moiety is

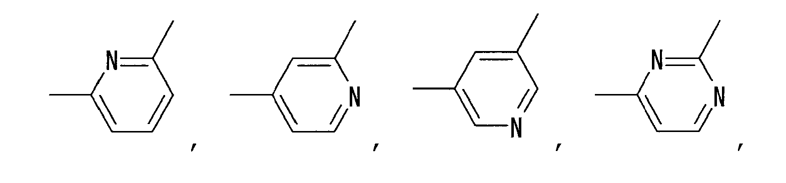

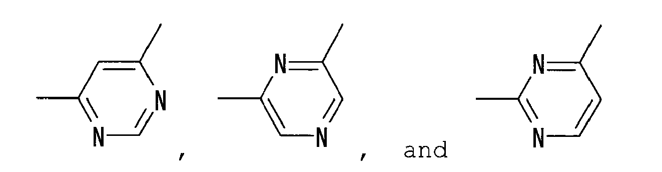

In the above-described formula (I), B represents a phenyl group which may be substituted with one or more halogen atoms.

However, when the moiety represented by formula: 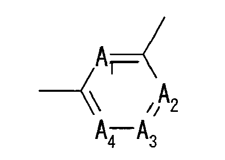 is 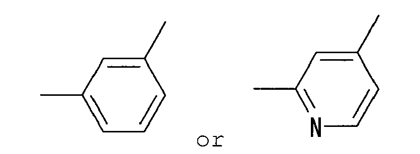 ring B is a phenyl group substituted with one or more halogen atoms.

B is preferably a phenyl group substituted with one or more (preferably, 1 to 2) halogen atoms (preferably, fluorine).

Preferred examples of Compound (I) include the following compounds or salts thereof.

### {Compound (I)-A}

This is the compound in which the moiety represented by the formula: 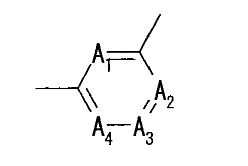 in formula (I) is 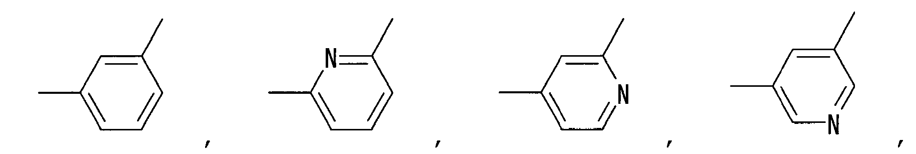 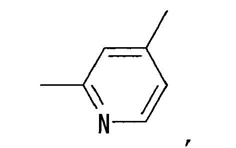 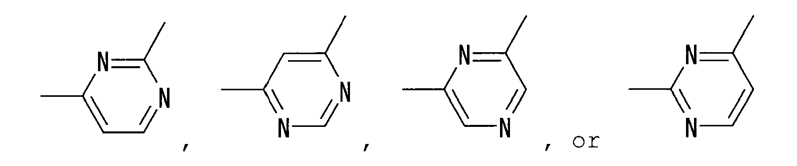 and ring B is a phenyl group substituted with one or more halogen atoms.

Among Compound (I)-A, a compound (I)-A' shown below is preferred.

### {Compound (I)-A'}

This is the compound in which R is a phenyl group (e.g., phenyl, etc.) or a 5- to 10-membered (preferably 5- to 6-membered) aromatic heterocyclic group (e.g., 3-pyridyl, 3,4-dimethyl-5-isoxazolyl, 3-pyridazinyl, 3-methyl-5-isoxazolyl, 2-pyrazinyl, 1-methyl-5-pyrazolyl, etc.) each of which may be substituted with one or more C₁₋₆ alkyl groups,
the moiety represented by formula: 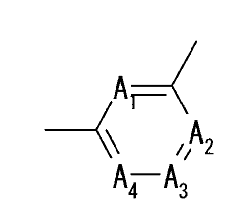 in formula (I) is 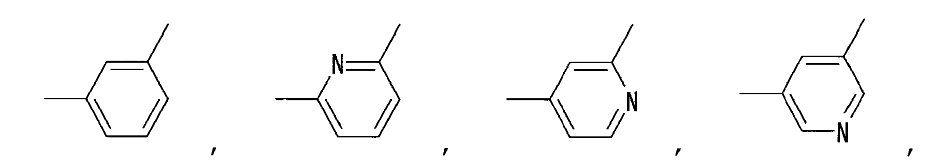 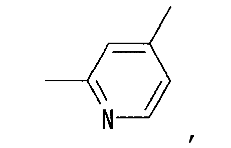 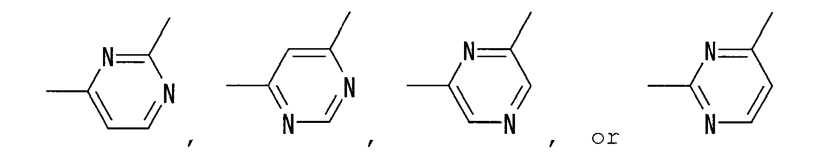 and ring B is a phenyl group substituted with one or more halogen atoms.

### {Compound (I)-B}

This is the compound in which the moiety represented by formula: 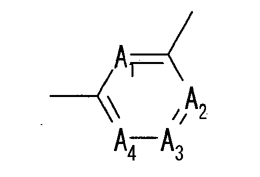 in formula (I) is 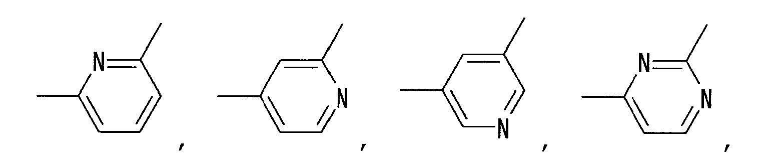 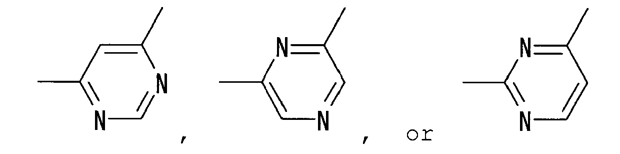 and ring B is a non-substituted phenyl group.

Among Compound (I)-B, Compound (I)-B' shown below is preferred.

### {Compound (I)-B'}

This is the compound in which R is a phenyl group (e.g., phenyl, etc.) or a 5- to 10-membered (preferably 5- to 6-membered) aromatic heterocyclic group (e.g., 3-pyridyl, 3,4-dimethyl-5-isoxazolyl, 3-pyridazinyl, 3-methyl-5-isoxazolyl, 2-pyrazinyl, 1-methyl-5-pyrazolyl, etc.) each of which may be substituted with one or more C₁₋₆ alkyl groups, the moiety represented by formula: 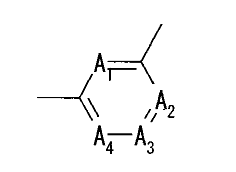 in formula (I) is 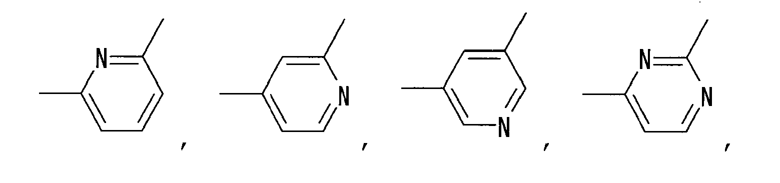 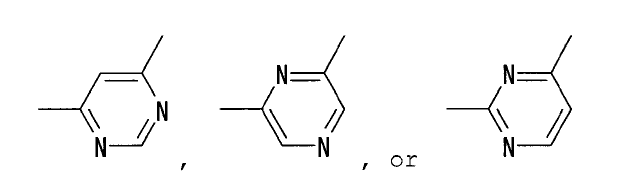 and ring B is a non-substituted phenyl group.

Salts of the compound represented by formula (I) are preferably pharmacologically acceptable salts and examples thereof include salts with an inorganic base, salts with an organic base, salts with an inorganic acid, salts with an organic acid, salts with a basic or acidic amino acid and the like.

Preferred examples of salts with the inorganic base include alkali metal salts such as sodium salts and potassium salts; alkali earth metal salts such as calcium salts and magnesium salts; aluminum salts; ammonium salts and the like.

Preferred examples of salts with the organic base include salts with trimethylamine, triethylamine, pyridine, picoline, ethanolamine, diethanolamine, triethanolamine, tromethamine (tris(hydroxymethyl)methylamine), tert-butylamine, cyclohexylamine, benzylamine, dicyclohexylamine, N,N-dibenzylethylenediamine and the like.

Preferred examples of salts with the inorganic acid include salts with hydrochloric, hydrobromic, nitric, sulfuric, phosphoric acid and the like.

Preferred examples of salts with the organic acid include salts with formic, acetic, trifluoroacetic, phthalic, fumaric, oxalic, tartaric, maleic, citric, succinic, malic, methanesulfonic, benzenesulfonic, p-toluenesulfonic acid and the like.

Preferred examples of salts with the basic amino acid include salts with arginine, lysine, ornithine and the like.

Preferred examples of salts with the acidic amino acid include salts with aspartic acid, glutamic acid and the like.

Prodrugs of Compound (I) refer to compounds which are converted into Compound (I) through the reaction by an enzyme or gastric acid under in vivo physiological conditions, that is, compounds which are converted into Compound (I) through enzymatic oxidation, reduction hydrolysis, or the like, and compounds which are converted into Compound (I) through hydrolysis and the like by gastric acid and the like. Examples of the prodrug of Compound (I) include compounds in which an amino group of Compound (I) is acylated, alkylated or phosphated (e.g., compounds in which an amino group of Compound (I) is eicosanoylated, alanylated, pentylaminocarbonylated, (5-methyl-2-oxo-1,3-dioxolen-4-yl)methoxycarbonylated, tetrahydrofuranylated, tetrahydropyranylated, pyrrolidylmethylated, pivaloyloxymethylated, or tert-butylated); compounds in which a hydroxy group of Compound (I) is acylated, alkylated, phosphated or borated (e.g., compounds in which a hydroxy group of Compound (I) is acetylated, palmitoylated, propanoylated, pivaloylated, succinylated, fumarylated, alanylated, dimethylaminomethylcarbonylated, or tetrahydropyranylated); compounds in which a carboxy group of Compound (I) is esterified or amidated (e.g., compounds in which a carboxy group of Compound (I) is ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified, or methylamidated);and the like. These compounds can be prepared from Compound (I) by a known method.

Prodrugs of Compound (I) may be those which are convenrted into Compound (I) under physiological conditions as described in Hirokawa Book Store, published in 1990, "Development of Drug", Vol. 7, Molecular Design, pp.163-198.

A method for preparing the compound of the present invention will be described below.

### Preparation Process 1

Compound (I) of the present invention can be prepared, for example, according to Preparation Process 1 represented by the following scheme or a process equivalent thereto: 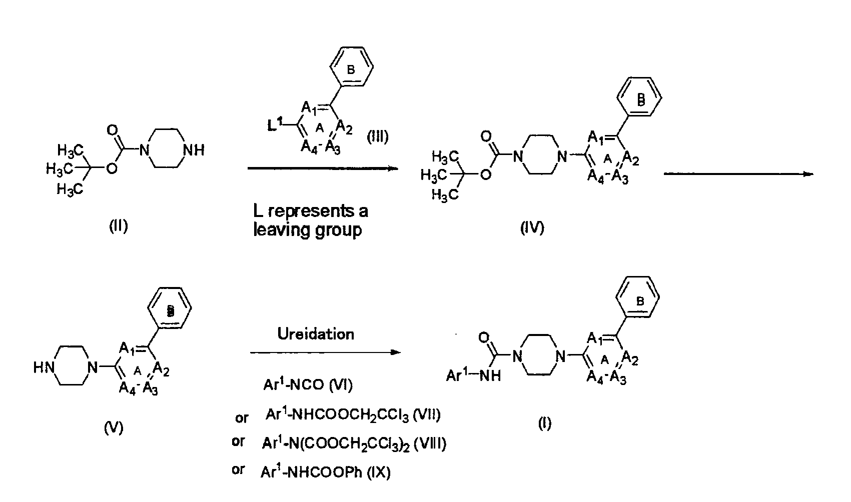 wherein each symbol is as defined above.

Examples of the leaving group L¹ include halides such as chloride, bromide, and iodide; or alkylsulfonyloxy groups such as a methanesulfonyloxy group and a trifluoromethanesulfonyloxy group, and the like.

According to Preparation Process 1, first, Compound (IV) is prepared by subjecting Compound (II) to a substitution reaction using Compound (III).

The substitution reaction is carried out according to a conventional method in the presence of a base and a catalyst in a solvent which does not have influence on the reaction.

Examples of the base include basic salts such as sodium carbonate, potassium carbonate, cesium carbonate, sodium hydrogen carbonate, tripotassium phosphate; aromatic amines such as pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine; metal alkoxides such as sodium methoxide, sodium ethoxide, sodium tert-butoxide, potassium tert-butoxide.

The amounts of the base and Compound (III) to be used are preferably about 1 to about 5 molar equivalents relative to Compound (II), respectively.

Examples of the catalyst to be used in the reaction include palladium catalysts such as palladium acetate, palladium chloride, tetrakis(triphenylphosphine)palladium, bis(dibenzylideneacetone)palladium, tris(dibenzylidenacetone)dipalladium, and preferred examples of "ligand" include phosphines such as trialkylphosphine, triarylphosphine, trialkoxyphosphine.

The amount of the palladium catalyst to be used is usually about 0.001 to about 5 molar equivalents, and preferably about 0.01 to about 0.5 molar equivalents relative to Compound (II). The amount of the "phosphines" to be used is usually about 0.001 to about 10 molar equivalents, and preferably about 0.01 to about 1 molar equivalent relative to Compound (II).

Examples of the solvent which does not have influence on the reaction include ethers such as tetrahydrofuran, 1,2-dimethoxyethane; halogenated hydrocarbons such as chloroform; aromatic hydrocarbons such as toluene; amides such as N,N-dimethyl formamide; and sulfoxides such as dimethyl sulfoxide. These solvents may be used in mixture of two or more kinds at an appropriate ratio. The amount of these solvents to be used is from 1 to 100 fold-volumes relative to Compound (II).

The reaction temperature is usually from about -50°C to about 250°C, and preferably from 0°C to 120°C.

The reaction time is usually from about 0.5 to about 36 hours.

Compound (IV) thus obtained can be isolated and purified by known separation and purification means such as, for example, concentration, reduced pressure concentration, solvent extraction, crystallization, recrystallization, phase transfer, chromatography. Compound (IV) may be used in the next reaction without being isolated.

Then, Compound (V) is prepared by eliminating a tert-butoxycarbonyl group from Compound (IV).

This reaction is carried out according to a conventional method by reacting with an acid in a solvent which does not have influence on the reaction.

Examples of the acid include hydrogen chloride, hydrogen bromide, sulfuric acid, trifluoroacetic acid, and trifluoromethanesulfonic acid, and the like. The amount of the acid to be used is preferably from about 1 to about 100 molar equivalents relative to Compound (IV).

Examples of the solvent which does not have influence on the reaction include hydrocarbons such as hexane; alcohols such as methanol; ethers such as tetrahydrofuran; esters such as ethyl acetate; halogenated hydrocarbons such as chloroform; aromatic hydrocarbons such as toluene; amides such as N,N-dimethyl formamide; and sulfoxides such as dimethyl sulfoxide, and the like. These solvents may be used in mixture to two or more kinds at an appropriate ratio. The amount of these solvents to be used is, for example, from 1 to 100 fold-volumes relative to Compound (IV) .

The reaction temperature is usually from about -50°C to about 250°C, and preferably from 0°C to 120°C.

The reaction time is usually from about 0.5 to about 24 hours.

Compound (V) thus obtained can be isolated and purified by known separation and purification means such as, for example, concentration, reduced pressure concentration , solvent extraction, crystallization, recrystallization, phase transfer, chromatography. Compound (V) may be used in the next reaction without being isolated.

Then, Compound (I) is prepared by subjecting Compound (V) to an ureidation reaction.

The ureidation can be carried out by reacting isocyanate (VI), or 2,2,2-trichloroethylcarbamate (VII), or bis(2,2,2-trichloroethylcarbamate)(VIII), or phenylcarbamate (IX) to Compound (V).

The preparation of Compound (I) by the reaction of Compound (V) and isocyante (VI) is carried out according to a conventional method in the presence of a base in a solvent which does not influence on the reaction. Examples of the base include pyridine, triethylamine, tributylamine, diisopropylethylamine, potassium carbonate, sodium carbonate, sodium hydride, and potassium hydride, and the like.

The amounts of the base and isocyanate (VI) to be used are preferably about 1 to about 5 molar equivalents relative to Compound (V), respectively.

Examples of the solvent which does not have influence on the reaction include ethers such as diethylether, tetrahydrofuran, dioxane, 1,2-dimethoxyethane; halogenated hydrocarbons such as chloroform, dichloromethane; esters such as ethyl acetate; aromatic hydrocarbons such as benzene, toluene; amides such as N,N-dimethyl formamide; and sulfoxides such as dimethyl sulfoxide, and the like. These solvents may be used in mixture to two or more kinds at an appropriate ratio. The amount of these solvents to be used is, for example, from 1 to 100 fold-volumes relative to Compound (V).

The reaction temperature is usually from about -50°C to 250°C, and preferably from 0°C to 120°C.

The reaction time is usually from about 0.5 to about 36 hours.

Compound (I) thus obtained can be isolated and purified by known separation and purification means such as, for example, concentration, reduced pressure concentration , solvent extraction, crystallization, recrystallization, phase transfer, chromatography.

The preparation of Compound (I) by the reaction of Compound (V) and 2,2,2-trichloroethylcarbamate (VII), or bis(2,2,2-trichloroethylcarbamate)(VIII), or phenylcarbamate (IX) is carried out according to a conventional method in the presence of a base in a solvent which does not influence on the reaction. Examples of the base include pyridine, triethylamine, tributylamine, diisopropylethylamine, potassium carbonate, sodium carbonate, sodium hydride, and potassium hydride, and the like.

The amounts of the base and 2,2,2-trichloroethylcarbamate (VII), or bis(2,2,2-trichloroethylcarbamate) (VIII), or phenylcarbamate (IX) to be used are preferably about 1 to about 5 molar equivalents relative to Compound (V), respectively.

Examples of the solvent which does not have influence on the reaction include ethers such as diethylether, tetrahydrofuran, dioxane, 1,2-dimethoxyethane; halogenated hydrocarbons such as chloroform, dichloromethane; esters such as ethyl acetate; aromatic hydrocarbons such as benzene, toluene; ketones such as acetone; amides such as N,N-dimethyl formamide; and sulfoxides such as dimethyl sulfoxide, and the like. These solvents may be used in mixture to two or more kinds at an appropriate ratio. The amount of these solvents to be used is, for example, from 1 to 100 fold-volumes relative to Compound (V).

The reaction temperature is usually from about -50°C to 200°C, and preferably from 0°C to 120°C.

The reaction time is usually from about 0.5 to about 36 hours.

Compound (I) thus obtained can be isolated and purified by known separation and purification means such as, for example, concentration, reduced pressure concentration, solvent extraction, crystallization, recrystallization, phase transfer, chromatography.

### Preparation Process 2

Compound (IV) in the Preparation Process 1 can be prepared, for example, according to Preparation Process 2 presented by the following scheme or a process equivalent thereto; 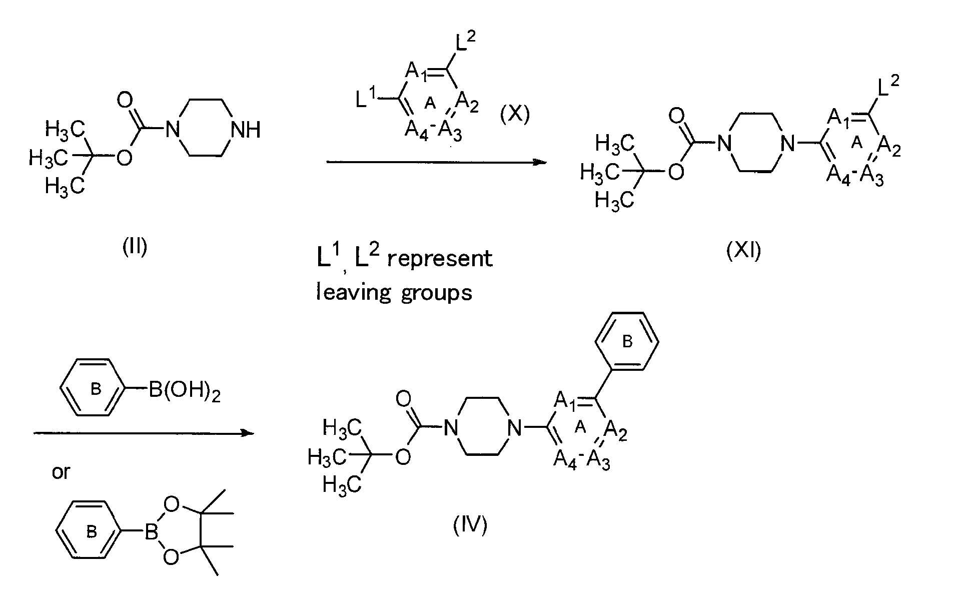 wherein L² represents a leaving group and other symbols are as defined above.

Examples of the leaving group L² include halides such as chloride, bromide, and iodide; or alkylsulfonyloxy groups such as methanesulfonyloxy group and trifluoromethanesulfonyloxy group, and the like.

According to Preparation Process 2, Compound (XI) is prepared by subjecting Compound (II) to a coupling reaction using Compound (X). Compound (XI) can be synthesized from Compound (II) and Compound (III) in a similar method described for the preparation of Compound (IV) of Preparation Process 1.

Compound (XI) thus obtained can be isolated and purified by known separation and purification means such as, for example, concentration, reduced pressure concentration, solvent extraction, crystallization, recrystallization, phase transfer, chromatography. Compound (XI) may be used in the next reaction without being isolated.

Then, Compound (IV) is prepared by subjecting Compound (XI) to coupling reaction with boronic acids or boronic esters.

The coupling reaction is carried out according to a conventional method in the presence of a base and a catalyst in a solvent which does not have influence on the reation.

The amount of the boronic acid or the boronic ester to be used is about 0.5 to about 10 molar equivalents, preferably about 0.9 to about 3 molar equivalents relative to Compound (XI).

Examples of the base include basic salts such as sodium carbonate, potassium carbonate, cesium carbonate, sodium hydrogen bicarbonate, tripotassium phosphate; aromatic amines such as pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine; metal alkoxides such as sodium methoxide, sodium ethoxide, sodium tert-butoxide, potassium tert-butoxide.

The amount of the base to be used is about 0.5 to about 10 molar equivalents, preferably about 1 to about 5 molar equivalents relative to Compound (XI), respectively.

Examples of the catalyst used in the reaction include palladium catalysts such as palladium acetate, palladium chloride, tetrakis(triphenylphosphine)palladium, bis(dibenzylideneacetone)palladium, tris(dibenzylidenacetone)dipalladium, and and the reaction is preferably carried out by adding a ligand. Preferred examples of ligand include phosphines such as trialkylphosphine (e.g., tributylphosphine, tricyclohexyphosphine), triarylphosphine (e.g., triphenylphosphine), trialkoxyphosphine.

The amount of the palladium catalyst to be used is usually about 0.001 to about 5 molar equivalents, preferably about 0.01 to about 0.5 molar equivalents relative to Compound (XI). The amount of the "phosphines" to be used is usually about 0.001 to about 10 molar equivalents relative to Compound (II), preferably about 0.01 to about 1 molar equivalent relative to Compound (II).

Examples of the solvent which does not have influence on the reaction include ethers such as tetrahydrofuran, 1,2-dimethoxyethane; alcohols such as methanol, ethanol, propanol; halogenated hydrocarbons such as chloroform; aromatic hydrocarbons such as benzene, toluene; nitriles such as acetnitrile, propionitrile; amides such as N,N-dimethyl formamide; sulfoxides such as dimethyl sulfoxide; and water. These solvents may be used in mixture of two or more kinds at an appropriate ratio. The amount of these solvents to be used is, for example, from 1 to 100 fold-volumes relative to Compound (II).

The reaction temperature is usually from about -50°C to about 250°C, and preferably from 0°C to 120°C.

The reaction time is usually from about 0.5 to about 36 hours.

The reaction time can be shortened by using a microwave apparatus, and the like.

Compound (I) thus obtained can be isolated and purified by known separation and purification means such as, for example, concentration, reduced pressure concentration, solvent extraction, crystallization, recrystallization, phase transfer, chromatography.

When Compound (I) has isomers such as optical isomers, stereoisomers, regioisomers, or rotational isomers, Compound (I) encompasses any one of such isomers and mixtures thereof. For example, when optical isomers of Compound (I) are present, optical isomers obtained by resolution of racemates are also included in Compound (I). These isomers can be obtained as an isolated product by synthetic means and separation means known per se in the art (concentration, solvent extraction, column chromatography, recrystallization, etc.).

Compound (I) may be in the form of crystals, and Compound (I) encompasses both single crystalline forms and mixed crystalline forms. Crystals can be prepared by crystallization according to crystallization methods known per se in the art.

Compound (I) may be either a solvate (e.g., hydrate, etc.) or a non-solvate, and encompasses both forms.

Compounds labeled with isotopes (e.g., ³H, ¹⁴C, ³⁵S, ¹²⁵I, etc.) are also comprised in Compound (I).

Since Compound (I) or a prodrug thereof (hereinafter, sometimes, referred to as the compound of the present invention) has an excellent FAAH inhibitory activity against mammals (e.g., mouse, rat, hamster, rabbit, cat, dog, cow, sheep, monkey, human, etc.), it is useful as a prophylactic and/or therapeutic agent for FAAH-mediated conditions or diseases.

As described in Examples hereinafter, a threshold in a pain model is decreased by administration of the compound of the present invention having a FAAH inhibitory activity. Therefore, the compound of the present invention is useful for prophylaxis and treatment of pain, for example, inflammatory pain associated with osteoarthritis and rheumatoid arthritis and neuropathic pain such as painful diabetic neuropathy/diabetic neuropathic pain, postherapetic neuraigia, trigeminus neuralgia.

In addition, examples of the disease of which the compound of the present invention is useful for prophylaxis and treatment include, but are not limited to, cerebrovascular disorder caused by disorder of cerebral nerve cells, cerebral nerve cell protection effect upon head trauma or spinal cord injury, brain disorder upon revival after cardiac arrest, brain functional decline before and after brain operation, hypoxidosis, hypoglycemia, trauma of brain or spinal cord, drug intoxication, gas poisoning, diabetes mellitus, administration of antitumor agent, disorder of nervous system caused by alcohol and the like, Huntington's chorea, prion disease, amyotrophic lateral sclerosis, spinocerebellar degeneration, eating disorder, adiposis, pollakisuria, urinary incontinence, rheumatism, hypertrophic arthritis, interstitial cystitis, Crohn's disease, colitis, colonitis, colon cancer, large bowel cancer, contraception, or AIDS.

The compound of the present invention is also useful, based on the above-described knowledge in the art, as a prophylatic and/or therapeutic agent for nausea, sicchasia or vomiting caused by anticancer agent; apocleisis or cachectic anorexia in cancer or infection (e.g., AIDS, etc.); convulsion, pain, tremor, nystagmus or enuresis due to multiple sclerosis; chronic pain; Huntington's chorea; Tourette's syndrome; levodopa-induced dyskinesia; locomotor disorder; asthma; glaucoma; allergy; inflammation; epilepsy; autoimmune disease; diarrhea; obesity; sleep disorder; depression; anxiety; mental diseases; Crohn's disease; Alzheimer's disease; interstitial cystitis; AIDS; colitis; colonitis; colon cancer; rectal cancer; hypertriglyceridemia; hyperlipemia; diabetes mellitus; arterial sclerosis; and Parkinson's disease, or as a contraceptive.

Furthermore, since FAAH is an enzyme which hydrolyzes an endogenous sleep substance, oleamide, a FAAH inhibitory agent induces sleep by suppressing the decomposition of oleamide. Therefore, the compound of the present invention is a useful prophylactic and/or therapeutic agent of sleep disorders, for example, sleep abnormality such as intrinsic sleep disorders (e.g., psychophysiological insomnia), extrinsic sleep disorders, circadian rhythm disorders (e.g., time zone change (jet lag) syndrome, shift work sleep disorder, irregular sleep-wake pattern, delayed sleep phase syndrome, advanced sleep phase syndrome, non-24 hour sleep-wake), and the like; parasomunias; and sleep disorders associated with medical or psychiaric disorders (e.g., chronic obstructive pulmonary disease, Alzheimer's disease, Parkinson's disease, cerebrovascular dementia, schizophrenia, depression, anxiety neurosis).

Compound (I) or a prodrug thereof has low toxicity (e.g., acute toxicity, chronic toxicity, genotoxicity, reprotoxy, cardiotoxicity, drug interaction, carcinogenicity, etc.) and can be used as prophylatic and/or therapeutic agents for various diseases described hereinafter in mammals (e.g., human, mouse, rat, rabbit, do g, cat, cow, horse, pig, monkey, etc.) as such, or after formulating into a pharmaceutical composition by mixing with a pharmacologically acceptable carrier.

Examples of the dosage form of the pharmaceutical composition include oral preparations such as tablets (including sugar coated tablets, film coated tablets, sublingual tablets, and orally disintegrating tablets), capsules (including soft capsules and microcapsules), granules, powders, troches, syrups, emulsions, suspensions, films (e.g., orally disintegrating films); and parenteral preparations such as injections (e.g., subcutaneous injections, intravenous injections, intramuscular injections, intraperitoneal injections, drops, etc.), external preparations (e.g., transdermal preparations, ointments, etc.), suppositories (e.g., rectal suppositories, vaginal suppositories, etc.), pellets, nasal agents, pulmonary agents (inhalants), and eye drops. These can be safely administered orally or parentrally (e.g., topical, rectal, intravenous administration).

These preparations may be release controlled preparations such as rapid release preparations or sustained release preparations (for example, sustained release microcapsules).

The pharmaceutical composition can be prepared by a conventional method in the art of formulation, for example, a method described in Japanese Pharmacopeia.

The content of the compound of the present invention in the pharmaceutical composition varies depending on the dosage form, the dose of the compound of the present invention, but it is, for example, from about 0.1 to 100% by weight.

Herein, as the pharmacologically acceptable carrier, a variety of organic or inorganic carrier materials that are conventionally used as materials used for preparation can be used, and they are incorporated as excipient, lubricant, binder or disintegrant in solid preparations; and as solvent, solubilizing agent, suspending agent, isotonic agent, buffer, soothing agent or the like in liquid preparations. In addition, preparation additives such as antiseptic, antioxidant, colorant or sweetener can be also used if necessary.

Preferred examples of excipients include lactose, sucrose, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous sicilic acid, synthetic aluminum silicate, magnesium aluminate metasilicate and the like.

Preferred examples of lubricants include magnesium stearate, calcium stearate, talc, colloidal silica and the like.

Preferred examples of binders include pregelatinized starch, sucrose, gelatin, gum arabic, methylcellulose, carboxymethyl cellulose, sodium carboxymethyl cellulose, crystalline cellulose, lactose, D-mannitol, trehalose, dextrin, pullulan, hydroxypropyl cellulose, hydroxypropylmethyl cellulose, polyvinyl pyrrolidone and the like.

Preferred examples of disintegrants include lactose, sucrose, starch, carboxymethyl cellulose, calcium carboxymethyl cellulose, sodium croscarmellose, sodium carboxymethyl starch, light anhydrous sicilic acid, low-substituted hydroxypropyl cellulose and the like.

Preferred examples of solvents include water for injection, physiological saline, Ringer's solution, alcohol, propylene glycol, polyethylene glycol, sesame oil, corn oil, olive oil, cotton seed oil and the like.

Preferred examples of solubilizers include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferred examples of suspending agents include surfactants such as stearyltriethanolamine, sodium lauryl sulfate, laurylaminopropionic acid, lecithin, benzalkonium chloride, benzethonium chloride, and glycerin monostearate; hydrophilic polymers such as polyvinyl alcohol, polyvinyl pyrrolidone, sodium carboxymethyl cellulose, methyl cellulose, hydroxymethyl cellulose, hydroxyethyl cellulose, and hydroxypropyl cellulose; polysorbates, polyoxyethylene hardened castor oil and the like.

Preferred examples of isotonizing agents include sodium chloride, glycerin, D-mannitol, D-sorbitol, glucose and the like.

Preferred examples of buffers include buffer solutions of phosphate, acetate, carbonate, citrate and the like.

Preferred examples of soothing agents include benzyl alcohol and the like.

Preferred examples of antiseptics include paraoxybenzoate esters, chlorobutanol, benzyl alcohol, phenethyl alcohol, dehydroacetic acid, sorbic acid and the like.

Preferred examples of antioxidants include sulfite, ascorbate and the like.

Preferred examples of coloring agents include water-soluble edible tar dyes (e.g., food dyes such as Food Red No. 2 and No. 3, Food Yellow No. 4 and No. 5, Food Blue No. 1 and No. 2, etc.), water-insoluble lake dyes (e.g., aluminum salts of the above water-soluble edible tar dyes), natural dyes (e.g., β-carotene, chlorophyll, colcothar, etc.) and the like.

Preferred examples of sweeteners include saccharine sodium, dipotassium glycyrrhizinate, aspartame, stevia and the like.

Furthermore, the compound of the present invention can be used in combination with drugs other than the compound of the present invention.

Examples of drugs which can be used in combination with the compound of the present invention (hereinafter may be abbreviated to a combination drug) include nonsteroidal anti-inflammatory agents (e.g., meloxicam, tenoxicam, indomethacin, ibuprofen, celecoxib, rofecoxib, aspirin, indomethacin, etc.), disease modifying anti-rheumatic drugs (DMARDs), antipyretic-analgesics (acetanilide, acetaminophen, phenacetin, etc.), steroidal anti-inflammatory agents (hydrocortisone, prednisolone, methylprednisolone, betamethazone, dexamethasone, etc.), narcotic analgesics (morphine, fentanyl, codeine phosphate, pethidine, oxycodone, etc.), nonnarcotic analgesics (tramadol, etc.), local anesthetics (lidocaine, etc.), anticonvulsants (gabapentin, bupivacaine, carbamazepine, phenytoin, etc.), antiarrhythmic drugs (procaine, etc.), anti-cytokine drugs (TNF inhibitor, MAPkinase inhibitor, etc.), aldose reductase inhibitors (kinedak, etc.), cannabinoid agonists (tetrahydrocannabinol, etc.) and the like. In addition, examples of combination drug include antidementia drugs, for example, acetylcholinesterase inhibitors (e.g., donepezil, rivastigmine, galanthamine, zanapezil, etc.), β-amyloid protein production, secretion, accumulation, aggregation and/or deposition inhibitors (β-secretase inhibitors (e.g., compounds described in WO98/38156, compounds described in WO02/2505, W002/2506 and WO02/2512, OM99-2 (WO01/00663)), γ-secretase inhibitors (LY-450139, etc.), γ-secretase modulators (E2012, etc.), β-amyloid protein aggregations (e.g., PTI-00703, ALZHEMED (NC-531), PPI-368 (JP 11-514333 A), PPI-558 (JP 2001-500852 A), SKF-74652 (Biochem. J.(1999),340(1),283-289)), β-amyloid antibody, β-amyloid vaccine, β-amyloid degrading enzyme, etc.), cerebral function activating agents (e.g., aniracetam, nicergoline, etc.), neurogenesis/neurotization promoters (e.g., Akt/PKB activator, GSK-3β inhibitor, etc.), therapeutic agents for Parkinson's disease (e.g., dopamine receptor agonist (L-dopa, bromocriptene, pergolide, talipexole, pramipexole, cabergoline, adamantadine, etc.), monoamine oxidase (MAO) inhibitor (deprenyl, selegiline, remacemide, riluzole, etc.), cholinolytic drugs (e.g., trihexyphenidyl, biperiden, etc.), COMT inhibitors (e.g., entacapone, etc.)), therapeutic agents amyotrophic lateral sclerosis (e.g., riluzole, neurotrophic factor, etc.), therapeutic agents for abnormal behavior and wandering accompanied by progress of dementia (e.g., sedative, anxiolytic, etc.), apoptosis inhibitors (e.g., CPI-1189, IDN-6556, CEP-1347, etc.), neuronal differentiation/neurotization promoters (leteprinim, Xaliproden; SR-57746-A, SB-216763, etc.), antidepressant drugs (e.g., MAO inhibitor, tricyclic antidepressant drug, selective serotonin reuptake inhibitor SSRI, selective serotonin-noradrenaline reuptake inhibitor SNRI, triple reuptake inhibitor, CRF antagonist, etc.), antianxiety drug s (e.g., benzodiazepine-based medicine, etc.), sleeping drugs (e.g., benzodiazepine-based medicine, non-benzodiazepine-based medicine, melatonin agonist, etc.), thrombolytic drug (e.g., tissue plasminogen activator, urokinase, etc.), anticoagulants (e.g., argatroban, warfarin, etc.), tenth factor inhibitors, thromboxane synthase inhibitors (e.g., ozagrel, etc.), antioxidants (e.g., edaravone, etc.), antihydropic agents (e.g., glycerol, mannitol, etc.), therapeutic agents for hyperlipemia such as cholesterol-lowering drug (statin (e.g., pravastatin sodium, atorvastatin, simvastatin, rosuvastatin, etc.), fibrate (e.g., clofibrate, etc.), squalene synthase inhibitor), antihypertensive agents (ACE inhibitor, angiotensin II antagonist, renin inhibitor, etc.), antidiabetics (e.g., insulin preparation, insulin secretion promoters such as sulfonylurea agents, insulin resistance improving agents such as PAR agonist and PPAR partial agonist, DPPIV inhibitors, etc.), anticancer drugs (e.g., platinum preparation, 5-FU, anthracyclines, molecular-target agent, hormone therapy drug, etc.), therapeutic agents for pollakisuria and urinary incontinence (e.g., harncare, solifenacin, etc.), therapeutic agents for overactive bladder (e.g., tolterodine, etc.), therapeutic agents for osteoporosis (e.g., vitamin D preparation, PTH, calcium receptor antagonist, calcitonin, risedronate, alendronate, etc.) and the like.

Combination of the compound of the present invention with the combination drug can obtain an excellent effect such as:
(1) the combination can reduce the dosage compared with the case where the compound of the present invention or combination drug is administered alone;
(2) the combination can set long treatment period by selecting the combination drug having action mechanism different from the compound of the present invention;
(3) the combination can maintain a therapeutic effect by selecting the combination drug having action mechanism different from the compound of the invention; and
(4) the combination can obtain a synergistic effect by combining the compound of the present invention with the combination drug.

Hereinafter, when the compound of the present invention and the combination drug are used in combination, administration timing of the compound of the present invention and the combination drug is no specifically limited, and the compound of the present invention and the combination drug may be administered simultaneously or in time intervals to subject of administration. The dosage of the combination drug may be based on the dosage used clinically and can select appropriately depending on subject of administration, administration route, diseases, combination thereof or the like.

Examples of the dosage form of the compound of the present invention and the combination drug include (1) administration of a single preparation obtained by formulating simultaneously the compound of the present invention and the combination drug, (2) simultaneous administration in the same administration route of two kinds of preparations obtained by formulating separately the compound of the present invention and the combination drug, (3) time lag administration in the same administration route of two kinds of preparations obtained by formulating separately the compound of the present invention and the combination drug, (4) simultaneous administration in a different administration route of two kinds of preparations obtained by formulating separately the compound of the present invention and the combination drug, and (5) time lag administration in a different administration route of two kinds of preparations obtained by formulating separately the compound of the present invention and the combination drug (e.g., administration of the compound of the present invention and the combination drug in this order, or in a reverse order).

### EXAMPLES

While the present invention is illustrated in more detail by the following Examples, Formulation Examples and Experimental Examples, these examples are just embodiments and not intended to limit the present invention. In addition, various changes can be made without departing from the scope of the present invention.

"Room temperature" in the following Reference Examples and Examples usually means a temperature from about 10°C to about 35°C.

Other abbreviations used in the context have the following meanings.
s: singlet
d: doublet
t: triplet
q: quartet
m: multiplet
br: broad
J: coupling constant
Hz: Hertz
CDCl₃: deuterated chloroform
DMSO-d₆: deuterated dimethyl sulfoxide
¹H NMR: proton nuclear magnetic resonance
(R,R)-Me-BPE: (+)-1,2-bis((2R,5R)-2,5-dimethylphospholano) ethane
(S,S)-Et-FerroTANE: (-)-1,1'-bis((2S,4S)-2,4-diethylphosphotano)ferrocene

### Example 1

### 4-[2-(2,4-difluorophenyl)pyrimidin-4-yl]-N-pyridin-3-ylpiperazin-1-carboxamide dihydrochloride

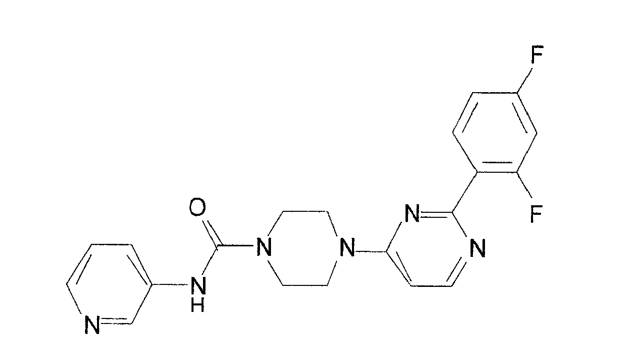

### (1) Tert-butyl 4-(2-chloropyrimidin-4-yl)piperazine-1-carboxylate

A mixture of 2,4-dichloropyrimidine (1.00 g, 6.71 mmol), tert-butyl piperazine-1-carboxylate (1.37 g, 7.38 mmol), triethylamine (1.40 ml, 10.1 mmol) and N,N-dimethylformamide (10 ml) was stirred at room temperature for 4 hours. The reaction was poured into water and extracted with ethyl acetate. The extract was washed with water, dried over anhydrous magnesium sulfate, and the solvent was distilled off under reduced pressure. To the residue was added diethylether and separated by filtration to obtain the title compound (1.80 g, 90%) as a solid.
¹H NMR (CDCl₃) δ: 1.49 (9H, s), 3.47 - 3.58 (4H, m), 3.60 - 3.71 (4H, m), 6.40 (1H, d, J = 6.2 Hz), 8.07 (1H, d, J = 6.2 Hz).

### (2) Tert-butyl 4-[2-(2,4-difluorophenyl)pyrimidin-4-yl]piperazine-1-carboxylate

To a mixture of tert-butyl 4-(2-chloropyrimidin-4-yl)piperazine-1-carboxylate (1.80 g, 6.02 mmol), 2,4-difluorophenylboroic acid (1.43 g, 9.04 mmol) in 2N aqueous sodium carbonate solution (24 ml) and toluene (60 ml) was added tetrakistriphenylphosphine palladium (835 mg, 0.723 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 4:1) to obtain the title compound (907 mg, 40%) as an oily material.
¹H NMR (CDCl₃) 5: 1.49 (9H, s), 3.48 - 3.76 (8H, m), 6.44 (1H, d, J = 6.4 Hz), 6.84 - 6.99 (2H, m), 8.02 - 8.12 (1H, m), 8.36 (1H, d, J = 6.4 Hz).

### (3) 2-(2,4-difluorophenyl)-4-piperazin-1-ylpyrimidine

To a solution of tert-butyl 4-[2-(2,4-difluorophenyl)pyrimidin-4-yl]piperazine-1-carboxylate (888 mg, 2.36 mmol) in ethyl acetate (9 ml) was added 4N hydrogen chloride-ethyl acetate solution (9 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (554 mg, 85%) as an oily material.
¹H NMR (CDCl₃) δ: 3.01 - 3.17 (4H, m), 3. 69 - 3.98 (4H, m), 6.37 - 6.52 (1H, m), 6.81 - 7.03 (2H, m), 7.93 - 8.17 (1H, m), 8.29 - 8.45 (1H, m).

### (4) 4-[2-(2,4-difluorophenyl)pyrimidin-4-yl]-N-pyridin-3-ylpiperazine-1-carboxamide dihydrochloride

To a solution of 2-(2,4-difluorophenyl)-4-piperazin-1-ylpyrimidine (100 mg, 0.362 mmol) and triethylamine (50.4 µl, 0.362 mmol) in tetrahydrofuran (1.5 ml) was added 3-pyridine isocyanate (65.2 µl, 0.543 mmol) at room temperature and the mixture was stirred at room temperature for 5 hours, and the solvent was distilled off under reduced pressure. The residue was purified by high performance liquid chromatography (YMC HPLC column, solution A: 0.1% trifluoroacetic acid-acetonitrile solution, solution B: 0.1% aqueous trifluoroacetic acid solution, eluted with 10% to 100% solution A) to obtain 4-[2-(2,4-difluorophenyl)pyrimidin-4-yl]-N-pyridin-3-ylpiperazine-1-carboxamide as an oily material. 4-[2-(2,4-difluorophenyl)pyrimidin-4-yl]-N-pyridin-3-ylpiperazine-1-carboxamide (51.2 mg, 0.129 mmol) was dissolved in ethyl acetate (1.0 ml) and 4N hydrogen chloride-ethyl acetate solution (1.0 ml) was added and stirred at room temperature for 1 hour, and the solvent was distilled off under reduced pressure. The residue was recrystallized from methanol and diethylether to obtain the title compound (52.3 mg, 31%) as a solid. Melting point: 187 - 188°C.
¹H NMR (DMSO-d₆) δ: 3.69 - 3.86 (4H, m), 3.89 - 4.10 (4H, m), 7.22 (1H, d, J = 7.2 Hz), 7.36 (1H, d, J = 2.3 Hz), 7.47 - 7.63 (1H, m), 7.93 (1H, dd, J = 8.6, 5.6 Hz), 8.03 - 8.20 (1H, m), 8.34 - 8.59 (2H, m), 8.67 (1H, d, J = 8.6 Hz), 9.18 (1H, d, J = 2.3 Hz), 10.18 (1H, s).

### Example 2

### 4-[2-(2,4-difluorophenyl)pyrimidin-4-yl]-N-(3,4-dimethylisoxazol-5-yl)piperazine-1-carboxamide

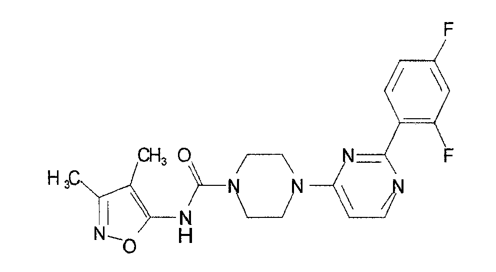

A mixture of 2,2,2-trichloroethyl (3,4-dimethylisoxazol-5-yl)carbamate (115 mg, 0.398 mmol), 2-(2,4-difluorophenyl)-4-piperazin-1-ylpyrimidine (100 mg, 0.362 mmol) and diisopropylethylamine (0.126 ml, 0.724 mmol) in dimethylsulfoxide (1.5 ml) was stirred at 70°C overnight. The reaction was poured into water and extracted with ethyl acetate. The extract was washed with water, dried over anhydrous magnesium sulfate, and the solvent was distilled off under reduced pressure. The residue was purified by high performance liquid chromatography (YMC HPLC column, solution A: 0.1% trifluoroacetic acid-acetonitrile solution, solution B: 0.1% aqueous trifluoroacetic acid solution, eluted with 10% to 100% solution A) and recrystallized from hexane and ethyl acetate to obtain the title compound (36.6 mg, 24%) as a solid. Melting point: 207 - 208°C.
¹H NMR (DMSO-d₆) δ: 1.76 (3H, s), 2.13 (3H, s), 3.50 - 3.62 (4H, m), 3.69 - 3.80 (4H, m), 6.86 (1H, d, J = 6.2 Hz), 7.13 - 7.23 (1H, m), 7.26 - 7.38 (1H, m), 8.02 - 8.14 (1H, m), 8.35 (1H, d, J = 6.2 Hz), 9.25 (1H, s).

### Example 3

### 4-[2-(2,4-difluorophenyl)pyrimidin-4-yl]-N-pyridazin-3-ylpiperazine-1-carboxamide

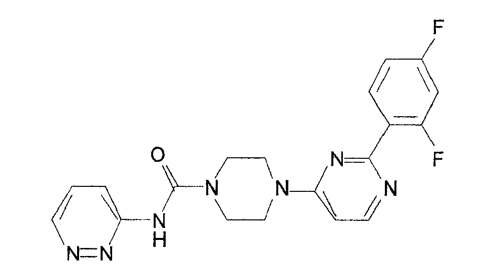

A mixture of 2,2,2-trichloroethyl pyridazin-3-ylcarbamate (108 mg, 0.398 mmol), 2-(2,4-difluorophenyl)-4-piperazin-1-ylpyrimidine (100 mg, 0.362 mmol) and diisopropylethylamine (0.126 ml, 0.724 mmol) in dimethylsulfoxide (1.5 ml) was stirred at 70°C overnight. The reaction was poured into water and extracted with ethyl acetate. The extract was washed with water, dried over anhydrous magnesium sulfate, and the solvent was distilled off under reduced pressure. The residue was purified by silica gel column chromatography (methanol:ethyl acetate = 1:19) and recrystallized from hexane and ethyl acetate to obtain the title compound (31.9 mg, 22%) as a solid. Melting point: 200 - 201°C.
¹H NMR (DMSO-d₆) δ: 3.58 - 3.71 (4H, m), 3.72 - 3.84 (4H, m), 6.88 (1H, d, J = 6.2 Hz), 7.14 - 7.25 (1H, m), 7.28 - 7.39 (1H, m), 7.59 (1H, dd, J = 9.1, 4.6 Hz), 7.96 - 8.14 (2H, m), 8.36 (1H, d, J = 6.2 Hz), 8.85 (1H, d, J = 4.0 Hz ), 9.99 (1H, s).

### Example 4

### 4-[2-(2,3-difluorophenyl)pyrimidin-4-yl]-N-pyridin-3-ylpiperazine-1-carboxamide

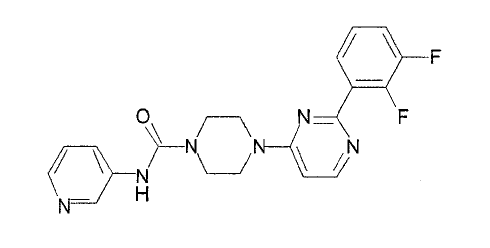

### (1) Tert-butyl 4-[2-(2,3-difluorophenyl)pyrimidin-4-yl]piperazine-1-carboxylate

To a mixture of tert-butyl 4-(2-chloropyrimidin-4-yl)piperazine-1-carboxylate (3.0 g, 10.0 mmol), 2,3-difluorophenylboronic acid (2.38 g, 15.1 mmol), 2N aqueous sodium carbonate solution (40 ml) and toluene (100 ml) was added tetrakistriphenylphosphine palladium (1.39 g, 1.20 mmol) at room temperature under nitrogen atmosphere, and the mixuture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate) and high performance liquid chromatography (YMC HPLC column, solution A: 0.1% trifluoroacetic acid-acetonitrile solution, solution B: 0.1% aqueous trifluoroacetic acid solution, eluted with 10% to 100% solution A) to obtain the title compound (424 mg, 11%) as an oily material.
¹H NMR (CDCl₃) δ: 1.49 (9H, s), 3.52 - 3.59 (4H, m), 3.68 - 3.77 (4H, m), 6.47 (1H, d, J = 6.2 Hz), 7.08 - 7.28 (2H, m), 7.74 - 7.85 (1H, m), 8.37 (1H, d, J = 6.2 Hz).

### (2) 2-(2,3-difluorophenyl)-4-piperazin-1-ylpyrimidine

To a solution of tert-butyl 4-[2-(2,3-difluorophenyl)pyrimidin-4-yl]piperazine-1-carboxylate (420 mg, 1.12 mmol) in ethyl acetate (4.2 ml) was added 4N hydrogen chloride-ethyl acetate solution (4.2 ml) and the mixture was stirred at room temperature for 5 hours, and the solvent was distilled off under reduced pressure. The residue was dissolved in water and the pH was adjutsted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (310 mg, quantitative) as an oily material.
¹H NMR (CDCl₃) δ: 2.93 - 3.00 (2H, m), 3.51 - 3.59 (2H, m), 3.66 - 3.77 (4H, m), 6.46 (1H, dd, J = 6.2, 4.9 Hz), 7.06 - 7.28 (2H, m), 7.74 - 7.84 (1H, m), 8.36 (1H, dd, J = 10.0, 6.2 Hz).

### (3) 4-[2-(2,3-difluorophenyl)pyrimidin-4-yl]-N-pyridin-3-ylpiperazine-1-carboxamide

A mixture of 2,2,2-trichloroethyl pyridin-3-ylcarbamate (107 mg, 0.398 mmol), 2-(2,3-difluorophenyl)-4-piperazin-1-ylpyrimidine (100 mg, 0.362 mmol) and diisopropylethylamine (0.126 ml, 0.724 mmol) in dimethylsulfoxide (1.2 ml) was stirred at 70°C overnight. The reaction was poured into water and extracted with ethyl acetate. The extract was washed with water, dried over anhydrous magnesium sulfate, and the solvent was distilled off under reduced pressure. The residue was purified by silica gel column chromatography (methanol:ethyl acetate = 1:9) and recrystallized from hexane and ethyl acetate to obtain the title compound (25.9 mg, 18%) as a solid. Melting point: 208 - 209°C.
¹H NMR (DMSO-d₆) δ: 3.56 - 3.66 (4H, m), 3.71 - 3.82 (4H, m), 6.91 (1H, d, J = 6.2 Hz), 7.22 - 7.37 (2H, m), 7.48 - 7.60 (1H, m), 7.77 - 7.93 (2H, m), 8.16 (1H, dd, J = 4.7, 1.3 Hz), 8.38 (1H, d, J = 6.2 Hz), 8.66 (1H, d, J = 2.3 Hz), 8.81 (1H, s).

### Example 5

### 4-[2-(2,3-difluorophenyl)pyrimidin-4-yl]-N-(3,4-dimethylisoxazol-5-yl)piperazine-1-carboxamide

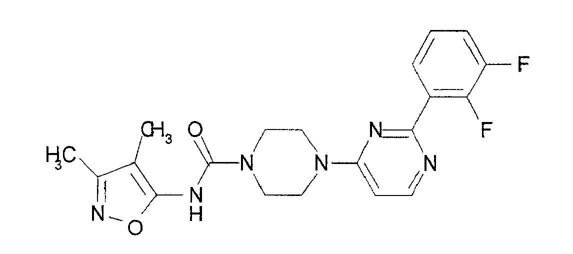

A mixture of 2,2,2-trichloroethyl (3,4-dimethylisoxazol-5-yl)carbamate (115 mg, 0.398 mmol), 2-(2,3-difluorophenyl)-4-piperazin-1-ylpyrimidine (100 mg, 0.362 mmol) and diisopropylethylamine (0.126 ml, 0.724 mmol) in dimethylsulfoxide (1.2 ml) was stirred at 70°C overnight. The reaction was poured into water and extracted with ethyl acetate. The extract was washed with water, dried over anhydrous magnesium sulfate, and the solvent was distilled off under reduced pressure. The residue was purified by silica gel column chromatography (methanol:ethyl acetate = 1:19) and recrystallized from hexane and ethyl acetate to obtain the desired product (27.0 mg, 18%) as a solid. Melting point: 209 - 210°C
¹H NMR (DMSO-d₆) δ: 1.76 (3H, s), 2.13 (3H, s), 3.52 - 3.63 (4H, m), 3.68 - 3.83 (4H, m), 6.89 (1H, d, J = 6.4 Hz), 7.24 - 7.36 (1H, m), 7.47 - 7.61 (1H, m), 7.77 - 7.89 (1H, m), 8.38 (1H, d, J = 6.4 Hz ), 9.25 (1H, s).

### Example 6

### 4-[2-(2,3-difluorophenyl)pyrimidin-4-yl]-N-pyridazin-3-ylpiperazine-1-carboxamide

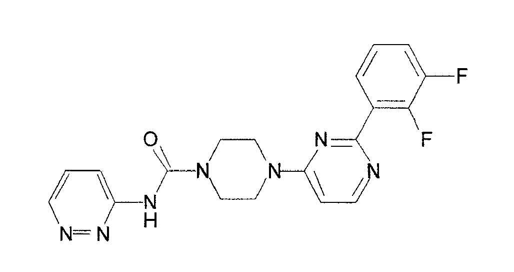

The title compound (26.0 mg, 17%) as a solid was prepared from 2,2,2-trichloroethyl pyridazin-3-ylcarbamate and 2-(2,3-difluorophenyl)-4-piperazin-1-ylpyrimidine in a manner similar to that of Example 3. Melting point: 235 - 236°C (ethyl acetate-hexane).
¹H NMR (DMSO-d₆) δ: 3.61 - 3.70 (4H, m), 3.72 - 3.82 (4H, m), 6.90 (1H, d, J = 6.2 Hz ), 7.26 - 7.36 (1H, m), 7.48 - 7.63 (2H, m), 7.79 - 7.87 (1H, m), 8.02 (1H, dd, J = 9.0, 1.3 Hz), 8.38 (1H, d, J = 6.2 Hz), 8.86 (1H, dd, J = 4.6, 1.3 Hz), 10.00 (1H, s).

### Example 7

### 4-[2-(2,4-difluorophenyl)pyridin-4-yl]-N-pyridin-3-ylpiperazine-1-carboxamide

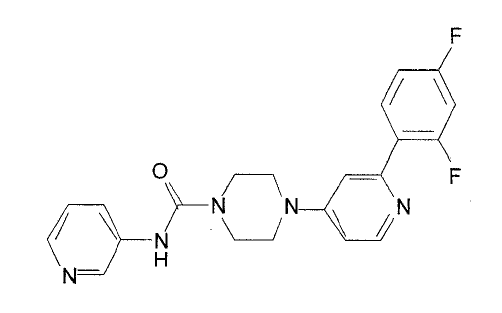

### (1) Tert-butyl 4-(2-chloropyridin-4-yl)piperazine-1-carboxylate

A mixture of 4-bromo-2-chloropyridine (3.87 ml, 34.9 mmol), tert-butyl piperazine-1-carboxylate (5.0 g, 26.9 mmol), trisdibenzylideneacetone dipalladium (492 mg, 0.537 mmol), 4,5-bis(diphenylphosphino)-9,9-dimethylxanthene (932 mg, 1.61 mmol), sodium tert-butoxide (3.87 g, 40.3 mmol) and toluene (270 ml) was stirred at 100°C for 6 hours. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (5.62 g, 70%) as a solid.
¹H NMR (CDCl₃) δ: 1.49 (9H, s), 3.30 - 3.37 (4H, m), 3.52 - 3.60 (4H, m), 6.57 (1H, dd, J = 6.1, 2.4 Hz), 6.65 (1H, d, J = 2.4 Hz), 8.04 (1H, d, J = 6.1 Hz).

### (2) Tert-butyl 4-[2-(2,4-difluorophenyl)pyridin-4-yl]piperazine-1-carboxylate

To a mixture of tert-butyl 4-(2-chloropyridin-4-yl)piperazine-1-carboxylate (2.0 g, 6.72 mmol), 2,4-difluorophenylboronic acid (1.59 g, 10.1 mmol), 2N aqueous sodium carbonate solution (27 ml) and toluene (67 ml) was added tetrakistriphenylphosphine palladium (931 mg, 0.806 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (927 mg, 37%) as an oily material.
¹H NMR (CDCl₃) δ: 1.49 (9H, s), 3.34 - 3.41 (4H, m), 3.54 - 3.63 (4H, m), 6.65 (1H, dd, J = 5.9, 2.5 Hz), 6.83 - 7.03 (2H, m), 7.08 - 7.14 (1H, m), 7.87 - 8.00 (1H, m), 8.39 (1H, d, J = 5.9 Hz).

### (3) 1-[2-(2,4-difluorophenyl)pyridin-4-yl]piperazine

To a solution of tert-butyl 4-[2-(2,4-difluorophenyl)pyridin-4-yl]piperazine-1-carboxylate (920 mg, 2.45 mmol) in ethyl acetate (9.2 ml) was added 4N hydrogen chloride-ethyl acetate solution (9.2 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (612 mg, 91%) as an oily material.
¹H NMR (CDCl₃) δ: 2.94 - 3.06 (4H, m), 3.26 - 3.39 (4H, m), 6.65 (1H, dd, J = 5.9, 2.5 Hz), 6.81 - 7.02 (2H, m), 7.11 (1H, t, J = 2.0 Hz), 7.84 - 7.99 (1H, m), 8.37 (1H, d, J = 5.9 Hz).

### (4) 4-[2-(2,4-difluorophenyl)pyridin-4-yl]-N-pyridin-3-ylpiperazine-1-carboxamide

The title compound (105 mg, 49%) as an amorphous powder was prepared from 2,2,2-trichloroethyl pyridin-3-ylcarbamate and 1-[2-(2,4-difluorophenyl)pyridin-4-yl]piperazine in a manner similar to that of Exmaple 3.
¹H NMR (DMSO-d₆) δ: 3.41 - 3.52 (4H, m), 3.56 - 3.69 (4H, m), 6.85 - 6.97 (1H, m), 7.13 - 7.40 (4H, m), 7.82 - 7.95 (2H, m), 8.16 (1H, dd, J = 4.6, 1.4 Hz), 8.31 (1H, d, J = 6.0 Hz), 8.66 (1H, d, J = 2.1 Hz), 8.82 (1H, s).

### Example 8

### 4-[2-(2,4-difluorophenyl)pyridin-4-yl]-N-(3,4-dimethylisoxazol-5-yl)piperazine-1-carboxamide

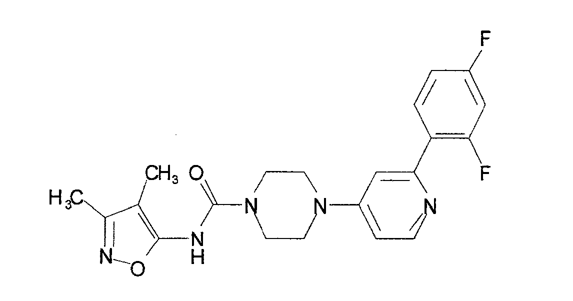

The title compound (84.6 mg, 56%) as a solid was prepared from 2,2,2-trichloroethyl (3,4-dimethylisoxazol-5-yl)carbamate and 1-[2-(2,4-difluorophenyl)pyridin-4-yl]piperazine in a manner similar to that of Example 3. Melting point: 234 - 235°C (ethyl acetate-hexane)
¹H NMR (DMSO-d₆) δ: 1.76 (3H, s), 2.13 (3H, s), 3.37 - 3.50 (4H, m), 3.52 - 3.66 (4H, m), 6.89 (1H, dd, J = 6.0, 2.4 Hz), 7.12 - 7.24 (2H, m), 7.29 - 7.40 (1H, m), 7.82 - 7.93 (1H, m), 8.30 (1H, d, J = 6.0 Hz ), 9.25 (1H, s).

### Example 9

### 4-[2-(2,4-difluorophenyl)pyridin-4-yl]-N-pyridazin-3-ylpiperazine-1-carboxamide

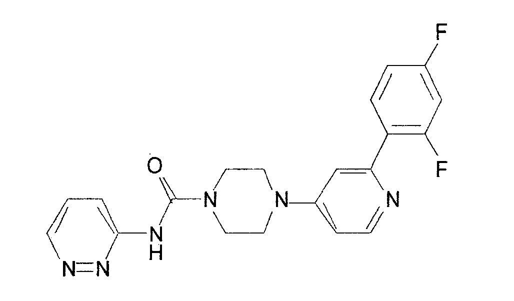

The title compound (123 mg, 57%) as a solid was prepared from 2,2,2-trichloroethyl pyridazin-3-ylcarbamate and 1-[2-(2,4-difluorophenyl)pyridin-4-yl]piperazine in a manner similar to that of Example 3. Melting point: 189 - 190°C (tetrahydrofuran-hexane).
¹H NMR (DMSO-d₆) 5: 3.40 - 3.51 (4H, m), 3.62 - 3.72 (4H, m), 6.90 (1H, dd, J = 5.9, 2.5 Hz), 7.12 - 7.25 (2H, m), 7.29 - 7.40 (1H, m), 7.58 (1H, dd, J = 9.1, 4.6 Hz), 7.82 - 8.05 (2H, m), 8.30 (1H, d, J = 5.9 Hz), 8.83 - 8.88 (1H, m), 9.99 (1H, s).

### Example 10

### 4-[5-(2,4-difluorophenyl)pyridin-3-yl]-N-pyridin-3-ylpiperazine-1-carboxamide dihydrochloride

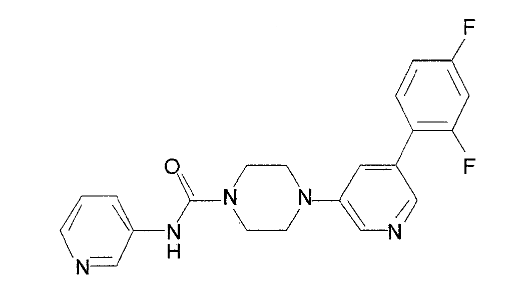

### (1) Tert-butyl 4-(5-bromopyridin-3-yl)piperazine-1-carboxylate

A mixture of 3,5-dibromopyridine (1.65 g, 6.97 mmol), tert-butyl piperazine-1-carboxylate (1.0 g, 5.37 mmol), trisdibenzylideneacetone dipalladium (98.4 mg, 0.107 mmol), 4,5-bis(diphenylphosphino)-9,9-dimethylxanthene (186 mg, 0.322 mmol), sodium tert-butoxide (774 mg, 8.06 mmol) and toluene (50 ml) was stirred at 100°C for 6 hours. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (1.46 g, 79%) as a solid.
¹H NMR (CDCl₃) δ: 1.49 (9H, s), 3.14 - 3.23 (4H, m), 3.55 - 3.63 (4H, m), 7.28 - 7.32 (1H, m), 8.15 (1H, d, J = 1.7 Hz), 8.21 (1H, d, J = 2.4 Hz).

### (2) Tert-butyl 4-[5-(2,4-difluorophenyl)pyridin-3-yl]piperazine-1-carboxylate

To a mixture of tert-butyl 4-(5-bromopyridin-3-yl)piperazine-1-carboxylate (1.0 g, 2.92 mmol), 2,4-difluorophenylboronic acid (692 mg, 4.38 mmol), 2N aqueous sodium carbonate solution (12 ml) and toluene (30 ml) was added tetrakistriphenylphosphine palladium (405 mg, 0.351 mmol) at room temperature under nitrogen atmosphere, and the mixuture was stirred at 100°C for 7 hours. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (1.03 g, 94%) as an oily material.
¹H NMR (CDCl₃) δ: 1.49 (9H, s), 3.18 - 3.26 (4H, m), 3.57 - 3.65 (4H, m), 6.88 - 7.04 (2H, m), 7.28 - 7.32 (1H, m), 7.35 - 7.46 (1H, m), 8.24 (1H, s), 8.31 (1H, d, J = 2.8 Hz).

### (3) 1-[5-(2,4-difluorophenyl)pyridin-3-yl]piperazine

To a solution of tert-butyl 4-[5-(2,4-difluorophenyl)pyridin-3-yl]piperazine-1-carboxylate (1.02 g, 10.7 mmol) in ethyl acetate (10 ml) was added 4N hydrogen chloride-ethyl acetate solution (10 ml) and the mixture was stirred at room temperature for 4 hours, and the solvent was distilled off under reduced pressure. The residue was dissolved in water and the pH was adjusted to 11 by adding 1N aqueous sodium hydroxide solution, and exracted with ethyl acetate. The extract was washed with water, dried over anhydrous magnesium sulfate, and the solvent was distilled off under reduced pressure to obtain the title compound (699 mg, 94%) as an oily material.
¹H NMR (CDCl₃) δ: 2.99 - 3.12 (4H, m), 3.16 - 3.28 (4H, m), 6.87 - 7.04 (2H, m), 7.24 - 7.33 (1H, m), 7.35 - 7.47 (1H, m), 8.21 (1H, s), 8.31 (1H, d, J = 2.8 Hz).

### (4) 4-[5-(2,4-difluorophenyl)pyridin-3-yl]-N-pyridin-3-ylpiperazine-1-carboxamide dihydrochloride

4-[5-(2,4-difluorophenyl)pyridin-3-yl]-N-pyridin-3-ylpiperazine-1-carboxamide as an oily material was prepared from 2,2,2-trichloroethyl pyridin-3-ylcarbamate and 1-[5-(2,4-difluorophenyl)pyridin-3-yl]piperazine in a manner similar to that of Example 3. 4-[5-(2,4-difluorophenyl)pyridin-3-yl]-N-pyridin-3-ylpiperazine-1-carboxamide (223.3 mg, 0.565 mmol) was dissolved in ethyl acetate (2.0 ml) and 4N hydrogen chloride-ethyl acetate solution (2.0 ml) was added and stirred at room temperature for 1 hour, and the solvent was distilled off under reduced pressure. The residue was recrystallized from methanol and diethylether to obtain the title compound (203.2 mg, 60%) as a solid. Melting point: 46 - 47°C.
¹H-NMR (DMSO-d₆) δ: 3.51 - 3.66 (4H, m), 3.71 - 3.85 (4H, m), 7.24 - 7.39 (1H, m), 7.45 - 7.60 (1H, m), 7.77 - 7.90 (1H, m), 7.96 (1H, dd, J = 8.7, 5.5 Hz), 8.16 (1H, s), 8.38 (1H, s), 8.46 - 8.57 (2H, m), 8.70 - 8.80 (1H, m), 9.22 (1H, d, J = 2.3 Hz), 10.28 (1H, s).

### Example 11

### 4-[5-(2,4-difluorophenyl)pyridin-3-yl]-N-(3,4-dimethylisoxazol-5-yl)piperazine-1-carboxamide

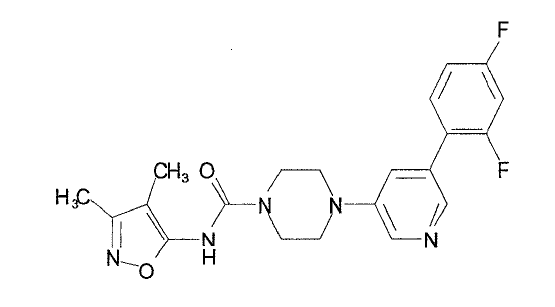

The title compound (181 mg, 60%) as a solid was prepared from 2,2,2-trichloroethyl (3,4-dimethylisoxazol-5-yl)carbamate and 1-[5-(2,4-difluorophenyl)pyridin-3-yl]piperazine in a manner similar to that of Example 3. Melting point: 147 - 148°C (tetrahydrofuran-hexane).
¹H NMR (DMSO-d₆) δ: 1.76 (3H, s), 2.13 (3H, s), 3.25 - 3.38 (4H, m), 3.53 - 3.69 (4H, m), 7.17 - 7.30 (1H, m), 7.34 - 7.52 (2H, m), 7.61 - 7.74 (1H, m), 8.16 (1H, br s), 8.38 (1H, d, J = 1.9 Hz ), 9.26 (1H, s).

### Example 12

### 4-[5-(2,4-difluorophenyl)pyridin-3-yl]-N-pyridazin-3-ylpiperazine-1-carboxamide

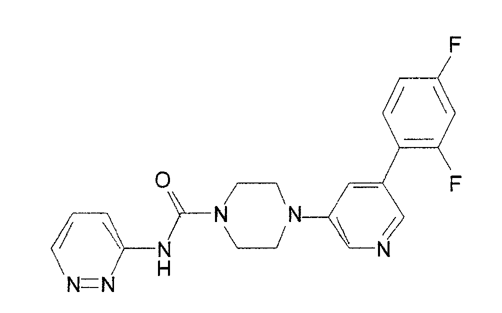

The title compound (79.4 mg, 22%) as a solid was prepared from 2,2,2-trichloroethyl pyridazin-3-ylcarbamate and 1-[5-(2,4-difluorophenyl)pyridin-3-yl]piperazine in a manner similar to that of Example 3. Melting point: 172 - 173°C (ethyl acetate-hexane).
¹H NMR (DMSO-d₆) δ: 3.23 - 3.42 (4H, m), 3.60 - 3.76 (4H, m), 7 .17 - 7.29 (1H, m), 7 .35 - 7.51 (2H, m), 7.54 - 7.74 (2H, m), 7.97 - 8.07 (1H, m), 8.16 (1H, s), 8.38 (1H, d, J = 2.6 Hz), 8.80 - 8.90 (1H, m), 10.01 (1H, s).

### Example 13

### 4-[6-(2,4-difluorophenyl)pyrazin-2-yl]-N-pyridin-3-ylpiperazine-1-carboxamide

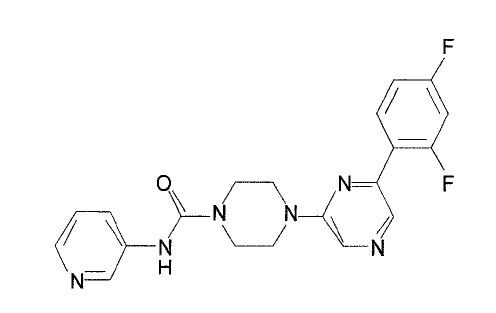

### (1) Tert-butyl 4-(6-chloropyrazin-2-yl)piperazine-1-carboxylate

A mixture of 2,6-dichloropyrazine (520 mg, 3.49 mmol), tert-butyl piperazine-1-carboxylate (500 mg, 2.69 mmol), trisdibenzylideneacetone dipalladium (49.2 mg, 0.054 mmol), 4,5-bis(diphenylphosphino)-9,9-dimethylxanthene (93.2 mg, 0.161 mmol), sodium tert-butoxide (387 mg, 4.03 mmol) and toluene (27 ml) was stirred at 100°C for 6 hours. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (385 mg, 48%) as a solid.
¹H NMR (CDCl₃) δ: 1.49 (9H, s), 3.51 - 3.65 (8H, m), 7.84 (1H, s), 7.98 (1H, s).

### (2) Tert-butyl 4-[6-(2,4-difluorophenyl)pyrazin-2-yl]piperazine-1-carboxylate

To a mixture of tert-butyl 4-(6-chloropyrazin-2-yl)piperazine-1-carboxylate (370 mg, 1.24 mmol), 2,4-difluorophenylboronic acid (293 mg, 1.86 mmol), 2N aqueous sodium carbonate solution (4.9 ml) and toluene (12 ml) was added tetrakistriphenylphosphine palladium (172 mg, 0.149 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (375 mg, 81%) as an oily material.
¹H NMR (CDCl₃) 5: 1.50 (9H, s), 3.55 - 3.70 (8H, m), 6.85 - 7.06 (2H, m), 7.95 - 8.06 (1H, m), 8.11 (1H, s), 8.40 (1H, d, J = 2.8 Hz).

### (3) 2-(2,4-difluorophenyl)-6-piperazin-1-ylpyrazine

To a solution of tert-butyl 4-[6-(2,4-difluorophenyl)pyrazin-2-yl]piperazine-1-carboxylate (370 mg, 0.983 mmol) in ethyl acetate (3.7 ml) was added 4N hydrogen chloride-ethyl acetate solution (3.7 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (204 mg, 75%) as an oily material.
¹H NMR (CDCl₃) δ: 2.96 - 3.07 (4H, m), 3.58 - 3.69 (4H, m), 6.85 - 7.04 (2H, m), 7.97 - 8.07 (1H, m), 8.09 (1H, s), 8.37 (1H, d, J = 2.8 Hz).

### (4) 4-[6-(2,4-difluorophenyl)pyrazin-2-yl]-N-pyridin-3-ylpiperazine-1-carboxamide

The title compound (53.4 mg, 37%) as a solid was prepared from 2,2,2-trichloroethyl pyridin-3-ylcarbamate and 2-(2,4-difluorophenyl)-6-piperazin-1-ylpyrazine in a manner similar to that of Example 3. Melting point: 155 - 156°C (ethyl acetate-hexane).
¹H NMR (DMSO-d₆) δ: 3.58 - 3.68 (4H, m), 3.69 - 3.77 (4H, m), 7.21 - 7.32 (2H, m), 7.37 - 7.47 (1H, m), 7.86 - 7.93 (1H, m), 7.99 - 8.11 (1H, m), 8.13 - 8.20 (1H, m), 8.27 (1H, d, J = 3.0 Hz), 8.40 (1H, s), 8.66 (1H, d, J = 2.3 Hz), 8.82 (1H, s).

### Example 14

### 4-[6-(2,4-difluorophenyl)pyrazin-2-yl]-N-(3,4-dimethylisoxazol-5-yl)piperazine-1-carboxamide

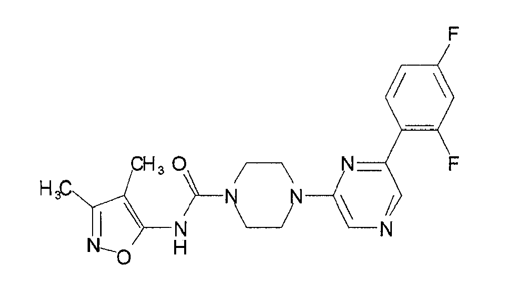

The title compound (51.9 mg, 36%) as a solid was prepared from 2,2,2-trichloroethyl (3,4-dimethylisoxazol-5-yl)carbamate and 2-(2,4-difluorophenyl)-6-piperazin-1-ylpyrazine in a manner similar to that of Example 3. Melting point: 225 - 226°C (tetrahydrofuran-hexane).
¹H NMR (DMSO-d₆) δ: 1.76 (3H, s), 2.13 (3H, s), 3.56 - 3.64 (4H, m), 3.67 - 3.75 (4H, m), 7.20 - 7.32 (1H, m), 7.36 - 7.48 (1H, m), 7.98 - 8.12 (1H, m), 8.27 (1H, d, J = 3.0 Hz ), 8.38 (1H, s), 9.26 (1H, s).

### Example 15

### 4-[4-(2,4-difluorophenyl)pyrimidin-2-yl]-N-pyridin-3-ylpiperazine-1-carboxamide

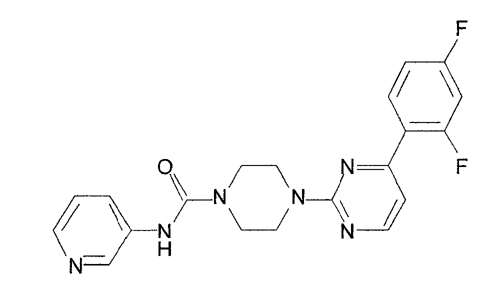

### (1) Tert-butyl 4-methylpiperazine-1-carboxylate

To a solution of 1-methylpiperazine (1.0 g, 9.98 mmol) and triethylamine (1.53 ml, 11.0 mmol) in tetrahydrofuran (20 ml) was added di-tert-butyl dicarbonate (2.40 g, 11.0 mmol) at room temperature, and the mixture was stirred at room temperature for 8 hours. The reaction was poured into water and extracted with ethyl acetate. The extract was washed with water, dried over anhydrous magnesium sulfate, and the solvent was distilled off under reduced pressure to obtain the title compound as an oily material. The resulting crude product was used for the subsequent reaction as such.

### (2) Tert-butyl 4-(4-chloropyrimidin-2-yl)piperazine-1-carboxylate

A mixture of tert-butyl 4-methylpiperazine-1-carboxylate (2.0 g, 9.98 mmol), 2,4-dichloropyrimidine (1.49 g, 9.98 mmol) and toluene (20 ml) was stirred at 110°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (1.83 g, 62%) as a solid.
¹H NMR (CDCl₃) δ: 1.49 (9H, s), 3.44 - 3.53 (4H, m), 3.75 - 3.84 (4H, m), 6.53 (1H, d, J = 5.1 Hz), 8.16 (1H, d, J = 5.1 Hz).

### (3) Tert-butyl 4-[4-(2,4-difluorophenyl)pyrimidin-2-yl]piperazine-1-carboxylate

To a mixture of tert-butyl 4-(4-chloropyrimidin-2-yl)piperazine-1-carboxylate (1.0 g, 3.35 mmol), 2,4-difluorophenylboricboronic acid (793 mg, 5.02 mmol), 2N aqueous sodium carbonate solution (13.4 ml) and toluene (33 ml) was added tetrakistriphenylphosphine palladium (464 mg, 0.402 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (894 mg, 71%) as an oily material.
¹H NMR (CDCl₃) δ: 1.50 (9H, s), 3.46 - 3.58 (4H, m), 3.85 - 3.93 (4H, m), 6.83 - 7.08 (3H, m), 8.08 - 8.22 (1H, m), 8.39 (1H, d, J = 5.3 Hz).

### (4) 4-(2,4-difluorophenyl)-2-piperazin-1-ylpyrimidine

To a solution of tert-butyl 4-[4-(2,4-difluorophenyl)pyrimidin-2-yl]piperazine-1-carboxylate (890 mg, 2.36 mmol) in ethyl acetate (9.0 ml) was added 4N hydrogen chloride-ethyl acetate solution (9.0 ml) and the mixture was stirred at room temperature for 4 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (523 mg, 80%) as a solid.
¹H NMR (CDCl₃) δ: 2.94 - 3.00 (4H, m), 3.83 - 3.90 (4H, m), 6. 83 - 7.03 (3H, m), 8.10 - 8.21 (1H, m), 8.37 (1H, d, J = 4.9 Hz).

### (5) 4-[4-(2,4-difluorophenyl)pyrimidin-2-yl]-N-pyridin-3-ylpiperazine-1-carboxamide

The title compound (77.0 mg, 36%) as a solid was prepared from 2,2,2-trichloroethyl pyridin-3-ylcarbamate and 4-(2,4-difluorophenyl)-2-piperazin-1-ylpyrimidine in a manner similar to that of Example 3. Melting point: 206 - 207°C (ethyl acetate-hexane).
¹H NMR (DMSO-d₆) δ: 3.49 - 3.70 (4H, m), 3.76 - 4.00 (4H, m), 6.98 - 7.15 (1H, m), 7.20 - 7.36 (2H, m), 7.36 - 7.51 (1H, m), 7.82 - 7.97 (1H, m), 8.07 - 8.25 (2H, m), 8.50 (1H, d, J = 4.5 Hz), 8.66 (1H, br s), 8.82 (1H, br s).

### Example 16

### 4-[4-(2,4-difluorophenyl)pyrimidin-2-yl]-N-(3,4-dimethylisoxazol-5-yl)piperazine-1-carboxamide

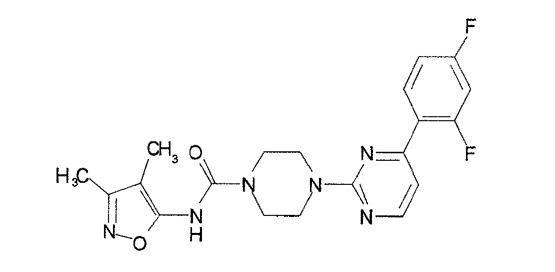

The title compound (66.4 mg, 30%) as a solid was prepared from 2,2,2-trichloroethyl (3,4-dimethylisoxazol-5-yl)carbamate and 4-(2,4-difluorophenyl)-2-piperazin-1-ylpyrimidine in a manner similar to that of Example 3. Melting point: 197 - 198°C (ethyl acetate-hexane).
¹H NMR (DMSO-d₆) δ: 1.76 (3H, s), 2.13 (3H, s), 3.50 - 3.61 (4H, m), 3.79 - 3.90 (4H, m), 7.06 (1H, dd, J = 5.1, 2.5 Hz ), 7.22 - 7.32 (1H, m), 7.36 - 7.50 (1H, m), 8.09 - 8.22 (1H, m), 8.50 (1H, d, J = 5.1 Hz ), 9.26 (1H, s).

### Example 17

### 4-[4-(2,4-difluorophenyl)pyrimidin-2-yl]-N-pyridazin-3-ylpiperazine-1-carboxamide

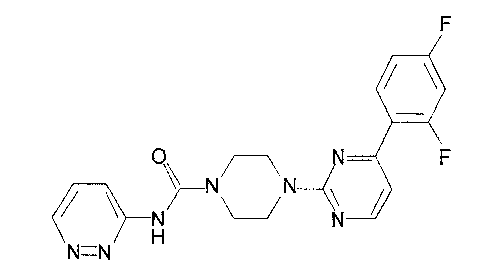

The title compound (31.6 mg, 22%) as a solid was prepared from 2,2,2-trichloroethyl pyridazin-3-ylcarbamate and 4-(2,4-difluorophenyl)-2-piperazin-1-ylpyrimidine in a manner similar to that of Example 3. Melting point: 184 - 185°C (ethyl acetate-hexane).
¹H NMR (DMSO-d₆) 5: 3.60 - 3.68 (4H, m), 3.82 - 3.91 (4H, m), 7.06 (1H, dd, J = 5.1, 2.5 Hz), 7.23 - 7.32 (1H, m), 7.38 - 7.47 (1H, m), 7.58 (1H, dd, J = 9.1, 5.1 Hz), 8.02 (1H, dd, J = 9.1, 1.5 Hz), 8.11 - 8.21 (1H, m), 8.50 (1H, d, J = 5.1 Hz), 8.85 (1H, dd, J = 4.5, 1.5 Hz ), 9.97 (1H, s).

### Example 18

### 4-[6-(2,4-difluorophenyl)pyrimidin-4-yl]-N-pyridin-3-ylpiperazine-1-carboxamide

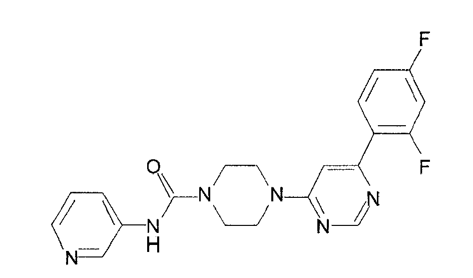

### (1) Tert-butyl 4-(6-chloropyrimidin-4-yl)piperazine-1-carboxylate

A mixture of 4,6-dichloropyrimidine (2.08 g, 14.0 mmol), tert-butyl piperazine-1-carboxylate (2.0 g, 10.7 mmol), trisdibenzylideneacetone dipalladium (197 mg, 0.215 mmol), 4,5-bis(diphenylphosphino)-9,9-dimethylxanthene (373 mg, 0.644 mmol), sodium tert-butoxide (1.55 g, 16.1 mmol) and toluene (100 ml) was stirred at 100°C for 6 hours. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (2.31 g, 72%) as a solid.
¹H NMR (CDCl₃) δ: 1.49 (9H, s), 3.50 - 3.57 (4H, m), 3.61 - 3.70 (4H, m), 6.50 (1H, s), 8.39 (1H, s).

### (2) Tert-butyl 4-[6-(2,4-difluorophenyl)pyrimidin-4-yl]piperazine-1-carboxylate

To a mixture of tert-butyl 4-(6-chloropyrimidin-4-yl)piperazine-1-carboxylate (1.0 g, 3.35 mmol), 2,4-difluorophenylboronic acid (793 mg, 5.02 mmol), 2N aqueous sodium carbonate solution (13.4 ml) and toluene (33 ml) was added tetrakistriphenylphosphine palladium (464 mg, 0.402 mmol) at room temperature under nitrogen atmosphere, and the mixutre was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (906 mg, 72%) as a solid.
¹H NMR (CDCl₃) δ: 1.50 (9H, s), 3.52 - 3.60 (4H, m), 3.68 - 3.75 (4H, m), 6.86 - 7.06 (3H, m), 8.05 - 8.16 (1H, m), 8.70 (1H, d, J = 1.1 Hz).

### (3) 4-(2,4-difluorophenyl)-6-piperazin-1-ylpyrimidine

To a solution of tert-butyl 4-[6-(2,4-difluorophenyl)pyrimidin-4-yl]piperazine-1-carboxylate (900 mg, 2.39 mmol) in ethyl acetate (9.0 ml) was added 4N hydrogen chloride-ethyl acetate solution (9.0 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (551 mg, 83%) as an oily material.
¹H NMR (CDCl₃) δ: 2. 91 - 3.03 (4H, m), 3. 63 - 3.74 (4H, m), 6.83 - 7.06 (3H, m), 8.01 - 8.15 (1H, m), 8.68 (1H, s).

### (4) 4-[6-(2,4-difluorophenyl)pyrimidin-4-yl]-N-pyridin-3-ylpiperazine-1-carboxamide

The title compound (120 mg, 56%) as a solid was prepared from 2,2,2-trichloroethyl pyridin-3-ylcarbamate and 4-(2,4-difluorophenyl)-6-piperazin-1-ylpyrimidine in a manner similar to that of Example 3. Melting point: 171 - 172°C (ethyl acetate-hexane).
¹H NMR (DMSO-d₆) δ: 3.54 - 3.65 (4H, m), 3.69 - 3.82 (4H, m), 7.16 (1H, s), 7.19 - 7.32 (2H, m), 7.36 - 7.47 (1H, m), 7.85 - 7.93 (1H, m), 7.94 - 8.05 (1H, m), 8.16 (1H, dd, d, J = 4.6, 1.4 Hz), 8.61 - 8.68 (2H, m), 8.82 (1H, s).

### Example 19

### 4-[6-(2,4-difluorophenyl)pyrimidin-4-yl]-N-(3,4-dimethylisoxazol-5-yl)piperazine-1-carboxamide

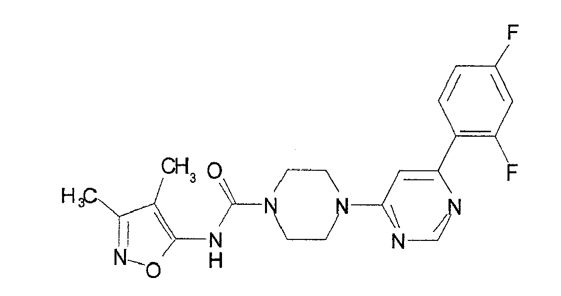

The title compound (112 mg, 50%) as a solid was prepared from 2,2,2-trichloroethyl (3,4-dimethylisoxazol-5-yl)carbamate and 4-(2,4-difluorophenyl)-6-piperazin-1-ylpyrimidine in a manner similar to that of Example 3. Melting point: 198 - 199°C (ethyl acetate-hexane).
¹H NMR (DMSO-d₆) δ: 1.76 (3H, s), 2.13 (3H, s), 3.52 - 3.60 (4H, m), 3.68 - 3.81 (4H, m), 7.15 (1H, s), 7.19 - 7.29 (1H, m), 7.37 - 7.47 (1H, m), 7.93 - 8.04 (1H, m), 8.63 (1H, d, J = 0.9 Hz ), 9.25 (1H, s).

### Example 20

### 4-[6-(2,4-difluorophenyl)pyrimidin-4-yl]-N-pyridazin-3-ylpiperazine-1-carboxamide

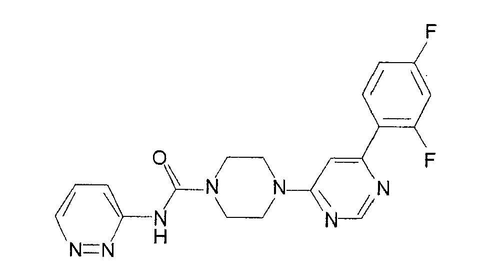

The title compound (21.2 mg, 10%) as a solid was prepared from 2,2,2-trichloroethyl pyridazin-3-ylcarbamate and 4-(2,4-difluorophenyl)-6-piperazin-1-ylpyrimidine in a manner similar to that of Example 3. Melting point: 249 - 250°C (tetrahydrofuran-hexane).
¹H NMR (DMSO-d₆) δ: 3.60 - 3.69 (4H, m), 3.71 - 3.80 (4H, m), 7.16 (1H, s), 7.20 - 7.29 (1H, m), 7.36 - 7.47 (1H, m), 7.59 (1H, dd, J = 9.1, 4.6 Hz), 7.94 - 8.05 (2H, m), 8.63 (1H, d, J = 1.4 Hz), 8.85 (1H, dd, J = 4.6, 1.4 Hz ), 9.98 (1H, s).

### Example 21

### 4-[6-(2,3-difluorophenyl)pyrimidin-4-yl]-N-pyridin-3-ylpiperazine-1-carboxamide

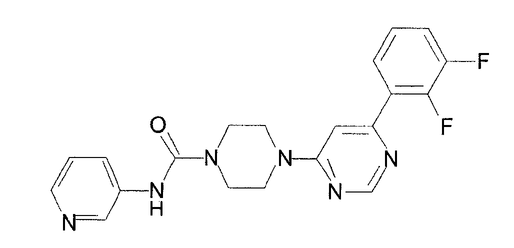

### (1) Tert-butyl 4-[6-(2,3-difluorophenyl)pyrimidin-4-yl]piperazine-1-carboxylate

To a mixture of tert-butyl 4-(6-chloropyrimidin-4-yl)piperazine-1-carboxylate (1.24 g, 4.15 mmol), 2,3-difluorophenylboronic acid (983 mg, 6.23 mmol), 2N aqueous sodium carbonate solution (17 ml) and toluene (40 ml) was added tetrakistriphenylphosphine palladium (576 mg, 0.498 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 3:7) to obtain the title compound (290 mg, 19%) as a solid.
¹H NMR (CDCl₃) δ: 1.49 (9H, s), 3.52 - 3.61 (4H, m), 3.68 - 3.76 (4H, m), 6.97 - 7.02 (1H, m), 7.15 - 7.30 (2H, m), 7.75 - 7.85 (1H, m), 8.71 (1H, d, J = 1.1 Hz).

### (2) 4-(2,3-difluorophenyl)-6-piperazin-1-ylpyrimidine

To a solution of tert-butyl 4-[6-(2,3-difluorophenyl)pyrimidin-4-yl]piperazine-1-carboxylate (280 mg, 0.744 mmol) in ethyl acetate (2.8 ml) was added 4N hydrogen chloride-ethyl acetate solution (2.8 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (168 mg, 82%) as an oily material.
¹H NMR (CDCl₃) δ: 2.93 - 3.02 (4H, m), 3.64 - 3.74 (4H, m), 6.99 (1H, s), 7.13 - 7.30 (2H, m), 7.74 - 7.83 (1H, m), 8.69 (1H, d, J = 1.1 Hz).

### (3) 4-[6-(2,3-difluorophenyl)pyrimidin-4-yl]-N-pyridin-3-ylpiperazine-1-carboxamide

The title compound (50.8 mg, 42%) as a solid was prepared from 2,2,2-trichloroethyl pyridin-3-ylcarbamate and 4-(2,3-difluorophenyl)-6-piperazin-1-ylpyrimidine in a manner similar to that of Example 3. Melting point: 179 - 180°C (tetrahydrofuran-hexane).
¹H NMR (DMSO-d₆) δ: 3.55 - 3.66 (4H, m), 3.72 - 3.83 (4H, m), 7.21 (1H, s), 7.24 - 7.40 (2H, m), 7.50 - 7.63 (1H, m), 7.65 - 7.74 (1H, m), 7.85 - 7.94 (1H, m), 8.16 (1H, dd, J = 4.6, 1.4 Hz), 8.61 - 8.69 (2H, m), 8.83 (1H, s).

### Example 22

### 4-[6-(2,3-difluorophenyl)pyrimidin-4-yl]-N-(3,4-dimethylisoxazol-5-yl)piperazine-1-carboxamide

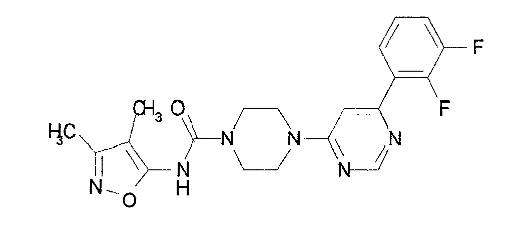

The title compound (81.3 mg, 65%) as a solid was prepared from 2,2,2-trichloroethyl (3,4-dimethylisoxazol-5-yl)carbamate and 4-(2,3-difluorophenyl)-6-piperazin-1-ylpyrimidine. Melting point: 210 - 211°C (tetrahydrofuran-hexane).
¹H NMR (DMSO-d₆) δ: 1.76 (3H, s), 2.13 (3H, s), 3.50 - 3.63 (4H, m), 3.69 - 3.83 (4H, m), 7.20 (1H, s), 7.30 - 7.40 (1H, m), 7.51 - 7.63 (1H, m), 7.65 - 7.74 (1H, m), 8.65 (1H, s ), 9.26 (1H, s).

### Example 23

### 4-[6-(2-fluorophenyl)pyrimidin-4-yl]-N-pyridin-3-ylpiperazine-1-carboxamide

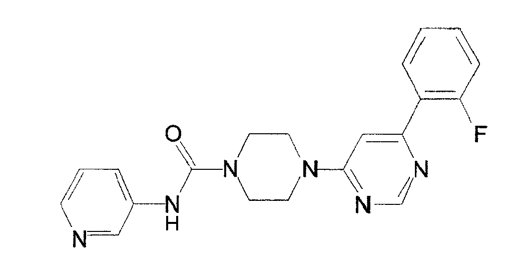

### (1) Tert-butyl 4-[6-(2-fluorophenyl)pyrimidin-4-yl]piperazine-1-carboxylate

To a mixture of tert-butyl 4-(6-chloropyrimidin-4-yl)piperazine-1-carboxylate (1.2 g, 4.02 mmol), 2-fluorophenylboronic acid (843 mg, 6.02 mmol), 2N aqueous sodium carbonate solution (16 ml) and toluene (40 ml) was added tetrakistriphenylphosphine palladium (557 mg, 0.482 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (1.28 g, 89%) as an oily material.
¹H NMR (CDCl₃) δ: 1.49 (9H, s), 3.53 - 3.59 (4H, m), 3.68 - 3.75 (4H, m), 7.03 (1H, s), 7.11 - 7.20 (1H, m), 7.23 - 7.31 (1H, m), 7.36 - 7.46 (1H, m), 8.01 - 8.08 (1H, m), 8.69 - 8.74 (1H, m).

### (2) 4-(2-fluorophenyl)-6-piperazin-1-ylpyrimidine

To a solution of tert-butyl 4-[6-(2-fluorophenyl)pyrimidin-4-yl]piperazine-1-carboxylate (1.28 g, 3.57 mmol) in ethyl acetate (12 ml) was added 4N hydrogen chloride-ethyl acetate solution (12 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (819 mg, 89%) as an oily material.
¹H NMR (CDCl₃) δ: 2.92 - 3.01 (4H, m), 3.62 - 3.75 (4H, m), 7.01 (1H, s), 7.09 - 7.20 (1H, m), 7.21 - 7.30 (1H, m), 7.35 - 7.46 (1H, m), 7.98 - 8.07 (1H, m), 8.70 (1H, d, J = 1.1 Hz).

### (3) 4-[6-(2-fluorophenyl)pyrimidin-4-yl]-N-pyridin-3-ylpiperazine-1-carboxamide

The title compound (120 mg, 55%) as a solid was prepared from 2,2,2-trichloroethyl pyridin-3-ylcarbamate and 4-(2-fluorophenyl)-6-piperazin-1-ylpyrimidine in a manner similar to that of Example 3. Melting point: 178 - 179°C (ethyl acetate-hexane).
¹H NMR (DMSO-d₆) δ: 3.56 - 3.66 (4H, m), 3.70 - 3.83 (4H, m), 7.18 (1H, s), 7.24 - 7.40 (3H, m), 7.47 - 7.60 (1H, m), 7.84 - 7.96 (2H, m), 8.16 (1H, dd, d, J = 4.6, 1.4 Hz), 8.61 - 8.69 (2H, m), 8.83 (1H, s).

### Example 24

### 4-[6-(2-fluorophenyl)pyrimidin-4-yl]-N-(3,4-dimethylisoxazol-5-yl)piperazine-1-carboxamide

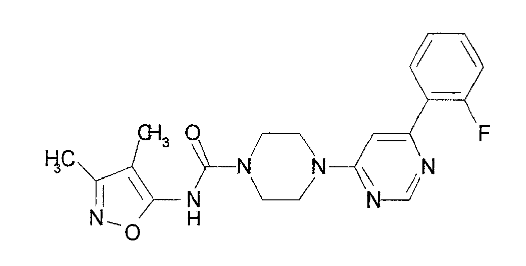

The title compound (168 mg, 73%) as a solid was prepared from 2,2,2-trichloroethyl (3,4-dimethylisoxazol-5-yl)carbamate and 4-(2-fluorophenyl)-6-piperazin-1-ylpyrimidine in a manner similar to that of Example 3. Melting point: 189 - 190°C (ethyl acetate-hexane).
¹H NMR (DMSO-d₆) δ: 1.76 (3H, s), 2.13 (3H, s), 3.51 - 3.62 (4H, m), 3.69 - 3.80 (4H, m), 7.17 (1H, s), 7.30 - 7.39 (2H, m), 7.49 - 7.58 (1H, m), 7.85 - 7.96 (1H, m), 8.64 (1H, d, J = 0.9 Hz ), 9.26 (1H, s).

### Example 25

### 4-[6-(2-fluorophenyl)pyrimidin-4-yl]-N-pyridazin-3-ylpiperazine-1-carboxamide

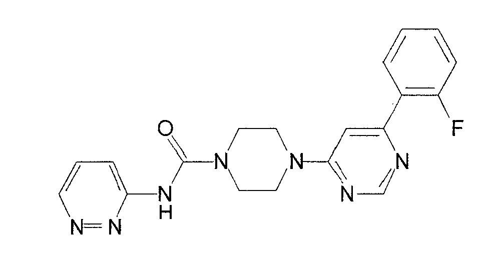

The title compound (92.7 mg, 42%) as a solid was prepared from 2,2,2-trichloroethyl pyridazin-3-ylcarbamate and 4-(2-fluorophenyl)-6-piperazin-1-ylpyrimidine in a manner similar to that of Example 3. Melting point: 207 - 208°C (tetrahydrofuran-hexane).
¹H NMR (DMSO-d₆) δ: 3.59 - 3.70 (4H, m), 3.71 - 3.82 (4H, m), 7.17 (1H, s), 7.29 - 7.41 (2H, m), 7.48 - 7.64 (2H, m), 7.88 - 7.96 (1H, m), 8.02 (1H, dd, J = 9.0, 1.3 Hz), 8.64 (1H, d, J = 0.9 Hz), 8.85 (1H, dd, J = 4.6, 1.3 Hz ), 9.99 (1H, s).

### Example 26

### 4-[6-(3-fluorophenyl)pyrimidin-4-yl]-N-pyridin-3-ylpiperazine-1-carboxamide

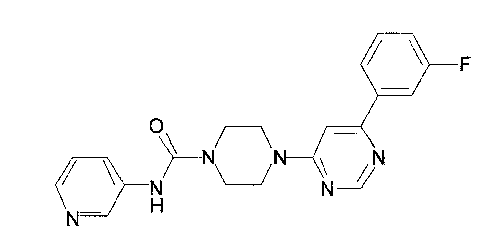

### (1) Tert-butyl 4-[6-(3-fluorophenyl)pyrimidin-4-yl]piperazine-1-carboxylate

To a mixture of tert-butyl 4-(6-chloropyrimidin-4-yl)piperazine-1-carboxylate (930 mg, 3.12 mmol), 3-fluorophenylboronic acid (656 mg, 4.68 mmol), 2N aqueous sodium carbonate solution (13 ml) and toluene (30 ml) was added tetrakistriphenylphosphine palladium (433 mg, 0.375 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (373 mg, 33%) as an oily material.
¹H NMR (CDCl₃) δ: 1.50 (9H, s), 3.52 - 3.61 (4H, m), 3.69 - 3.78 (4H, m), 6.85 (1H, d, J = 1.1 Hz), 7.10 - 7.21(1H, m), 7.38 - 7.49 (1H, m), 7.65 - 7.80 (2H, m), 8.70 (1H, d, J = 1.1 Hz).

### (2) 4-(3-fluorophenyl)-6-piperazin-1-ylpyrimidine

To a solution of tert-butyl 4-[6-(3-fluorophenyl)pyrimidin-4-yl]piperazine-1-carboxylate (370 mg, 1.03 mmol) in ethyl acetate (3.7 ml) was added 4N hydrogen chloride-ethyl acetate solution (3.7 ml) and the mixture was stirred at room temperature for 6 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (264 mg, 99%) as an oily material.
¹H NMR (CDCl₃) δ: 2.92 - 3.05 (4H, m), 3.65 - 3.77 (4H, m), 6.85 (1H, d, J = 1.1 Hz), 7.09 - 7.21 (1H, m), 7.37 - 7.49 (1H, m), 7.65 - 7.79 (2H, m), 8.68 (1H, d, J = 1.1 Hz).

### (3) 4-[6-(3-fluorophenyl)pyrimidin-4-yl]-N-pyridin-3-ylpiperazine-1-carboxamide

The title compound (81.0 mg, 43%) as a solid was prepared from 2,2,2-trichloroethyl pyridin-3-ylcarbamate and 4-(3-fluorophenyl)-6-piperazin-1-ylpyrimidine in a manner similar to that of Exmaple 3. Melting point: 205 - 206°C (ethyl acetate-hexane).
¹H NMR (DMSO-d₆) 5: 3.54 - 3.68 (4H, m), 3.76 - 3.89 (4H, m), 7.24 - 7.38 (2H, m), 7.44 (1H, s), 7.51 - 7.61 (1H, m), 7.87 - 7.94 (1H, m), 7.97 - 8.09 (2H, m), 8.17 (1H, dd, J = 4.5, 1.5 Hz ), 8.63 (1H, s), 8.67 (1H, d, J = 2.7 Hz), 8.84 (1H, s).

### Example 27

### 4-[6-(3-fluorophenyl)pyrimidin-4-yl]-N-(3,4-dimethylisoxazol-5-yl)piperazine-1-carboxamide

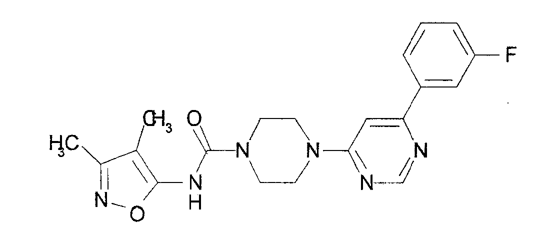

The title compound (92.2 mg, 46%) as a solid was prepared from 2,2,2-trichloroethyl (3,4-dimethylisoxazol-5-yl)carbamate and 4-(3-fluorophenyl)-6-piperazin-1-ylpyrimidine in a manner similar to that of Exmaple 3. Melting point: 185 - 186°C (ethyl acetate-hexane).
¹H NMR (DMSO-d₆) δ: 1.77 (3H, s), 2.13 (3H, s), 3.49 - 3.63 (4H, m), 3.72 - 3.88 (4H, m), 7.29 - 7.39 (1H, m), 7.44 (1H, s), 7.50 - 7.60 (1H, m), 7.96 - 8.10 (2H, m), 8.62 (1H, s ), 9.27 (1H, s).

### Example 28

### 4-[6-(4-fluorophenyl)pyrimidin-4-yl]-N-pyridin-3-ylpiperazine-1-carboxamide

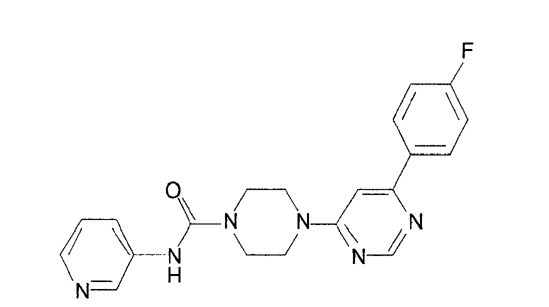

### (1) Tert-butyl 4-[6-(4-fluorophenyl)pyrimidin-4-yl]piperazine-1-carboxylate

To a mixture of tert-butyl 4-(6-chloropyrimidin-4-yl)piperazine-1-carboxylate (1.2 g, 4.03 mmol), 4-fluorophenylboronic acid (843 mg, 6.04 mmol), 2N aqueous sodium carbonate solution (16 ml) and toluene (40 ml) was added tetrakistriphenylphosphine palladium (557 mg, 0.484 mmol) at room temperature under nitrogen atmosphere, and the mixture was stirred at 100°C overnight. The reaction was poured into water and extracted with ethyl acetate. The extract was washed with water, dried over anhydrous magnesium sulfate, and the solvent was distilled off under reduced pressure. The residue was purified by silica gel column chromatography (ethyl acetate:hexane = 1:1) to obtain the title compound (714 mg, 50%) as an oily material.
¹H NMR (CDCl₃) δ: 1.50 (9H, s), 3.52 - 3.60 (4H, m), 3.69 - 3.77 (4H, m), 6.83 (1H, d, J = 0.9 Hz), 7.11 - 7.20 (2H, m), 7.93 - 8.02 (2H, m), 8.68 (1H, d, J = 0.9 Hz).

### (2) 4-(4-fluorophenyl)-6-piperazin-1-ylpyrimidine

To a solution of tert-butyl 4-[6-(4-fluorophenyl)pyrimidin-4-yl]piperazine-1-carboxylate (710 mg, 1.98 mmol) in ethyl acetate (7.0 ml) was added 4N hydrogen chloride-ethyl acetate solution (7.0 ml) and the mixture was stirred at room temperature for 5 hours, and the solvent was distilled off under reduced pressure. The residue was dissolved in water and the pH was adjusted to 11 by adding 1N aqueous sodium hydroxide solution, and extracted with ethyl acetate. The extract was washed with water, dried over anhydrous magnesium sulfate, and the solvent was distilled off under reduced pressure to obtain the title compound (456 mg, 89%) as a solid.
¹H NMR (CDCl₃) δ: 2.93 - 3.02 (4H, m), 3.66 - 3.75 (4H, m), 6.82 (1H, d, J = 1.1 Hz), 7.09 - 7.20 (2H, m), 7.91 - 8.02 (2H, m), 8.67 (1H, d, J = 1.1 Hz).

### (3) 4-[6-(4-fluorophenyl)pyrimidin-4-yl]-N-pyridin-3-ylpiperazine-1-carboxamide

The title compound (142 mg, 65%) as a solid was prepared from 2,2,2-trichloroethyl pyridin-3-ylcarbamate and 4-(4-fluorophenyl)-6-piperazin-1-ylpyrimidine in a manner similar to that of Example 3. Melting point: 180 - 182°C (ethyl acetate-hexane).
¹H NMR (DMSO-d₆) δ: 3.54 - 3.67 (4H, m), 3.74 - 3.88 (4H, m), 7.24 - 7.41 (4H, m), 7.85 - 7.94 (1H, m), 8.17 (1H, dd, J = 4.6, 1.4 Hz ), 8.21 - 8.31 (2H, m), 8.61 (1H, d, J = 0.8 Hz), 8.67 (1H, d, J = 2.4 Hz), 8.83 (1H, s).

### Example 29

### 4-[6-(4-fluorophenyl)pyrimidin-4-yl]-N-(3,4-dimethylisoxazol-5-yl)piperazine-1-carboxamide

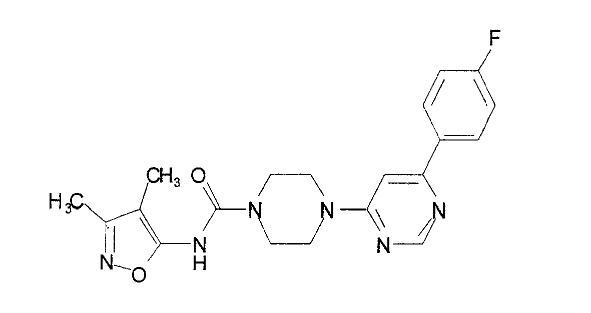

The title compound (151 mg, 66%) as a solid was prepared from 2,2,2-trichloroethyl (3,4-dimethylisoxazol-5-yl)carbamate and 4-(4-fluorophenyl)-6-piperazin-1-ylpyrimidine in a manner similar to that of Example 3. Melting point: 207 - 208°C (ethyl acetate-hexane).
¹H NMR (DMSO-d₆) δ: 1.77 (3H, s), 2.13 (3H, s), 3.50 - 3.63 (4H, m), 3.73 - 3.86 (4H, m), 7.27 - 7.41 (3H, m), 8.19 - 8.31 (2H, m), 8.60 (1H, d, J = 0.8 Hz ), 9.27 (1H, s).

### Example 30

### 4-[6-(4-fluorophenyl)pyrimidin-4-yl]-N-pyridazin-3-ylpiperazine-1-carboxamide

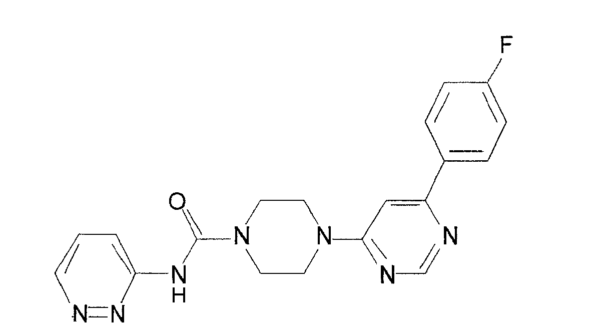

The title compound (85.7 mg, 58%) as a solid was prepared from 2,2,2-trichloroethyl pyridazin-3-ylcarbamate and 4-(4-fluorophenyl)-6-piperazin-1-ylpyrimidine in a manner similar to that of Example 3. Melting point: 254 - 255°C (tetrahydrofuran-hexane).
¹H NMR (DMSO-d₆) δ: 3.59 - 3.70 (4H, m), 3.75 - 3.86 (4H, m), 7.28 - 7.42 (3H, m), 7.59 9 (1H, dd, J = 9.0, 4.7 Hz), 8.02 (1H, dd, J = 9.0, 1.3 Hz), 8.20 - 8.31 (2H, m), 8.60 (1H, d, J = 0.8 Hz), 8.86 (1H, dd, J = 4.7, 1.3 Hz ), 10.0 (1H, s).

### Example 31

### 4-(2',4'-difluorobiphenyl-3-yl)-N-pyridin-3-ylpiperazine-1-carboxamide

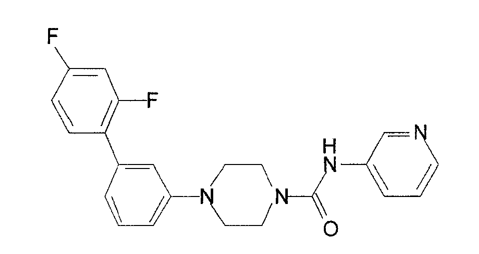

### (1) Tert-butyl 4-(3-bromophenyl)piperazine-1-carboxylate

To a solution of 1-(3-bromophenyl)piperazine (30.00 g, 0.125 mol) and di-tert-butyl dicarbonate (27.30 g, 0.125 mol) in methylene chloride (200 ml) was added dropwise triethylamine (27.3 g, 0.125 mol) at 0°C, and the mixture was stirred at room temperature for 3 hours. The reaction was poured into water and extracted with methylene chloride. The extract was washed with aqueous saturated sodium hydrogen carbonate solution, dried over anhydrous sodium sulfate, and the solvent was distilled off under reduced pressure. The residue was recrystallized from hexane to obtain the title compound (42.0 g, 99%).
¹H NMR (CDCl₃) δ: 1.48 (9H, s), 3.12 - 3.15 (4H, m), 3.56 - 3.58 (4H, m), 6.84 (1H, d, J = 6.8 Hz), 6.99 (1H, d, J = 7.2 Hz), 7.05 (1H, s), 7.09 - 7.11(1H, m).

### (2) 1-(2',4'-difluorobiphenyl-3-yl)piperazine

To a solution of tert-butyl 4-(3-bromophenyl)piperazine-1-carboxylate (2.00 g, 7.30 mmol) and 2,4-difluorophenylboronic acid (1.30 g, 10.2 mmol) in 1,2-dimethoxyethane-water (20 ml, V_{DME}: V_{H2O} = 10:1) was added sodium carbonate (1.25 g, 14.6 mmol) and tetrakistriphenylphosphine palladium (340 mg, 0.360 mmol) at room temperature under nitrogen atmosphere and heated under reflux for 4 hours. The reaction was cooled and poured into water, and extracted with ethyl acetate. The extract was washed with aqueous saturated sodium hydrogen carbonate solution, dried over anhydrous sodium sulfate, and the solvent was distilled off under reduced pressure. The residue was purified by silica gel column chromatography (petroleum ether:ethyl acetate = 10:1) to obtain tert-butyl 4-(2',4'-difluorobiphenyl-3-yl)piperazine-1-carboxylate (2.10 g, 95%).

A solution of tert-butyl 4-(2',4'-difluorobiphenyl-3-yl)piperazine-1-carboxylate (2.10 g, 5.60 mmol) in trifluoroacetic acid-methylene chloride (1:2) was stirred at room temperature for 3 hours and the reaction was distilled off under reduced pressure. The residue was neutralized by adding aqueous saturated sodium hydrogen carbonate solution and extracted with methylene chloride. The extract was dried over anhydrous sodium sulfate and the solvent was distilled off under reduced pressure. The residue was recrystallized from hexane to obtain the title compound (1.45 g, 97%).
¹H NMR (CDCl₃) δ: 3.00 - 3.02 (4H, m), 3.13 - 3.15 (4H, m), 6.70 - 6.79 (3H, m), 6.83 - 6.86 (2H, m), 7.18 - 7.25 (2H, m).

### (3) 4-(2',4'-difluorobiphenyl-3-yl)-N-pyridin-3-ylpiperazine-1-carboxamide

To a solution of 1-(2',4'-difluorobiphenyl-3-yl)piperazine (200 mg, 0.730 mmol) and triethylamine (147 mg, 1.46 mmol) in tetrahydrofuran (6.0 ml) was added 3-pyridine isocyanate (88 mg, 0.730 mmol) at 0°C under nitrogen atmosphere and the mixture was stirred at room temperature for 4 hours, and the solvent was distilled off under reduced pressure. The residue was recrystallized from ethyl acetate and diethylether to obtain the title compound (160 mg, 56%) as a solid.
¹H NMR (DMSO-d₆) δ: 3.22 - 3.24 (4H, m), 3.61 - 3.63 (4H, m), 6.94 (1H, d, J = 7.6 Hz), 7.05 - 7.07 (2H, m), 7.17 (1H, t, J = 8.0 Hz), 7.26 (1H, dd, J = 8.4, 4.4 Hz), 7.31 - 7.36 (2H, m), 7.58 (1H, q, J = 6.8 Hz), 7.88 (1H, d, J = 8.4 Hz), 8.15 (1H, d, J = 4.4 Hz), 8.65 (1H, d, J = 2.4 Hz), 8.80 (1H, br s).

### Example 32

### 4-(2',4'-difluorobiphenyl-3-yl)-N-(3,4-dimethylisoxazol-5-yl)piperazine-1-carboxamide

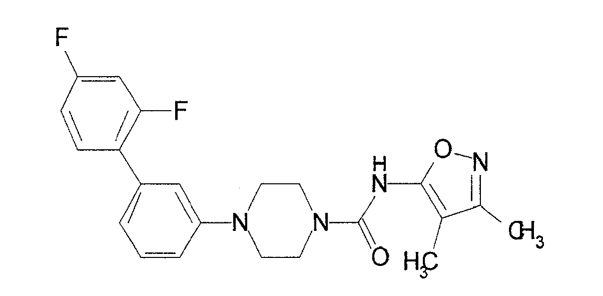

The title compound (132 mg, 44%) as a solid was prepared from 2,2,2-trichloroethyl (3,4-dimethylisoxazol-5-yl)carbamate and 1-(2',4'-difluorobiphenyl-3-yl)piperazine in a manner similar to that of Example 3.
¹H NMR (DMSO-d₆) δ: 1.76 (3H, s), 2.13 (3H, s), 3.21 - 3.24 (4H, m), 3.58 - 3.61 (4H, m), 6.95 (1H, d, J = 7.6 Hz), 7.03 - 7.07 (2H, m), 7.18 (1H, t, J = 2.4 Hz), 7.32 - 7.37 (2H, m), 7.58 (1H, q, J = 3.6 Hz), 9.25 (1H, br s).

### Example 33

### 4-(2',4'-difluorobiphenyl-3-yl)-N-pyridazin-3-ylpiperazine-1-carboxamide

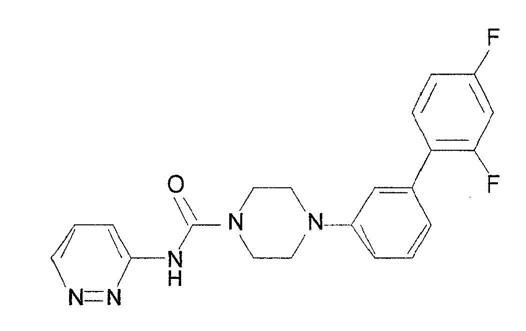

The title compound (175 mg, 61%) as a solid was prepared from 2,2,2-trichloroethyl pyridazin-3-ylcarbamate and 1-(2',4'-difluorobiphenyl-3-yl)piperazine in a manner similar to that of Exmaple 3.
¹H NMR (DMSO-d₆) δ: 3.20 - 3.23 (4H, m), 3.64 - 3.67 (4H, m), 6.93 (1H, d, J = 7.2 Hz), 7.01 - 7.05 (2H, m), 7.16 (1H, t, J = 2.4 Hz), 7.29 - 7.35 (2H, m), 7.54 - 7.58 (2H, m), 7.99 (1H, d, J = 8.8 Hz), 8.82 (1H, dd, J = 4.4, 0.8 Hz), 9.95 (1H, br s).

### Example 34

### 4-(2',4'-difluorobiphenyl-3-yl)-N-phenylpiperazine-1-carboxamide

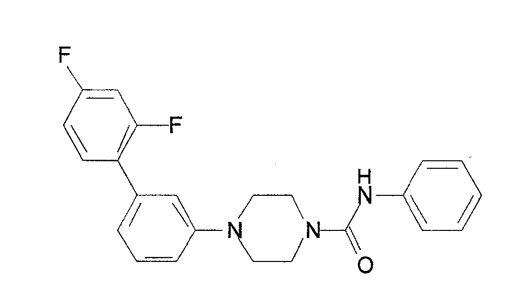

The title compound (160 mg, 56%) as a solid was prepared from 1-(2',4'-difluorobiphenyl-3-yl)piperazine and phenyl isocyanate in a manner similar to that of Example 31. ¹H NMR (DMSO-d₆) δ: 3.21 - 3.23 (4H, m), 3.59 - 3.61 (4H, m), 6.91 - 6.95 (2H, m), 7.02 - 7.06 (2H, m), 7.17 - 7.25 (3H, m), 7.30 - 7.36 (2H, m), 7.46 (2H, d, J = 8.0 Hz), 7.57 (1H, q, J = 6.8 Hz), 8.60 (1H, br s).

### Example 35

### 4-(2',4'-difluorobiphenyl-3-yl)-N-(3-methylisoxazol-5-yl)piperazine-1-carboxamide

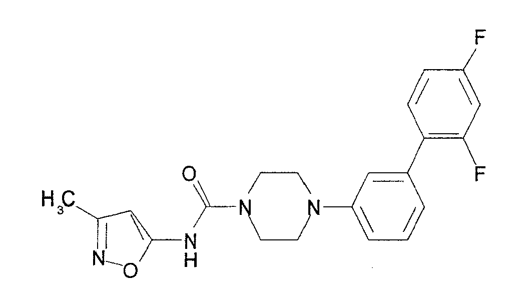

The title compound (160 mg, 55%) as a solid was prepared from 2,2,2-trichloroethyl (3-methylisoxazol-5-yl)carbamate and 1-(2',4'-difluorobiphenyl-3-yl)piperazine in a manner similar to that of Example 3.
¹H NMR (DMSO-d₆) δ: 2.13 (3H, s), 3.18 - 3.21 (4H, m), 3.59 - 3.62 (4H, m), 5.92 (1H, s), 6.93 (1H, d, J = 7.2 Hz), 7.00 - 7.05 (2H, m), 7.16 (1H, dt, J = 5.4, 2.4 Hz), 7.29 - 7.35 (2H, m), 7.54 (1H, q, J = 8.0 Hz), 10.29 (1H, br s).

### Example 36

### 4-(2',4'-difluorobiphenyl-3-yl)-N-pyrazin-2-ylpiperazine-1-carboxamide

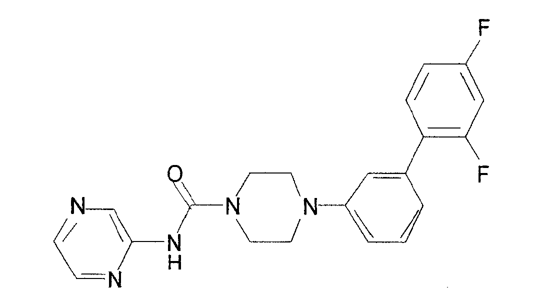

To a solution of pyrazine-2-carboxylic acid (12.4 g, 0.100 mol) and triethylamine (26.3 g, 0.26 mol) in tetrahydrofuran (125 ml) was added dropwise diphenylphosphorylazide (36 g, 0.13 mol) at 0°C under nitrogen atmosphere and the mixture was stirred at 0°C for 30 minutes, followed by at room temperature for 2 hours, and the reaction solution was distilled off under reduced pressure. The residue was dissolved in toluene (25 ml) and the mixture was stirred at 85°C for 10 minutes, and the solvent was distilled off under reduced pressure to obtain 2-pyrazine isocyanate. The resulting 2-pyrazine isocyanate was used for the subsequent reaction as such.

To a solution of 1-(2',4'-difluorobiphenyl-3-yl)piperazine (200 mg, 0.73 mmol) and triethylamine (147 mg, 1.46 mmol) in tetrahydrofuran (6 ml) was added dropwise a solution of 2-pyrazine isocyanate (2.00 g) in tetrahydrofuran (2 ml) at 0°C under nitrogen atmosphere, and the mixture was stirred at room temperature for 14 hours. The reaction was poured into water and extracted with ethyl acetate. The extract was washed with saturated brine, dried over anhydrous sodium sulfate, and the solvent was distilled off under reduced pressure. The residue was purified by high performance liquid chromatography (YMC HPLC column, solution A: 0.1% trifluoroacetic acid-acetonitrile solution, solution B: 0.1% aqueous trifluoroacetic acid solution) to obtain the title compound (95.0 mg, 33%).
¹H NMR (DMSO-d₆) δ: 3.21 - 3.24 (4H, m), 3.64 - 3.67 (4H, m), 6.94 (1H, d, J = 7.6 Hz), 7.02 - 7.06 (2H, m), 7.17 (1H, t, J = 2.0 Hz),7.30 - 7.34 (2H, m), 7.57 - 7.59 (1H, m), 8.21 (1H, d, J = 2.8 Hz), 8.30 (1H, dd, J = 2.4, 1.2 Hz), 9.04 (1H, d, J = 1.2 Hz), 9.63 (1H, br s).

### Example 37

### 4-(2',4'-difluorobiphenyl-3-yl)-N-(1-methyl-1H-pyrazol-5-yl)piperazine-1-carboxamide

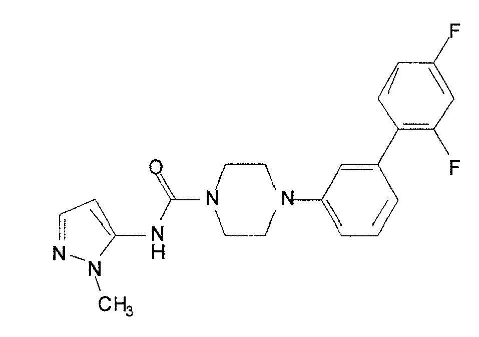

The title compound (170 mg, 59%) as a solid was prepared from 2,2,2-trichloroethyl (1-methyl-1H-pyrazol-5-yl)carbamate and 1-(2',4'-difluorobiphenyl-3-yl)piperazine in a manner similar to that of Example 3.
¹H NMR (DMSO-d₆) δ: 3.03 - 3.11 (4H, m), 3.51 - 3.65 (7H, m), 5.99 9 (1H, d, J = 2.0 Hz), 6.93 (1H, d, J = 7.2 Hz), 7.01 - 7.05 (2H, m), 7.14 (1H, dt, J = 8.4, 2.4 Hz), 7.28-7.36 (3H, m), 7.54 - 7.60 (1H, m), 8.60 (1H, br s).

### Example 38

### 4-(2',3'-difluorobiphenyl-3-yl)-N-pyridin-3-ylpiperazine-1-carboxamide

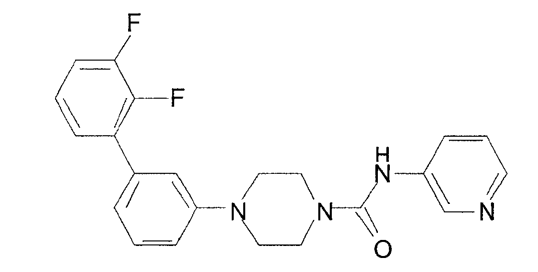

### (1) 1-(2',3'-difluorobiphenyl-3-yl)piperazine

To a mixture of tert-butyl 4-(3-bromophenyl)piperazine-1-carboxylate (3.00 g, 10.9 mmol), 2,3-difluorophenylboronic acid (1.75 g, 15.3 mmol) in 1,2-dimethoxyethane-water (30 ml, V_{DME}:V_{H2O} = 10:1) was added sodium carbonate (1.87 g, 21.9 mmol) and tetrakistriphenylphosphine palladium (520 mg, 0.550 mmol) at room temperature under nitrogen atmosphere and heated under reflux for 4 hours. The reaction was cooled and poured into water, and extracted with ethyl acetate. The extract was washed with aqueous saturated sodium hydrogen carbonate solution, dried over anhydrous sodium sulfate, and the solvent was distilled off under reduced pressure. The residue was purified by silica gel column chromatography (petroleum ether:ethyl acetate = 10:1) to obtain tert-butyl 4-(2',3'-difluorobiphenyl-3-yl)piperazine-1-carboxylate (2.86 g, 86%).

A solution of tert-butyl 4-(2',3'-difluorobiphenyl-3-yl)piperazine-1-carboxylate (2.86 g, 7.65 mmol) in trifluoroacetic acid-methylene chloride (1:2) was stirred at room temperature for 5 hour and the reaction was distilled off under reduced pressure. The residue was neutralized by adding aqueous saturated sodium hydrogen carbonate solution, and extracted with methylene chloride. The extract was dried over anhydrous sodium sulfate and the solvent was distilled off under reduced pressure. The residue was recrystallized from diethylether to obtain the title compound (2.00 g, 96%).
¹H NMR (CDCl₃) δ: 3.15 - 3.16 (4H, m), 3.41 - 3.43 (4H, m), 6.31 (1H, br s), 6.58 (1H, d, J = 8.4 Hz), 7.00 - 7.02 (2H, m), 7.03 - 7.05 (3H, m), 7.30 (1H, t, J = 8.2 Hz).

### (2) 4-(2',3'-difluorobiphenyl-3-yl)-N-pyridin-3-ylpiperazine-1-carboxamide

The title compound (110 mg, 38%) as a solid was prepared from 1-(2',3'-difluorobiphenyl-3-yl)piperazine and 3-pyridine isocyanate in a manner similar to that of Example 31.
¹H NMR (DMSO-d₆) δ: 2.99 - 3.06 (4H, m), 3.61 - 3.63 (4H, m), 6.97 (1H, d, J = 8.8 Hz), 7.06 (1H, d, J = 7.2 Hz), 7.11 (1H, s), 7.26 - 7.42 (5H, m), 7.88 (1H, d, J = 8.0 Hz), 8.14 (1H, d, J = 3.2 Hz), 8.65 (1H, s), 8.87 (1H, br s).

### Example 39

### 4-(2',3'-difluorobiphenyl-3-yl)-N-(3,4-dimethylisoxazol-5-yl)piperazine-1-carboxamide

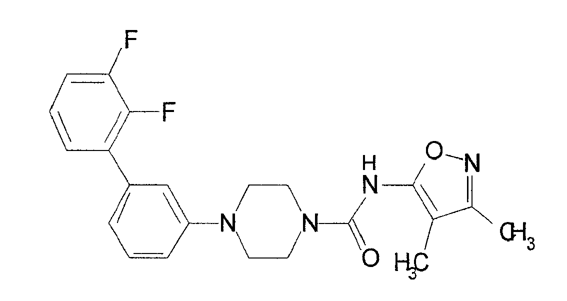

The title compound (130 mg, 43%) as a solid was prepared from 2,2,2-trichloroethyl (3,4-dimethylisoxazol-5-yl)carbamate and 1-(2',3'-difluorobiphenyl-3-yl)piperazine in a manner similar to that of Example 3.
¹H NMR (DMSO-d₆) δ: 1.74 (3H, s), 2.11 (3H, s), 3.16 - 3.22 (4H, m), 3.54 - 3.64 (4H, m), 6.96 - 7.10 (3H, m), 7.24 - 7.44 (4H, m), 9.24 (1H, br s).

### Example 40

### 4-(2',3'-difluorobiphenyl-3-yl)-N-pyridazin-3-ylpiperazine-1-carboxamide

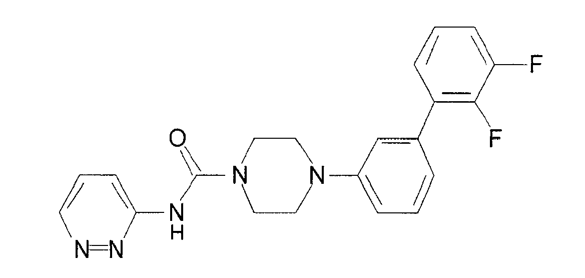

The title compound (170 mg, 59%) as a solid was prepared from 2,2,2-trichloroethyl pyridazin-3-ylcarbamate and 1-(2',3'-difluorobiphenyl-3-yl)piperazine in a manner similar to that of Example 3.
¹H NMR (DMSO-d₆) δ: 3.23 - 3.25 (4H, m), 3.66 - 3.68 (4H, m), 6.98 (1H, d, J = 7.6 Hz), 7.06 (1H, d, J = 8.4 Hz), 7.11 (1H, s), 7.27 - 7.43 (4H, m), 7.55 (1H, m), 8.00 (1H, d, J = 8.8 Hz), 8.84 (1H, d, J = 3.2 Hz), 9.95 (1H, br s).

### Example 41

### 4-(2',3'-difluorobiphenyl-3-yl)-N-phenylpiperazine-1-carboxamide

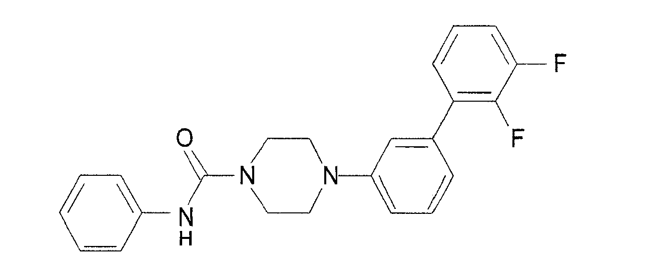

The title compound (165 mg, 58%) as a solid was prepared from 1-(2',3'-difluorobiphenyl-3-yl)piperazine and phenyl isocyanate in a manner similar to that of Example 31.
¹H NMR (DMSO-d₆) δ: 3.22 - 3.24 (4H, m), 3.59 - 3.62 (4H, m), 6.92 (1H, t, J = 7.2 Hz), 6.98 (1H, d, J = 3.6 Hz), 7.07 (1H, d, J = 8.4 Hz), 7.12 (1H, s), 7.21 (2H, t, J = 7.8 Hz), 7.29 - 7.33 (1H, m), 7.35 (2H, t, J = 8.0 Hz), 7.42 - 7.48 (3H, m), 8.60 (1H, br s).

### Example 42

### 4-(2',3'-difluorobiphenyl-3-yl)-N-(3-methylisoxazol-5-yl)piperazine-1-carboxamide

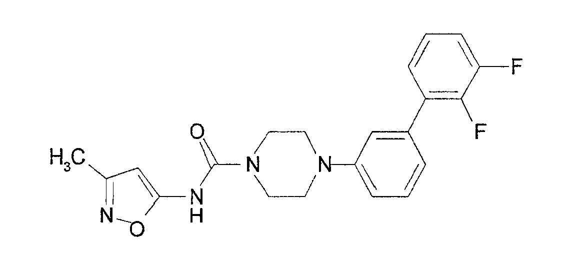

The title compound (125 mg, 43%) as a solid was prepared from 2,2,2-trichloroethyl (3-methylisoxazol-5-yl)carbamate and 1-(2',3'-difluorobiphenyl-3-yl)piperazine in a manner similar to that of Example 3.
¹H NMR (DMSO-d₆) δ: 2.13 (3H, s), 3.19 - 3.22 (4H, m), 3.59 - 3.62 (4H, m), 5.94 (1H, s), 6.98 (1H, d, J = 7.6 Hz), 7.05 (1H, d, J = 8.4 Hz), 7.10 (1H, s), 7.24 - 7.45 (4H, m), 10.29 (1H, br s).

### Example 43

### 4-(2',3'-difluorobiphenyl-3-yl)-N-pyrazin-2-ylpiperazine-1-carboxamide

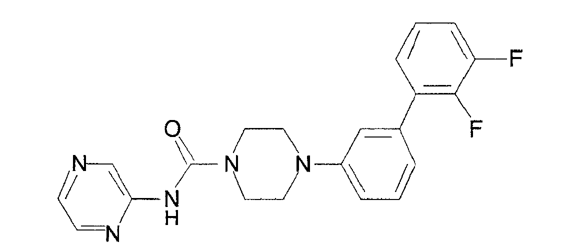

The title compound (95 mg, 33%) as a solid was prepared from 2-pyrazinecarboxylic acid and 1-(2',3'-difluorobiphenyl-3-yl)piperazine in a manner similar to that of Example 36.
¹H NMR (DMSO-d₆) δ: 3.23 - 3.25 (4H, m), 3.65 - 3.67 (4H, m), 6.98 (1H, d, J = 7.2 Hz), 7.06 (1H, d, J = 8.4 Hz), 7.11 (1H, s), 7.27 - 7.43 (4H, m), 8.21 (1H, d, J = 2.4 Hz), 8.30 (1H, s), 9.04 (1H, d, J = 1.6 Hz), 9.63 (1H, br s).

### Example 44

### 4-(2',3'-difluorobiphenyl-3-yl)-N-(1-methyl-1H-pyrazol-5-yl)piperazine-1-carboxamide

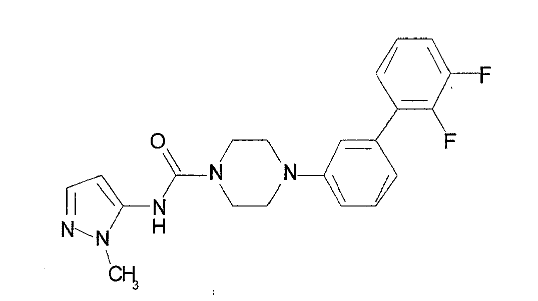

The title compound (175 mg, 61%) as a solid was prepared from 2,2,2-trichloroethyl (1-methyl-1H-pyrazol-5-yl)carbamate and 1-(2',3'-difluorobiphenyl-3-yl)piperazine in a manner similar to that of Example 3.
¹H NMR (DMSO-d₆) δ: 3.31 - 3.33 (4H, m), 3.66 - 3.68 (7H, m), 6.08 (1H, d, J = 1.6 Hz), 7.07 (1H, d, J = 7.6 Hz), 7.15 (1H, d, J = 8.0 Hz), 7.20 (1H, s), 7.34 - 7.54 (5H, m), 8.69 (1H, br s).

### Example 45

### 4-[6-(2,4-difluorophenyl)pyridin-2-yl]-N-pyridin-3-ylpiperazine-1-carboxamide

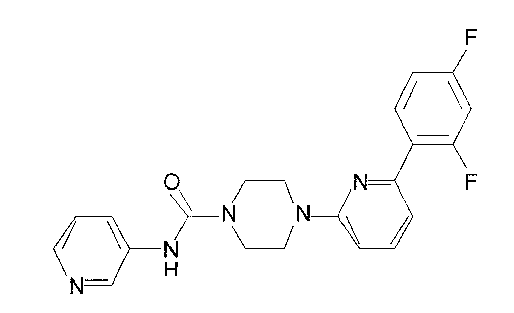

### (1) 1-[6-(2,4-difluorophenyl)pyridin-2-yl]piperazine

To a mixture of tert-butyl 4-(6-bromopyridin-2-yl)piperazine-1-carboxylate (3.00 g, 8.77 mmol) and 2,4-difluorophenylboronic acid (1.40 g, 12.3 mmol) in 1,2-dimethoxyethane-water (30 ml, V_{DME}:V_{H2O} = 10:1) was added sodium carbonate (1.25 g, 17.5 mmol) and tetrakistriphenylphosphine palladium (340 mg, 0.440 mmol) at room temperature under nitrogen atmosphere and heated under reflux for 1 hour. The reaction was cooled, poured into water, and extracted with ethyl acetate. The extract was washed with aqueous saturated sodium hydrogen carbonate solution, dried over anhydrous sodium sulfate, and the solvent was distilled off under reduced pressure. The residue was purified by silica gel column chromatography (petroleum ether:ethyl acetate = 10:1) to obtain tert-butyl 4-[6-(2,4-difluorophenyl)pyridin-2-yl]piperazine-1-carboxylate (2.84 g, 86%).

A solution of tert-butyl 4-[6-(2,4-difluorophenyl)pyridin-2-yl]piperazine-1-carboxylate (2.84 g, 7.55 mmol) in trifluoroacetic acid-methylene chloride (1:2) was stirred at room temperature for 2 hours and the reaction was distilled off under reduced pressure. The residue was neutralized by adding aqueous saturated sodium hydrogen carbonate solution, and extracted with methylene chloride. The extract was dried over anhydrous sodium sulfate and the solvent was distilled off under reduced pressure. The residue was recrystallized from hexane and diethylether to obtain the title compound (1.50 g, 72%).
¹H NMR (CDCl₃) δ: 2.97 - 3.05 (4H, m), 3.39 - 3.41 (4H, m), 6.55 (1H, d, J = 8.4 Hz), 6.82 - 6.79 (1H, m), 6.86 - 6.88 (1H, m), 7.08 (1H, dd, J = 7.6, 2.4 Hz), 7.46 (1H, t, J = 8.0 Hz), 7.95 (1H, q, J = 8.8 Hz).

### (2) 4-[6-(2,4-difluorophenyl)pyridin-2-yl]-N-pyridin-3-ylpiperazine-1-carboxamide

The title compound (220 mg, 77%) as a solid was prepared from 1-[6-(2,4-difluorophenyl)pyridin-2-yl]piperazine and 3-pyridine isocyanate in a manner similar to that of Example 31.
¹H NMR (DMSO-d₆) δ: 3.59 - 3.62 (8H, m), 6.92 (1H, d, J = 8.4 Hz), 7.09 (1H, dd, J = 7.2, 2.4 Hz), 7.20 - 7.34 (3H, m), 7.66 (1H, t, J = 8.0 Hz), 7.89 (1H, d, J = 8.4 Hz), 8.03 (1H, q, J = 8.8 Hz), 8.15 (1H, dd, J = 4.4, 1.2 Hz), 8.65 (1H, d, J = 2.4 Hz), 8.79 (1H, br s).

### Example 46

### 4-[6-(2,4-difluorophenyl)pyridin-2-yl]-N-(3,4-dimethylisoxazol-5-yl)piperazine-1-carboxamide

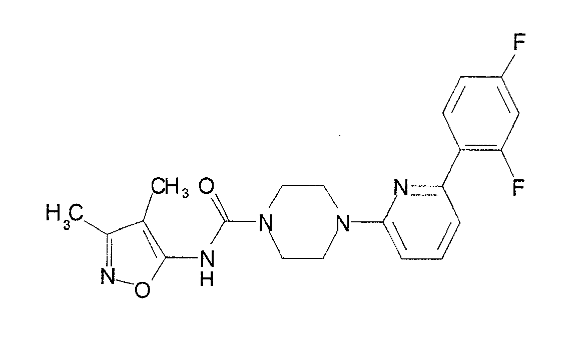

The title compound (185 mg, 62%) as a solid was prepared from 2,2,2-trichloroethyl (3,4-dimethylisoxazol-5-yl)carbamate and 1-[6-(2,4-difluorophenyl)pyridin-2-yl]piperazine in a manner similar to that of Example 3.
¹H NMR (DMSO-d₆) 5: 1.75 (3H, s), 2.12 (3H, s), 3.56 - 3.61 (8H, m), 6.90 (1H, d, J = 8.4 Hz), 7.08 (1H, dd, J = 5.0, 2.4 Hz), 7.18 (1H, dt, J = 9.6, 2.2 Hz), 7.30 - 7.36 (1H, m), 7.66 (1H, t, J = 4.0 Hz), 8.02 (1H, q, J = 8.3 Hz), 9.22 (1H, br s).

### Example 47

### 4-[6-(2,4-difluorophenyl)pyridin-2-yl]-N-pyridazin-3-ylpiperazine-1-carboxamide

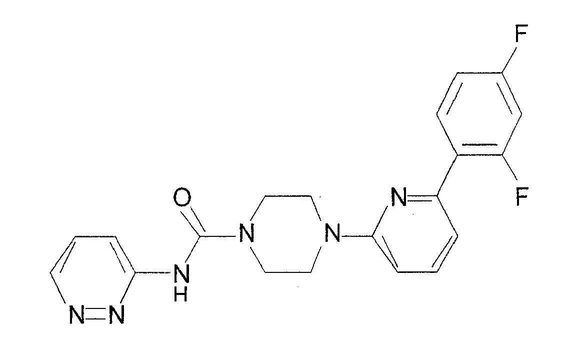

The title compound (150 mg, 52%) as a solid was prepared from 2,2,2-trichloroethyl pyridazin-3-ylcarbamate and 1-[6-(2,4-difluorophenyl)pyridin-2-yl]piperazine in a manner similar to that of Example 3.
¹H NMR (DMSO-d₆) δ: 3.62 - 3.64 (8H, m), 6.90 (1H, d, J = 9.2 Hz), 7.07 (1H, dd, J = 4.8, 2.4 Hz), 7.18 - 7.22 (1H, m), 7.30 - 7.36 (1H, m), 7.56 (1H, dd, J = 6.8, 4.4 Hz), 7.65 (1H, t, J = 8.0 Hz), 7.97 - 8.05 (2H, m), 8.83 (1H, dd, J = 3.0, 1.2 Hz), 9.94 (1H, br s).

### Example 48

### 4-[6-(2,4-difluorophenyl)pyridin-2-yl]-N-phenylpiperazine-1-carboxamide

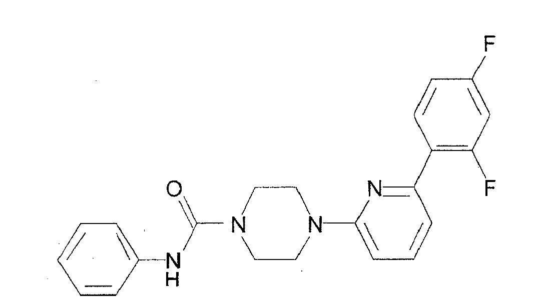

The title compound (135 mg, 47%) as a solid was prepared from 1-[6-(2,4-difluorophenyl)pyridin-2-yl]piperazine and phenyl isocyanate in a manner similar to that of Example 31.
¹H NMR (DMSO-d₆) δ: 3.58 - 3.60 (8H, m), 6.89 - 6.94 (2H, m), 7.08 (1H, dd, J = 7.2, 2.4 Hz), 7.18 - 7.24 (3H, m), 7.30 - 7.35 (1H, m), 7.46 (2H, d, J = 7.6 Hz), 7.65 (1H, t, J = 8.0 Hz), 8.02 (1H, q, J = 3.6 Hz), 8.59 (1H, br s).

### Example 49

### 4-[6-(2,4-difluorophenyl)pyridin-2-yl]-N-(3-methylisoxazol-5-yl)piperazine-1-carboxamide

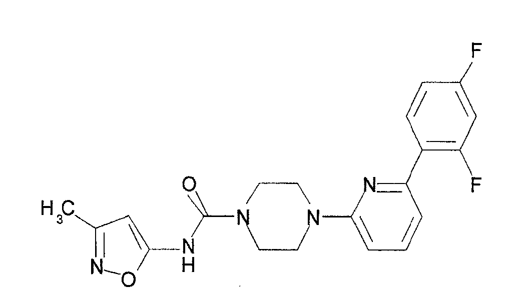

The title compound (150 mg, 52%) as a solid was prepared from 2,2,2-trichloroethyl (3-methylisoxazol-5-yl)carbamate and 1-[6-(2,4-difluorophenyl)pyridin-2-yl]piperazine in a manner similar to that of Example 3.
¹H NMR (DMSO-d₆) δ: 2.14 (3H, s), 3.58 - 3.61 (8H, m), 5.93 (1H, s), 6.90 (1H, d, J = 8.4 Hz), 7.08 (1H, dd, J = 4.8, 2.4 Hz), 7.18 (1H, dt, J = 9.6, 2.4 Hz), 7.30 - 7.36 (1H, m), 7.65 (1H, t, J = 8.0 Hz), 8.02 (1H, q, J = 8.3 Hz), 10.28 (1H, br s).

### Example 50

### 4-[6-(2,4-difluorophenyl)pyridin-2-yl]-N-pyrazin-2-ylpiperazine-1-carboxamide

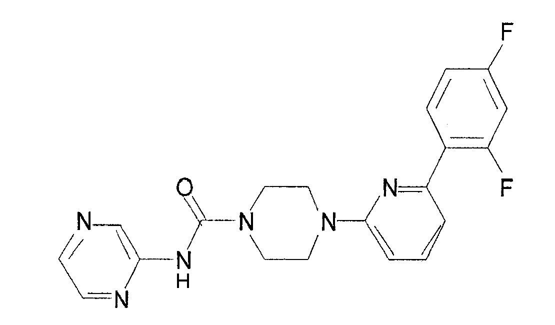

The title compound (120 mg, 42%) as a solid was prepared from 2-pyrazinecarboxylic acid and 1-[6-(2,4-difluorophenyl)pyridin-2-yl]piperazine in a manner similar to that of Example 36.
¹H NMR (DMSO-d₆) δ: 3.60 - 3.63 (8H, m), 6.90 (1H, d, J = 8.4 Hz), 7.08 (1H, dd, J = 7.4, 2.2 Hz), 7.19 (1H, dt, J = 8.4, 2.4 Hz), 7.31 (1H, dt, J = 11.8, 2.4 Hz), 7.64 (1H, t, J = 7.8 Hz), 7.99 (1H, q, J = 8.4 Hz), 8.21 (1H, d, J = 2.4 Hz), 8.30 (1H, s), 9.04 (1H, s), 9.63 (1H, br s).

### Example 51

### 4-[6-(2,4-difluorophenyl)pyridin-2-yl]-N-(1-methyl-1H-pyrazol-5-yl)piperazine-1-carboxamide

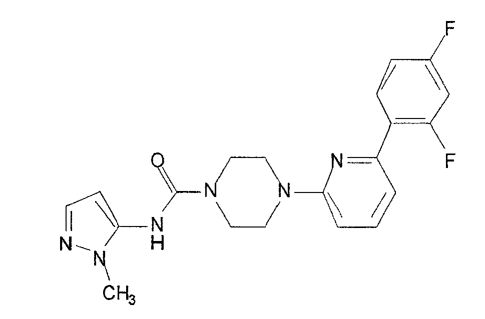

The title compound (165 mg, 57%) as a solid was prepared from 2,2,2-trichloroethyl (1-methyl-1H-pyrazol-5-yl)carbamate and 1-[6-(2,4-difluorophenyl)pyridin-2-yl]piperazine in a manner similar to that of Example 3.
¹H NMR (DMSO-d₆) δ: 3.57 - 3.62 (11H, m), 6.00 (1H, d, J = 2.0 Hz), 6.90 (1H, d, J = 8.8 Hz), 7.08 (1H, dd, J = 4.8, 2.4 Hz), 7.18 - 7.22 (1H, m), 7.29 - 7.36 (2H, m), 7.66 (1H, t, J = 7.8 Hz), 8.03 (1H, q, J = 8.4 Hz), 8.60 (1H, br s).

### Example 52

### 4-[6-(2,3-difluorophenyl)pyridin-2-yl]-N-pyridin-3-ylpiperazine-1-carboxamide

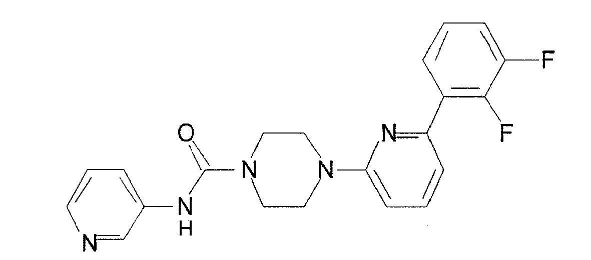

### (1) 1-[6-(2,3-difluorophenyl)pyridin-2-yl]piperazine

To a mixture of tert-butyl 4-(6-bromopyridin-2-yl) piperazine-1-carboxylate (4.00 g, 11.8 mmol) and 2,3-difluorophenylboronic acid (2.59 g, 16.5 mmol) in 1,2-dimethoxyethane-water (30 ml, V_{DME}:V_{H2O} = 10:1) was added sodium carbonate (2.49 g, 16.46 mmol) and tetrakistriphenylphosphine palladium (680 mg, 0.880 mmol) at room temperature under nitrogen atmosphere and heated under reflux for 3 hours. The reaction was cooled, poured into water, and extracted with ethyl acetate. The extract was washed with aqueous saturated sodium hydrogen carbonate solution, dried over anhydrous sodium sulfate, and the solvent was distilled off under reduced pressure. The residue was purified by silica gel column chromatography (petroleum ether:ethyl acetate = 10:1) to obtain tert-butyl 4-[6-(2,3-difluorophenyl)pyridin-2-yl]piperazine-1-carboxylate (4.06 g, 92%).

A solution of tert-butyl 4-[6-(2,3-difluorophenyl)pyridin-2-yl]piperazine-1-carboxylate (4.06 g, 10.9 mmol) in trifluoroacetic acid-methylene chloride (1:2) was stirred at room temperature for 4 hours and the reaction was distilled off under reduced pressure. The residue was neutralized by adding aqueous saturated sodium hydrogen carbonate solution, and extracted with methylene chloride. The extract was dried over anhydrous sodium sulfate and the solvent was distilled off under reduced pressure. The residue was recrystallized from hexane and diethylether to obtain the title compound (2.50 g, 84%).
¹H NMR (CDCl₃) : δ 3.22 - 3.20 (4H, m), 3.79 - 3.81 (4H, m), 6.70 (1H, d, J = 8.4 Hz), 7.16 - 7.19 (1H, m), 7.23 - 7.26 (2H, m), 7.63 (1H, t, J = 4.2 Hz), 7.71 - 7.72 (1H, m).

### (2) 4-[6-(2,3-difluorophenyl)pyridin-2-yl]-N-pyridin-3-ylpiperazine-1-carboxamide

The title compound (120 mg, 42%) as a solid was prepared from 1-[6-(2,3-difluorophenyl)pyridin-2-yl]piperazine and 3-pyridine isocyanate in a manner similar to that of Example 31.
¹H NMR (DMSO-d₆) δ: 3.61 - 3.63 (8H, m), 6.95 (1H, d, J = 8.4 Hz), 7.11 (1H, dd, J = 7.6, 2.0 Hz), 7.26 - 7.33 (2H, m), 7.46 (1H, q, J = 8.4 Hz), 7.69 (1H, t, J = 8.0 Hz), 7.75 (1H, t, J = 7.2 Hz), 7.89 (1H, d, J = 8.8 Hz), 8.15 (1H, d, J = 4.0 Hz), 8.66 (1H, s), 8.80 (1H, br s).

### Example 53

### 4-[6-(2,3-difluorophenyl)pyridin-2-yl]-N-(3,4-dimethylisoxazol-5-yl)piperazine-1-carboxamide

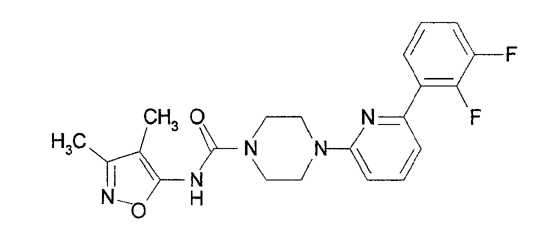

The title compound (150 mg, 50%) as a solid was prepared from 2,2,2-trichloroethyl (3,4-dimethylisoxazol-5-yl)carbamate and 1-[6-(2,3-difluorophenyl)pyridin-2-yl]piperazine in a manner similar to that of Example 3.
¹H NMR (DMSO-d₆) δ: 1.75 (3H, s), 2.12 (3H, s), 3.56 - 3.62 (8H, m), 6.94 (1H, d, J = 8.4 Hz), 7.12 (1H, dd, J = 4.8, 2.4 Hz), 7.28 - 7.33 (1H, m), 7.43 - 7.50 (1H, m), 7.66 - 7.76 (2H, m), 9.23 (1H, br s).

### Example 54

### 4-[6-(2,3-difluorophenyl)pyridin-2-yl]-N-pyridazin-3-ylpiperazine-1-carboxamide

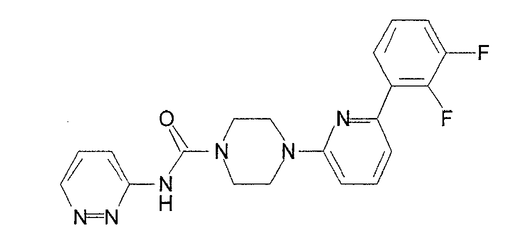

The title compound (158 mg, 55%) as a solid was prepared from 2,2,2-trichloroethyl pyridazin-3-ylcarbamate and 1-[6-(2,3-difluorophenyl)pyridin-2-yl]piperazine in a manner similar to that of Example 3.
¹H NMR (DMSO-d₆) δ: 3.61 - 3.64 (8H, m), 6.94 (1H, d, J = 8.4 Hz), 7.11 (1H, dd, J = 7.6, 2.4 Hz), 7.29 - 7.31 (1H, m), 7.45 - 7.47 (1H, m), 7.55 - 7.58 (1H, m), 7.66 - 7.76 (2H, m), 8.01 (1H, dd, J = 5.0, 1.2 Hz), 8.83 (1H, dd, J = 4.4, 1.2 Hz), 9.95 (1H, br s).

### Example 55

### 4-[6-(2,3-difluorophenyl)pyridin-2-yl]-N-phenylpiperazine-1-carboxamide

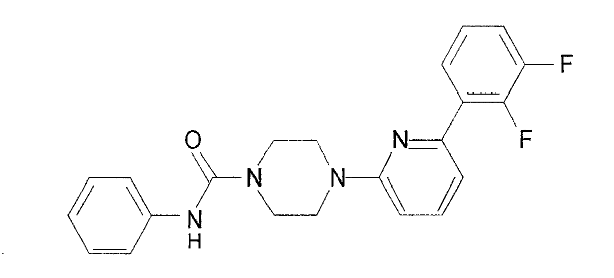

The title compound (130 mg, 45%) as a solid was prepared from 1-[6-(2,3-difluorophenyl)pyridin-2-yl]piperazine and phenyl isocyanate in a manner similar to that of Example 31.
¹H NMR (DMSO-d₆) δ: 3.58 - 3.60 (8H, m), 6.91 - 6.95 (2H, m), 7.11 (1H, d, J = 5.6 Hz), 7.22 (2H, t, J = 7.8 Hz), 7.27 - 7.32 (1H, m), 7.42 - 7.47 (3H, m), 7.66 - 7.76 (2H, m), 8.57 (1H, br s).

### Example 56

### 4-[6-(2,3-difluorophenyl)pyridin-2-yl]-N-(3-methylisoxazol-5-yl)piperazine-1-carboxamide

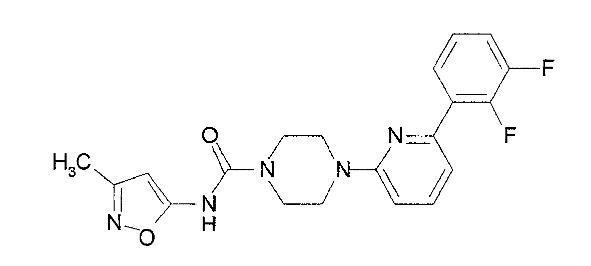

The title compound (130 mg, 45%) as a solid was prepared from 2,2,2-trichloroethyl (3-methylisoxazol-5-yl)carbamate and 1-[6-(2,3-difluorophenyl)pyridin-2-yl]piperazine in a manner similar to that of Example 3.
¹H NMR (DMSO-d₆) δ: 3.61 - 3.64 (8H, m), 6.94 (1H, d, J = 8.4 Hz), 7.11 (1H, dd, J = 7.6, 2.4 Hz), 7.29 - 7.31 (1H, m), 7.45 - 7.47 (1H, m), 7.55 - 7.58 (1H, m), 7.66 - 7.76 (2H, m), 8.01 (1H, dd, J = 5.0, 1.2 Hz), 8.83 (1H, dd, J = 4.4, 1.2 Hz), 9.95 (1H, br s).

### Example 57

### 4-[6-(2,3-difluorophenyl)pyridin-2-yl]-N-pyrazin-2-ylpiperazine-1-carboxamide

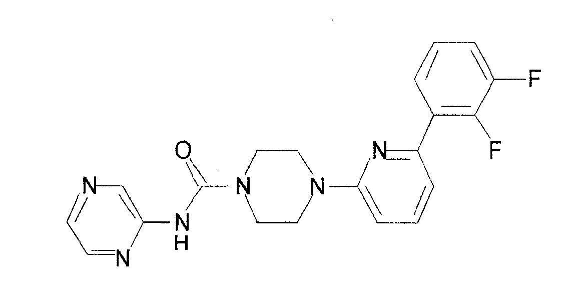

The title compound (130 mg, 45%) as a solid was prepared from 2-pyrazinecarboxylic acid and 1-[6-(2,3-difluorophenyl)pyridin-2-yl]piperazine in a manner similar to that of Example 36.
¹H NMR (DMSO-d₆) δ: 3.60 - 3.63 (8H, m), 6.94 (1H, d, J = 8.4 Hz), 7.11 (1H, dd, J = 7.6, 2.0 Hz), 7.27 - 7.31 (1H, m), 7.45 - 7.48 (1H, m), 7.67 - 7.76 (2H, m), 8.21 (1H, d, J = 2.4 Hz), 8.30 (1H, s), 9.05 (1H, s), 9.62 (1H, br s).

### Example 58

### 4-[6-(2,3-difluorophenyl)pyridin-2-yl]-N-(1-methyl-1H-pyrazol-5-yl)piperazine-1-carboxamide

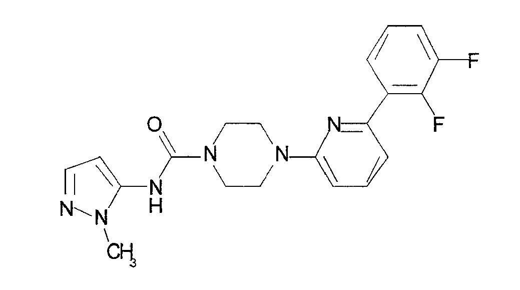

The title compound (150 mg, 52%) as a solid was prepared from 2,2,2-trichloroethyl (1-methyl-1H-pyrazol-5-yl)carbamate and 1-[6-(2,3-difluorophenyl)pyridin-2-yl]piperazine in a manner similar to that of Example 3.
¹H NMR (DMSO-d₆) δ: 3.56 - 3.62 (11H, m), 5.99 (1H, d, J = 1.6 Hz), 6.94 (1H, d, J = 8.4 Hz), 7.11 (1H, dd, J = 4.8, 2.0 Hz), 7.28 - 7.32 (2H, m), 7.45 (1H, q, J = 5.6 Hz), 7.66 - 7.76 (2H, m), 8.59 (1H, br s).

### Example 59

### 4-(5-phenylpyridin-3-yl)-N-pyridin-3-ylpiperazine-1-carboxamide

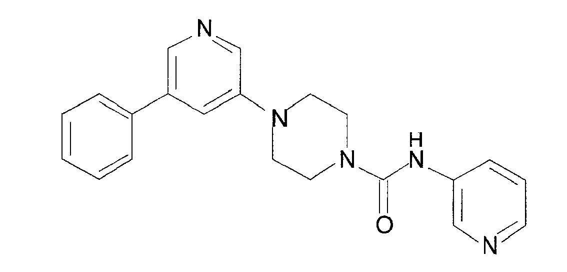

### (1) Tert-butyl 4-(5-bromopyridin-3-yl)piperazine-1-carboxylate

To a solution of 3,5-dibromopyridine (15.0 g, 63.8 mmol) and tert-butyl piperazine-1-carboxylate (11.9 g, 63.83 mmol) in anhydrous toluene (600 ml) was added sodium tert-butoxide (9.19 g, 95.8 mmol), 4,5-bis(diphenylphosphino)-9,9-dimethylxanthene (1.48 g, 2.56 mmol) and trisdibenzylideneacetone dipalladium (366 mg, 0.64 mmol) under nitrogen atmosphere and the reaction was degassed, and heated under reflux for 5 hours. The reaction was distilled off under reduced pressure, and to the residue was added ethyl acetate and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain the title compound (10.0 g, 50%).
¹H NMR (MeOD) δ: 1.47 (9H, s), 3.20 - 3.30 (4H, m), 3.50 - 3.60 (4H, m), 7.56 (1H, s), 8.02 (1H, s), 8.20 (1H, s).

### (2) 1-(5-phenylpyridin-3-yl)piperazine dihydrochloride

To a mixture of tert-butyl 4-(5-bromopyridin-3-yl)piperazine-1-carboxylate (9.00 g, 26.4 mmol) and phenylboronic acid (4.19 g, 34.3 mmol) in 1,2-dimethoxyethane-water (300 ml, V_{DME}:V_{H2O} = 10:1) was added sodium carbonate (5.59 g, 52.8 mmol) and tetrakistriphenylphosphine palladium (914 mg, 0.790 mmol) at room temperature under nitrogen atmosphere and the reaction was degassed, and heated under reflux for 10 hours. The reaction was distilled off under reduced pressure, and to the residue was added ethyl acetate and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain tert-butyl 4-(5-phenylpyridin-3-yl)piperazine-1-carboxylate (8.00 g, 90%).

A solution of tert-butyl 4-(5-phenylpyridin-3-yl)piperazine-1-carboxylate (8.00 g, 7.65 mmol) in 4N hydrogen chloride-ethyl acetate (100 ml) was stirred at room temperature for 14 hours and a solid was separated by filtration to obtain the title compound (6.00 g, 93%).
¹H NMR (DMSO-d₆) δ: 3.16 - 3.28 (4H, m), 3.71 - 3.82 (4H, m), 7.48 - 7.60 (3H, m), 7.89 (2H, d, J = 6.8 Hz), 8.27 (1H, s), 8.50 (1H, d, J = 2.4 Hz), 8.58 (1H, s), 9.54 (2H, br s).

### (3) 4-(5-phenylpyridin-3-yl)-N-pyridin-3-ylpiperazine-1-carboxamide

The title compound (185 mg, 60%) as a solid was prepared from 1-(5-phenylpyridin-3-yl)piperazine dihydrochloride and 3-pyridine isocyanate in a manner similar to that of Example 31.
¹H NMR (DMSO-d₆) δ: 3.32 - 3.43 (4H, m), 3.57 - 3.72 (4H, m), 7.22 - 7.32 (1H, m), 7.41 (1H, t, J = 7.6 Hz), 7.49 (2H, t, J = 7.4 Hz), 7.56 (1H, d, J = 2.0 Hz), 7.72 (2H, d, J = 7.4 Hz), 7.88 - 7.92 (1H, m), 8.15 (1H, dd, J = 4.6, 1.4 Hz), 8.31 (1H, d, J = 1.6 Hz), 8.35 (1H, d, J = 2.4 Hz), 8.65 (1H, d, J = 2.8 Hz), 8.83 (1H, s).

### Example 60

### N-(3,4-dimethylisoxazol-5-yl)-4-(5-phenylpyridin-3-yl)piperazine-1-carboxamide

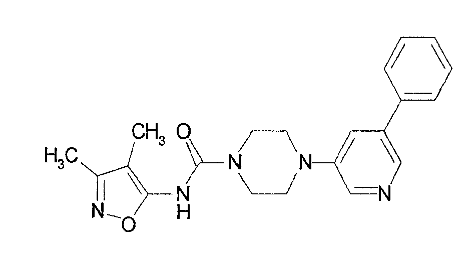

The title compound (147 mg, 49%) as a solid was prepared from 2,2,2-trichloroethyl (3,4-dimethylisoxazol-5-yl)carbamate and 1-(5-phenylpyridin-3-yl)piperazine dihydrochloride in a manner similar to that of Example 3.
¹H NMR (DMSO-d₆) δ: 1.75 (3H, s), 2.12 (3H, s), 3.29 - 3.35 (4H, m), 3.59 - 3.65 (4H, m), 7.38 - 7.42 (1H, m), 7.48 (2H, t, J = 7.4 Hz), 7.56 (1H, s), 7.72 (2H, d, J = 7.6 Hz), 8.32 (2H, dd, J = 8.0, 2.0 Hz), 9.25 (1H, s).

### Example 61

### 4-(5-phenylpyridin-3-yl)-N-pyridazin-3-ylpiperazine-1-carboxamide

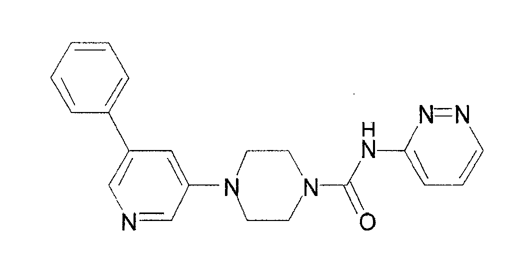

The title compound (80 mg, 21%) as a solid was prepared from 2,2,2-trichloroethyl pyridazin-3-ylcarbamate and 1-(5-phenylpyridin-3-yl)piperazine dihydrochloride in a manner similar to that of Example 3.
¹H NMR (CDCl₃) δ: 3.30 - 3.54 (4H, m), 3.64 - 3.78 (4H, m), 7.38 - 7.61 (5H, m), 7.75 (2H, d, J = 7.2 Hz), 8.03 (1H, d, J = 8.4 Hz), 8.34 (2H, d, J = 6.8 Hz), 8.86 (1H, s), 10.01 (1H, s).

### Example 62

### 4-(6-phenylpyridin-2-yl)-N-pyridin-3-ylpiperazine-1-carboxamide

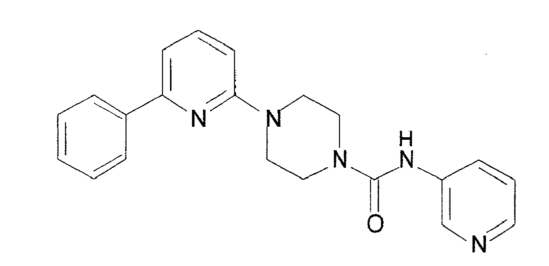

### (1) 1-(6-phenylpyridin-2-yl)piperazine dihydrochloride

To a mixture of tert-butyl 4-(6-bromopyridin-2-yl)piperazine-1-carboxylate (10.0 g, 29.3 mmol) and phenylboronic acid (5.01 g, 41.1 mmol) in 1,2-dimethoxyethane-water (300 ml, V_{DME}:V_{H2O} = 10:1) was added sodium carbonate (6.22 g, 58.7 mmol) and tetrakistriphenylphosphine palladium (1.01 g, 0.880 mmol) at room temperature under nitrogen atmosphere and the reaction was degassed, and heated under reflux for 5 hours. The reaction was distilled off under reduced pressure, and to the residue was added ethyl acetate and water followed by extracted. The extract was washed with saturated brine and dried over anhydrous sodium sulfate, and the solvent was distilled off under reduced pressure. The residue was purified by silica gel column chromatography (petroleum ether:ethyl acetate = 5:1) to obtain tert-butyl 4-(6-phenylpyridin-2-yl)piperazine-1-carboxylate (8.00 g, 70%).

A solution of tert-butyl 4-(6-phenylpyridin-2-yl)piperazine-1-carboxylate (8.00g) in 4N hydrogen chloride-ethyl acetate (100 ml) was stirred at room temperature for 14 hours and a solid was separated by filtration to obtain the title compound (5.20 g, 81%).
¹H NMR (DMSO-d₆) δ: 3.12 - 3.22 (4H, m), 3.82 - 3.90 (4H, m), 6.92 (1H, d, J = 8.4 Hz), 7.31 (1H, d, J = 7.2 Hz), 7.38 - 7.47 (3H, m), 7.71 (1H, t, J = 7.8 Hz), 8.02 (2H, d, J = 7.2 Hz), 9.64 (2H, br s).

### (2) 4-(6-phenylpyridin-2-yl)-N-pyridin-3-ylpiperazine-1-carboxamide

The title compound (110 mg, 36%) as a solid was prepared from 1-(6-phenylpyridin-2-yl)piperazine dihydrochloride and 3-pyridine isocyanate in a manner similar to that of Example 31.
¹H NMR (DMSO-d₆) δ: 3.58 - 3.73 (8H, m), 6.87 (1H, d, J = 8.4 Hz), 7.20 - 7.30 (2H, m), 7.37 - 7.50 (3H, m), 7.65 (1H, t, J = 7.8 Hz), 7.91 (1H, d, J = 8.4 Hz), 8.05 (2H, d, J = 7.6 Hz), 8.16 (1H, d, J = 4.0 Hz), 8.67 (1H, d, J = 2.0 Hz), 8.82 (1H, s).

### Example 63

### N-(3,4-dimethylisoxazol-5-yl)-4-(6-phenylpyridin-2-yl)piperazine-1-carboxamide

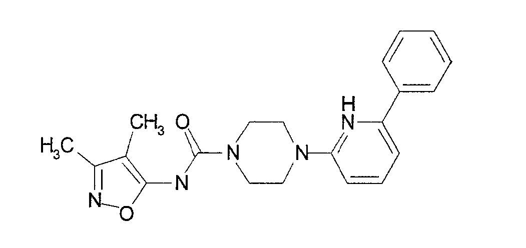

The title compound (260 mg, 84%) as a solid was prepared from 2,2,2-trichloroethyl (3,4-dimethylisoxazol-5-yl)carbamate and 1-(6-phenylpyridin-2-yl)piperazine dihydrochloride in a manner similar to that of Example 3.
¹H NMR (DMSO-d₆) δ: 1.75 (3H, s), 2.12 (3H, s), 3.54 - 3.68 (8H, m), 6.84 (1H, d, J = 8.4 Hz), 7.25 (1H, d, J = 7.2 Hz), 7.35 - 7.40 (1H, m), 7.40 - 7.48 (2H, m), 7.64 (1H, t, J = 8.0 Hz), 8.03 (2H, dd, J = 8.4, 1.6 Hz), 9.23 (1H, s).

### Example 64

### 4-(6-phenylpyridin-2-yl)-N-pyridazin-3-ylpiperazine-1-carboxamide

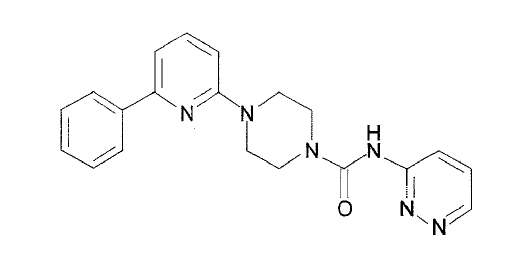

The title compound (109 mg, 35%) as a solid was prepared from 2,2,2-trichloroethyl pyridazin-3-ylcarbamate and 1-(6-phenylpyridin-2-yl)piperazine dihydrochloride in a manner similar to that of Example 3.
¹H NMR (DMSO-d₆) δ: 3.62 - 3.72 (8H, m), 6.85 (1H, d, J = 8.4 Hz), 7.25 (1H, d, J = 7.2 Hz), 7.36 - 7.42 (1H, m), 7.43 - 7.50 (2H,m), 7.57 (1H, dd, J = 8.8, 4.4 Hz), 7.64 (1H, t, J = 8.0 Hz), 7.99 - 8.07 (3H, m), 8.84 (1H, d, J = 4.4 Hz), 9.96 (1H, s).

### Example 65

### 4-(6-phenylpyrazin-2-yl)-N-pyridin-3-ylpiperazine-1-carboxamide

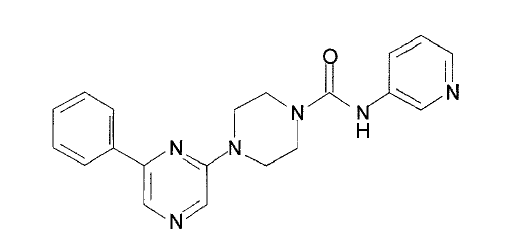

### (1) Tert-butyl 4-(6-chloropyrazin-2-yl)piperazine-1-carboxylate

To a solution of 2,6-dichloropyrazine (15.0 g, 0.100 mol) in acetonitrile (300 ml) was added tert-butyl piperazine-1-carboxylate (38.0 g, 0.22 mol) and heated under reflux for 14 hours. The reaction was distilled off under reduced pressure and to the residue was added methylene chloride and water followed by extracted. The extract was washed with saturated brine, dried over anhydrous sodium sulfate, and the solvent was distilled off under reduced pressure. The residue was crystallized from diethylether to obtain the title compound (20.0 g, 67%) as a solid.
¹H NMR (CDCl₃) δ: 1.44 (9H, s), 3.50 - 3.59 (8H, m), 7.81 (1H, s), 7.95 (1H, s).

### (2) 2-phenyl-6-piperazin-1-ylpyrazine dihydrochloride

To a solution of tert-butyl 4-(6-chloropyrazin-2-yl)piperazine-1-carboxylate (13.0 g, 43.6 mmol) and phenylboronic acid (8.00 g, 65.4 mmol) in anhydrous toluene (400 ml) was added potassium phosphate (18.5 g, 87.2 mmol)), 4,5-bis(diphenylphosphino)-9,9-dimethylxanthene (1.00 g, 1.74 mmol) and trisdibenzylideneacetone dipalladium (251 mg, 0.44 mmol) under nitrogen atmosphere, and the reaction was degassed and heated under reflux for 14 hours. The reaction was distilled off under reduced pressure and to the residue was added methylene chloride and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain tert-butyl 4-(6-phenylpyrazin-2-yl)piperazine-1-carboxylate (6.23 g, 42%).

A solution of tert-butyl 4-(6-phenylpyrazin-2-yl)piperazine-1-carboxylate (6.23 g) in 4N hydrogen chloride-ethyl acetate (100 ml) was stirred at room temperature for 7 hours and a solid was separated by filtration to obtain the title compound (5.30 g, 93%).
¹H NMR (DMSO-d₆) δ: 3.10 - 3.29 (4H, m), 3.93 (4H, t, J = 4.2 Hz), 7.41 - 7.56 (3H, m), 8.09 (2H, dd, J = 7.8, 1.0 Hz), 8.38 (1H, s), 8.55 (1H, s), 9.52 (2H, br s).

### (3) 4-(6-phenylpyrazin-2-yl)-N-pyridin-3-ylpiperazine-1-carboxamide

The title compound (330 mg, 85%) as a solid was prepared from 2-phenyl-6-piperazin-1-ylpyrazine dihydrochloride and 3-pyridine isocyanate in a manner similar to that of Example 31.
¹H NMR (DMSO-d₆) δ: 3.58 - 3.66 (4H, m), 3.66 - 3.75 (4H, m), 7.24 (1H, dd, J = 8.4, 4.4 Hz), 7.38 - 7.52 (3H, m), 7.86 (1H, dd, J = ), 8.05 (2H, dd, J = 8.2, 1.4 Hz), 8.13 (1H, dd, J = 4.8, 1.2 Hz), 8.31 (1H, s), 8.45 (1H, s), 8.63 (1H, d, J = 2.8 Hz), 8.80 (1H, s).

### Example 66

### N-(3,4-dimethylisoxazol-5-yl)-4-(6-phenylpyrazin-2-yl)piperazine-1-carboxamide

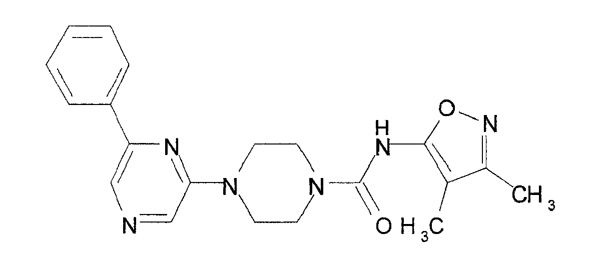

The title compound (300 mg, 64%) as a solid was prepared from 2,2,2-trichloroethyl (3,4-dimethylisoxazol-5-yl)carbamate and 2-phenyl-6-piperazin-1-ylpyrazine dihydrochloride in a manner similar to that of Example 3.
¹H NMR (DMSO-d₆) δ: 1.73 (3H, s), 2.10 (3H, s), 3.51 - 3.61 (4H, m), 3.66 - 3.75 (4H, m), 7.41 - 7.49 (3H, m), 8.04 - 8.07 (2H, m), 8.30 (1H, s), 8.45 (1H, s), 9.23 (1H, br s).

### Example 67

### 4-(6-phenylpyrazin-2-yl)-N-pyridazin-3-ylpiperazine-1-carboxamide

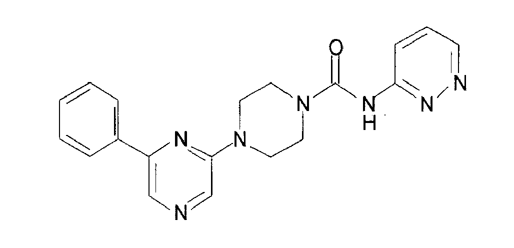

The title compound (340 mg, 57%) as a solid was prepared from 2,2,2-trichloroethyl pyridazin-3-ylcarbamate and 2-phenyl-6-piperazin-1-ylpyrazine dihydrochloride in a manner similar to that of Example 3.
¹H NMR (DMSO-d₆) δ: 3.63 - 3.75 (8H, m), 7.43 - 7.49 (3H, m), 7.55 (1H, dd, J = 8.8, 4.4 Hz), 7.98 (1H, dd, J = 9.0, 1.4 Hz), 8.05 (2H, dd, J = 8.0, 1.6 Hz), 8.30 (1H, s), 8.45 (1H, s), 8.82 (1H, dd, J = 4.4, 1.2 Hz), 9.96 (1H, s).

### Example 68

### 4-(6-phenylpyrimidin-4-yl)-N-pyridin-3-ylpiperazine-1-carboxamide

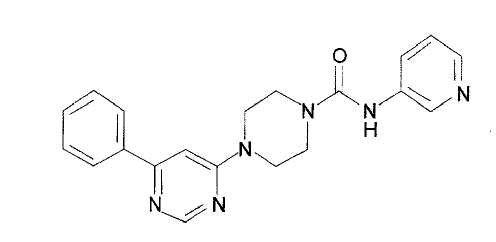

### (1) Tert-butyl 4-(6-chloropyrimidin-4-yl)piperazine-1-carboxylate

To a solution of 4,6-dichloropyrimidine (15.0 g, 0.100 mol) in N,N-dimethylformamide (150 ml) was added tert-butyl piperazine-1-carboxylate (22.0 g, 0.12 mol) and triethylamine (12.0 g, 0.12 mol), and the mixture was stirred at room temperature for 14 hours. The reaction was poured into water and extracted with methylene chloride. The extract was washed with saturated brine, dried over anhydrous sodium sulfate, and the solvent was distilled off under reduced pressure. The residue was crystallized from diethylether to obtain the title compound (15.0 g, 50%) as a solid.
¹H NMR (CDCl₃) δ: 1.45 (9H, s), 3.41 - 3.53 (4H, m), 3.55 - 3.65 (4H, m), 6.47 (1H, s), 8.35 (1H, s).

### (2) 4-phenyl-6-piperazin-1-ylpyrimidine dihydrochloride

To a solution of tert-butyl 4-(6-chloropyrimidin-4-yl)piperazine-1-carboxylate (12.0 g, 40.3 mmol) and phenylboronic acid (7.37 g, 60.4 mmol) in anhydrous toluene (400 ml) was added potassium phosphate (17.0 g, 80.5 mmol), 4,5-bis(diphenylphosphino)-9,9-dimethylxanthene (931 mg, 1.61 mmol) and trisdibenzylideneacetone dipalladium (232 mg, 0.40 mmol) under nitrogen atmosphere and the reaction was deaerated and heated under reflux for 14 hours. The reaction was distilled off under reduced pressure and to the residue was added methylene chloride and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain tert-butyl 4-(6-phenylpyrimidin-4-yl)piperazine-1-carboxylate (5.50 g, 40%).

A solution of tert-butyl 4-(6-phenylpyrimidin-4-yl)piperazine-1-carboxylate (5.50 g) in 4N hydrogen chloride-ethyl acetate (100 ml) was stirred at room temperature for 6 hours and a solid was separated by filtration to obtain the title compound (5.00 g, 99%).
¹H NMR (DMSO-d₆) δ: 3.20 - 3.30 (4H, m), 4.15 - 4.22 (4H, m), 7.57 - 7.67 (4H, m), 8.12 (2H, d, J = 6.8 Hz), 8.84 (1H, s), 9.84 (2H, br s).

### (3) 4-(6-phenylpyrimidin-4-yl)-N-pyridin-3-ylpiperazine-1-carboxamide

The title compound (410 mg, 91%) as a solid was prepared from 4-phenyl-6-piperazin-1-ylpyrimidine dihydrochloride and 3-pyridine isocyanate in a manner similar to that of Example 31.
¹H NMR (DMSO-d₆) δ: 3.56 - 3.59 (4H, m), 3.76 - 3.79 (4H, m), 7.25 (1H, dd, J = 8.4, 4.4 Hz), 7.33 (1H, d, J = 1.2 Hz), 7.46 - 7.49 (3H, m), 7.85 - 7.88 (1H, m), 8.12 - 8.15 (3H, m), 8.58 (1H, d, J = 0.8 Hz), 8.63 (1H, d, J = 2.4 Hz), 8.81 (1H, s).

### Example 69

### N-(3,4-dimethylisoxazol-5-yl)-4-(6-phenylpyrimidin-4-yl)piperazine-1-carboxamide

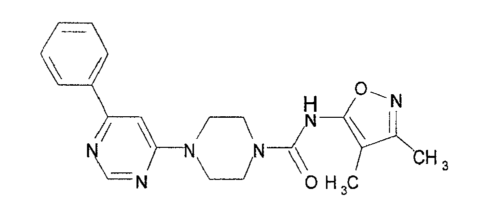

The title compound (380 mg, 81%) as a solid was prepared from 2,2,2-trichloroethyl (3,4-dimethylisoxazol-5-yl)carbamate and 4-phenyl-6-piperazin-1-ylpyrimidine dihydrochloride in a manner similar to that of Example 3.
¹H NMR (DMSO-d₆) δ: 1.73 (3H, s), 2.10 (3H, s), 3.50 - 3.58 (4H, m), 3.75 - 3.80 (4H, m), 7.33 (1H, s), 7.46 - 7.48 (3H, m), 8.12 - 8.15 (2H, m), 8.57 (1H, d, J = 0.8 Hz), 9.23 (1H, br s).

### Example 70

### 4-(6-phenylpyrimidin-4-yl)-N-pyridazin-3-ylpiperazine-1-carboxamide

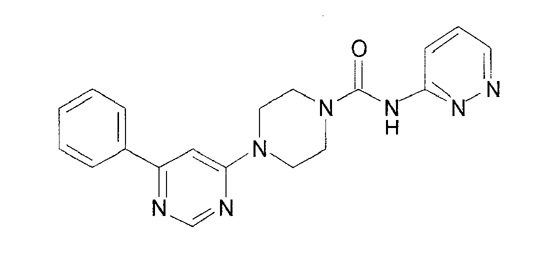

The title compound (220 mg, 49%) as a solid was prepared from 2,2,2-trichloroethyl pyridazin-3-ylcarbamate and 4-phenyl-6-piperazin-1-ylpyrimidine dihydrochloride in a manner similar to that of Example 3.
¹H NMR (DMSO-d₆) δ: 3.55 - 3.62 (4H, m), 3.72 - 3.80 (4H, m), 7.34 (1H, d, J = 0.8 Hz), 7.45 - 7.47 (3H, m), 7.55 (1H, dd, J = 9.0, 4.6 Hz), 7.98 (1H, dd, J = 9.2, 1.2 Hz), 8.13 - 8.15 (2H, m), 8.57 (1H, d, J = 1.2 Hz), 8.81 - 8.82 (1H, m), 9.95 (1H, br s).

### Example 71

### 4-(2-phenylpyridin-4-yl)-N-pyridin-3-ylpiperazine-1-carboxamide

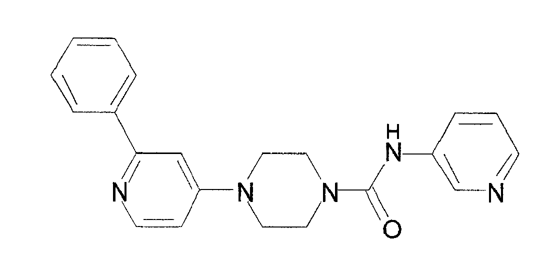

### (1) Tert-butyl 4-(2-chloropyridin-4-yl)piperazine-1-carboxylate

To a solution of 4-bromo-2-chloropyridine (15.0 g, 77.3 mmol) and tert-butyl piperazine-1-carboxylate (18.0 g, 77.3 mmol) in anhydrous toluene (400 ml) was added sodium tert-butoxide (11.0 g, 116 mmol), 4,5-bis(diphenylphosphino)-9,9-dimethylxanthene (1.34 g, 2.32 mmol) and trisdibenzylideneacetone dipalladium (445 mg, 0.77 mmol) under nitrogen atmosphere, and the reaction was degassed and heated under reflux for 14 hours. The reaction was distilled off under reduced pressure and to the residue was added ethyl acetate and water followed by extracted. The extract was washed with saturated brine and dried over anhydrous sodium sulfate, and the solvent was distilled off under reduced pressure. The residue was purified by silica gel column chromatography (petroleum ether:ethyl acetate = 5:1) to obtain the title compound (16.0 g, 70%) as a solid.
¹H NMR (CDCl₃) δ: 1.45 (9H, s), 3.30 - 3.32 (4H, m), 3.52 - 3.54 (4H, m), 6.52 - 6.55 (1H, m), 6.01 (1H, s), 7.97 - 8.04 (1H, m).

### (2) 1-(2-phenylpyridin-4-yl)piperazine dihydrochloride

To a solution of tert-butyl 4-(2-chloropyridin-4-yl)piperazine-1-carboxylate (16.0 g, 53.7 mmol) and phenylboronic acid (9.80 g, 80.5 mmol) in anhydrous toluene (500 ml) was added potassium phosphate (22.8 g, 107 mmol), 4,5-bis(diphenylphosphino)-9,9-dimethylxanthene (1.24 g, 2.15 mmol) and trisdibenzylideneacetone dipalladium (309 mg, 0.54 mmol) under nitrogen atmosphere, and the reaction was degassed and heated under reflux for 20 hours. The reaction was distilled off under reduced pressure and to the residue was added ethyl acetate and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain tert-butyl 4-(2-phenylpyridin-4-yl)piperazine-1-carboxylate (8.00 g, 44%).

A solution of tert-butyl 4-(2-phenylpyridin-4-yl)piperazine-1-carboxylate (8.00 g) in 4N hydrogen chloride-ethyl acetate (120 ml) was stirred at room temperature for 6 hours and a solid was separated by filtration to obtain the title compound (7.20 g, 98%).
¹H NMR (DMSO-d₆) δ: 3.73 - 3.93 (4H, m), 4.00 - 4.15 (4H, m), 7.26 (1H, dd, J = 7.2, 2.0 Hz), 7.50 (1H, d, J = 2.0 Hz), 7.54 - 7.64 (3H, m), 8.02 (2H, dd, J = 8.0, 1.6 Hz), 8.29 (1H, d, J = 7.2 Hz), 10.01 (2H, br s).

### (3) 4-(2-phenylpyridin-4-yl)-N-pyridin-3-ylpiperazine-1-carboxamide

The title compound (200 mg, 44%) as a solid was prepared from 1-(2-phenylpyridin-4-yl)piperazine dihydrochloride and 3-pyridine isocyanate in a manner similar to that of Example 31.
¹H NMR (DMSO-d₆) δ: 3.38 - 3.51 (4H, m), 3.55 - 3.67 (4H, m), 6.82 (1H, dd, J = 6.0, 2.4 Hz), 7.24 (1H, J = 8.4, 4.4 Hz), 7.30 - 7.47 (4H, m), 7.86 (1H, dd, J = 5.6, 1.2 Hz), 8.04 (2H, dd, J = 8.4, 1.2 Hz), 8.13 (1H, dd, J = 8.4, 1.6 Hz), 8.25 (1H, d, J = 5.6 Hz), 8.63 (1H, d, J = 2.4 Hz), 8.80 (1H, s).

### Example 72

### N-(3,4-dimethylisoxazol-5-yl)-4-(2-phenylpyridin-4-yl)piperazine-1-carboxamide

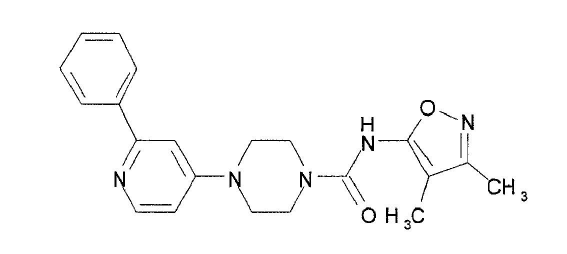

The title compound (350 mg, 74%) as a solid was prepared from 2,2,2-trichloroethyl (3,4-dimethylisoxazol-5-yl)carbamate and 1-(2-phenylpyridin-4-yl)piperazine dihydrochloride in a manner similar to that of Example 3.
¹H NMR (DMSO-d₆) 5: 1.73 (3H, s), 2.10 (3H, s), 3.40 - 3.50 (4H, m), 3.52 - 3.61 (4H, m), 6.80 (1H, dd, J = 6.0, 2.4 Hz), 7.30 - 7.45 (4H, m), 8.03 (2H, dd, J = 6. 6, 1.4 Hz), 8.24 (1H, d, J = 6.0 Hz), 9.27 (1H, br s).

### Example 73

### 4-(2-phenylpyridin-4-yl)-N-pyridazin-3-ylpiperazine-1-carboxamide

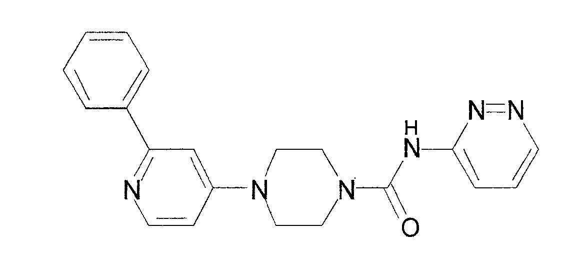

The title compound (100 mg, 33%) as a solid was prepared from 2,2,2-trichloroethyl pyridazin-3-ylcarbamate and 1-(2-phenylpyridin-4-yl)piperazine dihydrochloride in a manner similar to that of Example 3.
¹H NMR (DMSO-d₆) δ: 3.65 - 3.72 (4H, m), 3.81 - 3.92 (4H, m), 7.15 - 7.22 (1H, m), 7.43 (1H, d, J = 2.0 Hz), 7.53 - 7.67 (4H, m), 7.83 - 7.91 (2H, m), 8.02 (1H, d, J = 9.2 Hz), 8.27 (1H, d, J = 7.6 Hz), 8.85 (1H, d, J = 3.6 Hz).

### Example 74

### 4-(2-phenylpyrimidin-4-yl)-N-pyridin-3-ylpiperazine-1-carboxamide

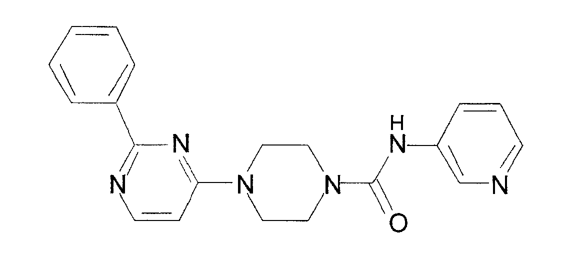

### (1) Tert-butyl 4-(2-chloropyrimidin-4-yl)piperazine-1-carboxylate

To a solution of 2,4-dichloropyrimidine (50.0 g, 0.338 mol) in ethanol (500 ml) was added tert-butyl piperazine-1-carboxylate (62.8 g, 0.338 mol) and sodium hydrogen carbonate (56.8 g, 0.676 mol) and heated under reflux for 1.5 hours. The reaction was filtered and the filtrate was concentrated. To the residue was added methylene chloride and water followed by extracted. The extract was washed with saturated brine, dried over anhydrous sodium sulfate, and the solvent was distilled off under reduced pressure. The residue was purified by silica gel column chromatography (petroleum ether:ethyl acetate = 10:1) to obtain the title compound (40.0 g, 40%).
¹H NMR (CDCl₃) δ: 1.49 (9H, s), 3.46 - 3.53 (4H, m), 3.62 - 3.69 (4H, m), 6.40 (1H, d, J = 6.4 Hz), 8.06 (1H, d, J = 6.4 Hz).

### (2) 2-phenyl-4-piperazin-1-ylpyrimidine dihydrochloride

To a solution of tert-butyl 4-(2-chloropyrimidin-4-yl)piperazine-1-carboxylate (13.0 g, 43.5 mmol) and phenylboronic acid (7.95 g, 65.2 mmol) in anhydrous toluene (500 ml) was added potassium phosphate (18.4 g, 87.0 mmol), 4,5-bis(diphenylphosphino)-9,9-dimethylxanthene (1.00 g, 1.74 mmol) and trisdibenzylideneacetone dipalladium (250 mg, 0.43 mmol) under nitrogen atmosphere, and the reaction was degassed and heated under reflux for 14 hours. The reaction was distilled off under reduced pressure and to the residue was added methylene chloride and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain tert-butyl 4-(2-phenylpyrimidin-4-yl)piperazine-1-carboxylate (9.00 g, 61%).

A solution of tert-butyl 4-(2-phenylpyrimidin-4-yl)piperazine-1-carboxylate (9.00 g) in 4N hydrogen chloride-ethyl acetate (100 ml) was stirred at room temperature fro 6 hours and a solid was separated by filtration to obtain the title compound (6.50 g, 78%).
¹H NMR (DMSO-d₆) δ: 3.19 - 3.32 (4H, m), 4.12 - 4.33 (4H, m), 7.24 (1H, d, J = 7.6 Hz), 7.61 - 7.65 (2H, m), 7.69 - 7.73 (1H, m), 8.35 - 8.45 (3H, m), 9.95 (2H, br s).

### (3) 4-(2-phenylpyrimidin-4-yl)-N-pyridin-3-ylpiperazine-1-carboxamide

The title compound (287 mg, 96%) as a solid was prepared from 2-phenyl-4-piperazin-1-ylpyrimidine dihydrochloride and 3-pyridine isocyanate in a manner similar to that of Example 31.
¹H NMR (DMSO-d₆) δ: 3.53 - 3.62 (4H, m), 3.71 - 3.83 (4H, m), 6.80 (1H, d, J = 5.6 Hz), 7.25 (1H, dd, J = 8.4, 4.8 Hz), 7.40 - 7.48 (3H,m), 7.82 - 7.88 (1H, m), 8.13 (1H, dd, J = 4.8, 1.4 Hz), 8.26 - 8.32 (3H, m), 8.63 (1H, d, J = 2.4 Hz), 8.81 (1H, s).

### Example 75

### N-(3,4-dimethylisoxazol-5-yl)-4-(2-phenylpyrimidin-4-yl)piperazine-1-carboxamide

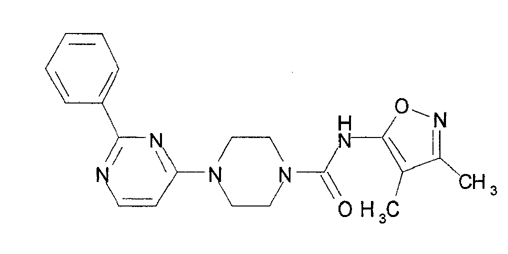

The title compound (150 mg, 48%) as a solid was prepared from 2,2,2-trichloroethyl (3,4-dimethylisoxazol-5-yl)carbamate and 2-phenyl-4-piperazin-1-ylpyrimidine dihydrochloride in a manner similar to that of Example 3.
¹H NMR (DMSO-d₆) δ: 1.74 (3H, s), 2.10 (3H, s), 3.51 - 3.59 (4H, m), 3.70 - 3.80 (4H, m), 6.79 (1H, d, J = 6.4 Hz), 7.41 - 7.48 (3H, m), 8.28 - 8.33 (3H, m), 9.24 (1H, s).

### Example 76

### 4-(2-phenylpyrimidin-4-yl)-N-pyridazin-3-ylpiperazine-1-carboxamide

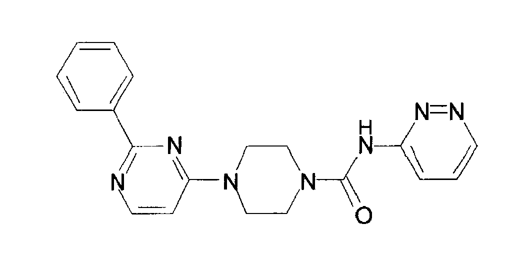

The title compound (95 mg, 32%) as a solid was prepared from 2,2,2-trichloroethyl pyridazin-3-ylcarbamate and 2-phenyl-4-piperazin-1-ylpyrimidine dihydrochloride in a manner similar to that of Example 3.
¹H NMR (DMSO-d₆) δ: 3.50 - 3.60 (4H, m), 3.72 - 3.86 (4H, m), 6.79 (1H, d, J = 6.4 Hz), 7.40 - 7.50 (3H,m), 7.56 (1H, dd, J = 8.4, 4.4 Hz), 7.98 (1H, d, J = 8.8 Hz), 8.25 - 8.40 (3H, m), 8.82 (1H, d, J = 4.0 Hz), 9.56 (1H, s).

### Example 77

### 4-(4-phenylpyrimidin-2-yl)-N-pyridin-3-ylpiperazine-1-carboxamide

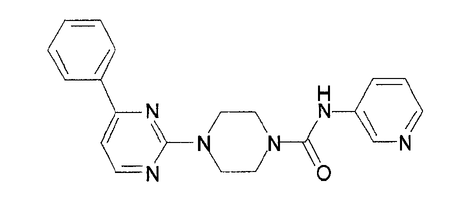

### (1) Tert-butyl 4-(4-chloropyrimidin-2-yl)piperazine-1-carboxylate

To a solution of 2,4-dichloropyrimidine (50.0 g, 0.338 mol) in ethanol (500 ml) was added tert-butyl piperazine-1-carboxylate (62.8 g, 0.338 mol) and sodium hydrogen carbonate (56.8 g, 0.676 mol) and heated under reflux for 1.5 hours. The reaction was filtered and the filtrate was concentrated. To the residue was added methylene chloride and water followed by extracted. The extract was washed with saturated brine, dried over anhydrous sodium sulfate, and the solvent was distilled off under reduced pressure. The residue was purified by silica gel column chromatography (petroleum ether:ethyl acetate = 10:1) to obtain the title compound (6.30 g, 6%).
¹H NMR (CDCl₃) δ: 1.49 (9H, s), 3.45 - 3.52 (4H, m), 3.75 - 3.83 (4H, m), 6.53 (1H, d, J = 4.8 Hz), 8.16 (1H, d, J = 4.8 Hz).

### (2) 4-phenyl-2-piperazin-1-ylpyrimidine dihydrochloride

To a solution of tert-butyl 4-(4-chloropyrimidin-2-yl)piperazine-1-carboxylate (6.30 g, 20.7 mmol) and phenylboronic acid (3.75 g, 31.1 mmol) in anhydrous toluene (250 ml) was added potassium phosphate (9.20 g, 41.5 mmol), 4,5-bis(diphenylphosphino)-9,9-dimethylxanthene (480 mg, 0.83 mmol) and trisdibenzylideneacetone dipalladium (119 mg, 0.21 mmol) under nitrogen atmosphere, and the reaction was degassed and heated under reflux for 14 hours. The reaction was distilled off under reduced pressure and to the residue was added methylene chloride and water followed by extracted. The extract was washed with saturated brine, dried over anhydrous sodium sulfate, and the solvent was distilled off under reduced pressure. The residue was purified by silica gel column chromatography (petroleum ether:ethyl acetate = 5:1) to obtain tert-butyl 4-(4-phenylpyrimidin-2-yl)piperazine-1-carboxylate (5.00 g, 70%).

A solution of tert-butyl 4-(4-phenylpyrimidin-2-yl)piperazine-1-carboxylate (5.00 g) in 4N hydrogen chloride-ethyl acetate (100 ml) was stirred at room temperature for 6 hours and a solid was separated by filtration to obtain the title compound (4.50 g, 98%).
¹H NMR (DMSO-d₆) 5: 3.11 - 3.20 (4H, m), 4.05 - 4.13 (4H, m), 7.35 (1H, d, J = 5.6 Hz), 7.47 - 7.55 (3H, m), 8.13 - 8.15 (2H, m), 8.47 (1H, d, J = 5.6 Hz), 9.57 (2H, br s).

### (3) 4-(4-phenylpyrimidin-2-yl)-N-pyridin-3-ylpiperazine-1-carboxamide

The title compound (273 mg, 91%) as a solid was prepared from 4-phenyl-2-piperazin-1-ylpyrimidine dihydrochloride and 3-pyridine isocyanate in a manner similar to that of Example 31.
¹H NMR (DMSO-d₆) δ: 3.58 - 3.62 (4H, m), 3.85 - 3.93 (4H, m), 7.21 - 7.30 (2H, m), 7.50 - 7.55 (3H, m), 7.82 - 7.90 (1H, m), 8.12 - 8.20 (2H, m), 8.45 (1H, d, J = 5.2 Hz), 8.64 (1H, d, J = 2.4 Hz), 8.83 (1H, s).

### Example 78

### N-(3,4-dimethylisoxazol-5-yl)-4-(4-phenylpyrimidin-2-yl)piperazine-1-carboxamide

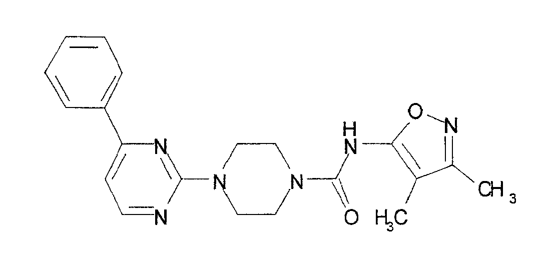

The title compound (250 mg, 80%) as a solid was prepared from 2,2,2-trichloroethyl (3,4-dimethylisoxazol-5-yl)carbamate and 4-phenyl-2-piperazin-1-ylpyrimidine dihydrochloride in a manner similar to that of Example 3.
¹H NMR (DMSO-d₆) δ: 1.73 (3H, s), 2.10 (3H, s), 3.51 - 3.57 (4H, m), 3.80 - 3.88 (4H, m), 7.22 (1H, d, J = 5.2 Hz), 7.46 - 7.50 (3H, m), 8.06 - 8.13 (2H, m), 8.44 (1H, d, J = 5.2 Hz), 9.21 (1H, br s).

### Example 79

### 4-(4-phenylpyrimidin-2-yl)-N-pyridazin-3-ylpiperazine-1-carboxamide

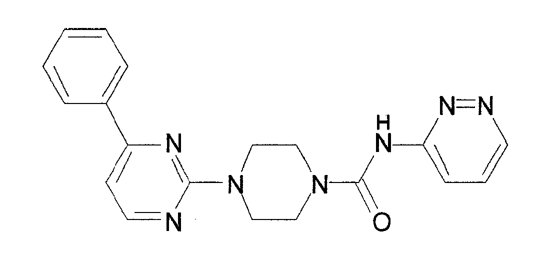

The title compound (140 mg, 37%) as a solid was prepared from 2,2,2-trichloroethyl pyridazin-3-ylcarbamate and 4-phenyl-2-piperazin-1-ylpyrimidine dihydrochloride in a manner similar to that of Example 3.
¹H NMR (DMSO-d₆) δ: 3.55 - 3.65 (4H, m), 3.81 - 3.89 (4H, m), 7.21 (1H, d, J = 5.2 Hz), 7.45 - 7.50 (3H, m), 7.55 (1H, dd, J = 8.8, 4.4 Hz), 7.98 (1H, dd, J = 9.2, 1.2 Hz), 8.09 - 8.13 (2H, m), 8.4 (1H, d, J = 5.2 Hz), 8.81 (1H, d, J = 4.4 Hz), 9.92 (1H, br s).

### Example 80

### 4-[4-(2,4-difluorophenyl)pyridin-2-yl]-N-pyridin-3-ylpiperazine-1-carboxamide

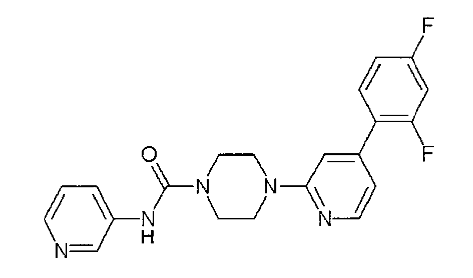

### (1) 2-chloro-4-(2,4-difluorophenyl)pyridine

To a solution of 4-bromo-2-choloropyridine (30 g, 156 mmol), 2,4-difluorophenylboronic acid (24.6 g, 156 mmol), and sodium carbonate (43.1 g, 312 mmol) in methanol (195 ml) was added tetrakisphenylphosphine palladium (9.01 g, 7.79 mmol) at room temperature under nitrogen atmosphere, and the mixture was stirred at 50°C for 17 hours. The reaction was cooled to room temperature and the resulting solid was filtered. The filtrate was dried over anhydrous magnesium sulfate and the solvent was distilled off under reduced pressure. The residue was purified by silica gel column chromatography (ethyl acetate:hexane = 1:8) to obtain the title compound (31.9 g, 91%) as a solid.
¹H-NMR (CDCl₃) δ: 6. 95 - 7.05 (2H, s), 7.37 - 7.39 (1H, m), 7.43 - 7.47 (1H, m), 7.49 (1H, br s), 8.47 (1H, d, J = 13.2 Hz).

### (2) Tert-butyl 4-[4-(2,4-difluorophenyl)pyridin-2-yl]piperazine-1-carboxylate

A mixture of 2-chloro-4-(2,4-difluorophenyl)pyridine (15.0 g, 66.5 mmol), tert-butyl piperazine-1-carboxylate (12.4 g, 66.6 mmol), palladium acetate (746 mg, 3.32 mmol), 2,2-bis(diphenylphosphino)-1,1-binaphthyl (3.31 g, 5.32 mmol), sodium tert-butoxide (13.0 g, 133 mmol), and 1,4-dioxane (133 ml) was stirred at 85°C for 18 hours. The reaction was cooled to room temperature and the solvent was distilled off under reduced pressure. To the residue was added ethyl acetate, washed with distilled water, dried over anhydrous magnesium sulfate, and the solvent was distilled off under reduced pressure. The residue was purified by silica gel column chromatography (dichloromethane:hexane = 1:1) to obtain the title compound (19.0 g, 76%) as a solid.
¹H-NMR (CDCl₃) δ: 1.49 (9H, s), 3.57 (8H, s), 6.75 - 6.78 (2H, m), 6.90 - 6.98 (2H, m), 7.39 - 7.45 (1H, m), 8.24 (1H, d, J = 4.8 Hz).

### (3) 1-[4-(2,4-difluorophenyl)pyridin-2-yl]piperazine dihydrochloride

To a solution of tert-butyl 4-[4-(2,4-difluorophenyl)pyridin-2-yl]piperazine-1-carboxylate (19.0 g, 50.6 mmol) in methanol (63 ml) was added 4N hydrogen chloride-methanol solution (50.6 ml), and the mixture was stirred at room temperature for 16 hours. The resulting solid was filtered, washed with methanol, and dried under reduced pressure to obtain the title compound (15.5 g, 98%) as a solid.
¹H-NMR (CDCl₃) δ: 3.22 (4H, s), 3.91 (4H, s), 7.01 - 7.02 (1H, m), 7.23 (1H, br s), 7.26 - 7.31 (1H, m), 7.44 - 7.50 (1H, m), 7.72 - 7.78 (1H, m), 8.20 (1H, d, J = 5.6 Hz), 9.40 (2H, br s).

### (4) 4-[4-(2,4-difluorophenyl)pyridin-2-yl]-N-pyridin-3-ylpiperazine-1-carboxamide

To a solution of 1-[4-(2,4-difluorophenyl)pyridin-2-yl]piperazine dihydrochloride (500 mg, 1.60 mmol) in methylene chloride (2.2 ml) was added triethylamine (0.564 ml, 4.01 mmol) and 3-pyridine isocyanate (231 mg, 1.93 mmol) at room temperature and the mixture was stirred at room temperature for 12 hours, and the solvent was distilled off under reduced pressure. The residue was purified by silica gel column chromatography (methanol:ethyl acetate = 1:6) to obtain the title compound (453 mg, 71%) as a solid.
¹H-NMR (DMSO-d₆) δ: 3.63 (8H, s), 6.82 - 6.84 (1H, m), 6.98 (1H, s), 7.22 - 7.29 (2H, m), 7.39 - 7.44 (1H, m), 7.66 - 7.72 (1H, m), 7.88 - 7.92 (1H, m), 8.15 - 8.17 (1H, m), 8.21 (1H, d, J = 5.6 Hz), 8.66 (1H, d, J = 2.4 Hz), 8.81 (1H, s).

### Example 81

### 4-[4-(2,4-difluorophenyl)pyridin-2-yl]-N-(3,4-dimethylisoxazol-5-yl)piperazine-1-carboxamide

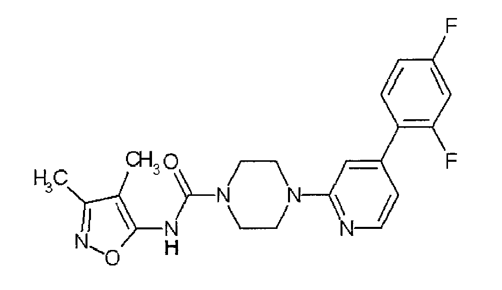

To a solution of 1-[4-(2,4-difluorophenyl)pyridin-2-yl]piperazine dihydrochloride (500 mg, 1.60 mmol) in dimethylsulfoxide (2.2 ml) was added N,N-diisopropylethylamine (0.698 ml, 4.01 mmol) at room temperature, and the mixture was stirred at room temperature for 30 minutes. 2,2,2-trichloroethyl (3,4-dimethylisoxazol-5-yl)carbamate (507 mg, 1.76 mmol) was added and the mixture was stirred at 45°C for 12 hours, cooled to room temperature, and the reaction was poured into water, followed by the mixture was stirred for further 1 hour. The resulting solid was filtered, washed with water and ethyl acetate, and dried under reduced pressure to obtain the title compound (155 mg, 23%) as a solid.
¹H-NMR (DMSO-d₆) δ: 1.76 (3H, s), 2.13 (3H, s), 3.55 - 3.61 (8H, m), 6.82 - 6.84 (1H, m), 6.97 (1H, s), 7.21 - 7.26 (1H, m), 7.38 - 7.44 (1H, m), 7.66 - 7.72 (1H, m), 8.20 (1H, d, J = 5.2 Hz), 9.24 (1H, br s).

### Example 82

### 4-[4-(2,4-difluorophenyl)pyridin-2-yl]-N-pyridazine-3-ylpiperazine-1-carboxamide

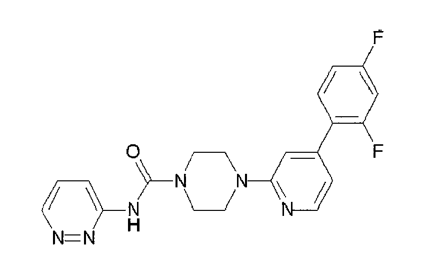

To a solution of 1-[4-(2,4-difluorophenyl)pyridin-2-yl]piperazine dihydrochloride (500 mg, 1.60 mmol) in dimethylsulfoxide (2.2 ml) was added N,N-diisopropylethylamine (0.698 ml, 4.01 mmol) at room temperature, and the mixture was stirred at room temperature for 30 minutes. 2,2,2-trichloroethyl pyridazin-3-ylcarbamate (477 mg, 1.76 mmol) was added, and the mixture was stirred at 45°C for 12 hours. The reaction was poured into water and extracted with ethyl acetate, followed by the extract was washed with water, dried over anhydrous magnesium sulfate, and the solvent was distilled off under reduced pressure. The residue was purified by silica gel column chromatography (ethyl acetate:dichloromethane:methanol = 3:1:0.5) to obtain the title compound (199 mg, 31%) as a solid.
¹HNMR (DMSO-d₆) δ: 3.63 (8H, s), 6.82 - 6.84 (1H, m), 6.98 (1H, s), 7.21 - 7.26 (1H, m), 7.38 - 7.44 (1H, m), 7.56 - 7.60 (1H, m), 7.66 - 7.72 (1H, m), 8.00 - 8.03 (1H, m), 8.21 (1H, d, J = 5.2 Hz), 8.85 (1H, d, J = 3.6 Hz ), 9.96 (1H, s).

### Example 83

### N-1,2-benzoisoxazol-3-yl-4-[4-(2,4-difluorophenyl)pyridin-2-yl]piperadine-1-carboxamide

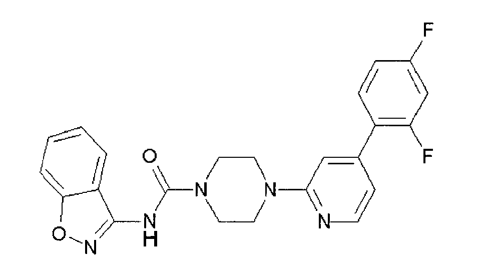

The title compound (176 mg, 42%) as a solid was prepared from 2,2,2-trichloroethyl 1,2-bnzoisoxazol-3-ylcarbamate and 1-[4-(2,4-difluorophenyl)pyridin-2-yl]piperazine in a manner similar to that of Example 81.
¹HNMR (DMSO-d₆) 5: 3.66 (8H, s), 6.83 - 6.85 (1H, m), 6.70 (1H, s), 7.22 - 7.27 (1H, m), 7.29 - 7.33 (1H, m), 7.39 - 7.44 (1H, m), 7.59 - 7.73 (3H, m), 7.85 (1H, d, J = 8.0 Hz), 8.22 (1H, d, J = 5.6 Hz), 9.99 (1H, s).

### Example 84

### 4-[4-(2,3-difluorophenyl)pyridin-2-yl]-N-pyridin-3-ylpiperazine-1-carboxamide

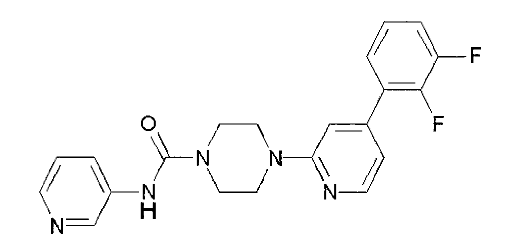

### (1) 2-chloro-4-(2,3-difluorophenyl)pyridine

To a solution of 4-bromo-2-chloropyridine (10.0 g, 52.0 mmol), 2,3-difluorophenylboronic acid (8.21 g, 52.0 mmol), and sodium carbonate (14.4 g, 104 mmol) in methanol (104 ml) was added tetrakisphenylphosphine palladium (3.00 g, 2.60 mmol) at room temperature under nitrogen atmosphere, and the mixture was stirred at 50°C for 17 hours. The reaction was cooled to room temperature and the resulting solid was filtered. The filtrate was dried over anhydrous magnesium sulfate and the solvent was distilled off under reduced pressure. The residue was purified by silica gel column chromatography (ethyl acetate:hexane = 1:8) to obtain the title compound (11.2 g, 96%) as a solid.
¹H-NMR (CDCl₃) δ: 7.21 - 7.24 (2H, m), 7.26 - 7.31 (1H, m), 7.41 - 7.43 (1H, m), 7.52 (1H, s), 8.48 (1H, d, J = 5.2 Hz).

### (2) Tert-butyl 4-[4-(2,3-difluorophenyl)pyridin-2-yl]piperazine-1-carboxylate

A mixture of 2-chloro-4-(2,3-difluorophenyl)pyridine (10.0 g, 44.3 mmol), tert-butyl piperazine-1-carboxylate (9.91 g, 53.2 mmol), palladium acetate (498 mg, 2.22 mmol), 2,2-bis(diphenylphosphino)-1,1-binaphtyl (2.21 g, 3.55 mmol), sodium tert-butoxide (8.69 g, 89.0 mmol), and 1,4-dioxane (89 ml) was stirred at 85°C for 18 hours. The reaction was cooled to room temperature and the solvent was distilled off under reduced pressure. To the residue was added ethyl acetate, wished with distilled water, dried over anhydrous magnesium sulfate, and the solvent was distilled off under reduced pressure. The residue was purified by silica gel column chromatography (ethyl acetate:hexane = 1:3) to obtain the title compound (13.4 g,
81%) as a solid.
¹H-NMR (CDCl₃) δ: 1.49 (9H, s), 3.58 (8H, s), 6.78 - 6.81 (2H, m), 7.15 - 7.22 (3H, m), 8.27 (1H, d, J = 4.8 Hz).

### (3) 1-[4-(2,3-difluorophenyl)pyridin-2-yl]piperazine

To a solution of tert-butyl 4-[4-(2,3-difluorophenyl)pyridin-2-yl]piperazine-1-carboxylate (13.4 g, 35.8 mmol) in methanol (36 ml) was added 4N hydrogen chloride-methanol solution (44.8 ml) and the mixture was stirred at room temperature for 16 hours, and the resulting solid was filtered. The solid was dissolved in water, neutralized by adding 1N aqueous sodium hydroxide solution, and extracted with ethyl acetate. The extract was wished with water, dried over anhydrous magnesium sulfate and the solvent was distilled off under reduced pressure to obtain the title compound (6.50 g, 66%) as a solid.
¹H-NMR (CDCl₃) 5: 2.78 (4H, t, J = 4.8 Hz), 3.45 (4H, t, J = 4.8 Hz), 6.79 (1H, d, J = 4.8 Hz), 6.90 (1H, s), 7.29 - 7.35 (1H, m), 7.39 - 7.43 (1H, m), 7.47 - 7.54 (1H, m), 8.19 (1H, d, J = 5.2 Hz).

### (4) 4-[4-(2,3-difluorophenyl)pyridin-2-yl]-N-pyridin-3-ylpiperazine-1-carboxamide

To a solution of 1-[4-(2,3-difluorophenyl)pyridin-2-yl]piperazine (300 mg, 1.09 mmol) in methylene chloride (1.2 ml) was added triethylamine (0.184 ml, 1.31 mmol) and 3-pyridine isocyanate (157 mg, 1.31 mmol) at room temperature, and the mixture was stirred at room temperature for 12 hours and the solvent was distilled off under reduced pressure. The residue was purified by silica gel column chromatography (methanol:ethyl acetate = 1:3) to obtain the title compound (285 mg, 66%) as a solid.
¹H-NMR (DMSO-d₆) δ: 3.62 - 3.63 (8H, m), 6.87 (1H, d, J = 4.8 Hz), 7.03 (1H, s), 7.28 - 7.30 (1H, m), 7.32 - 7.37 (1H, m), 7.42 - 7.46 (1H, m), 7.50 - 7.56 (1H, m), 7.89 - 7.97 (1H, m), 8.16 - 8.17 (1H, m), 8.25 (1H, d, J = 5.2 Hz), 8.67 (1H, d, J = 2.4 Hz), 8.82 (1H, s).

### Example 85

### 4-[4-(2,3-difluorophenyl)pyridin-2-yl]-N-(3,4-dimethylisoxazol-5-yl)piperazine-1-carboxamide

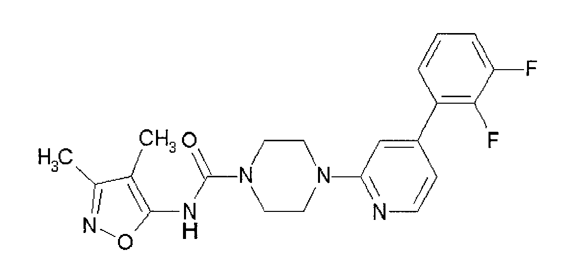

To a solution of 1-[4-(2,3-difluorophenyl)pyridin-2-yl]piperazine (300 mg, 1.09 mmol) in dimethylsulfoxide was added N,N-diisopropylethylamine (0.200 ml, 1.20 mmol) at room temperature, and the mixture was stirred at room temperature for 30 minutes. 2,2,2-trichloroethyl (3,4-dimethylisoxazol-5-yl)carbamate (345 mg, 1.20 mmol) was added and the mixture was stirred 45°C for 12 hours, cooled to room temperature, and the reaction was poured into water, followed by the mixture was stirred for further 1 hour. The resulting solid was filtered, washed with water and ethyl acetate, and dried under reduced pressure to obtain the title compound (310 mg, 69%) as a solid.
¹H-NMR (DMSO-d₆) δ: 1.76 (3H, s), 2.13 (3H, s), 3.56 - 3.62 (8H, m), 6.86 (1H, d, J = 4.8 Hz), 7.02 (1H, s), 7.31 - 7.36 (1H, m), 7.42 - 7.45 (1H, m), 7.49 - 7.55 (1H, m), 8.23 (1H, d, J = 5.2 Hz), 9.24 (1H, br s).

### Example 86

### 4-[4-(2,3-difluorophenyl)pyridin-2-yl]-N-pyridazin-3-ylpiperazine-1-carboxamide

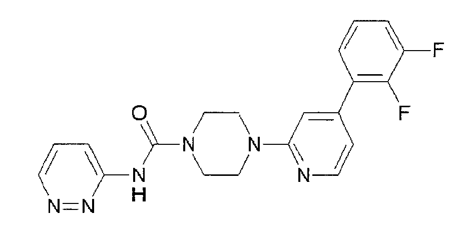

The title compound (315 mg, 73%) as a solid was prepared from 2,2,2-trichloroethyl pyridazin-3-ylcarbamate and 1-[4-(2,3-difluorophenyl)pyridin-2-yl]piperazine in a manner similar to that of Example 85.
¹HNMR (DMSO-d₆) δ: 3.64 (8H, s), 6.85 - 6.87 (1H, m), 7.02 (1H, s), 7.31 - 7.37 (1H, m), 7.42 - 7.46 (1H, m), 7.49 - 7.60 (2H, m), 8.00 - 8.03 (1H, m), 8.23 (1H, d, J = 5.2 Hz), 8.84 - 8.85 (1H, m), 9.97 (1H, br s).

### Example 87

### N-1,2-benzoisoxazol-3-yl-4-[4-(2,3-difluorophenyl)pyridin-2-yl]piperazine-1-carboxamide

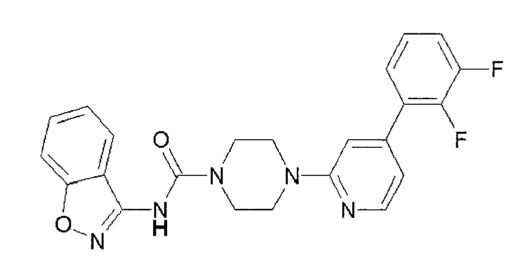

The title compound (235 mg, 50%) as a solid was prepared from 2,2,2-trichloroethyl 1,2-benzoisoxazol-3-ylcarbamate and 1-[4-(2,3-difluorophenyl)pyridin-2-yl]piperazine in a manner similar to that of Example 85.
¹HNMR (DMSO-d₆) δ: 3.67 (8H, s), 6.86 - 6.89 (1H, m), 7.04 (1H, s), 7.29 - 7.37 (2H, m), 7.42 - 7.47 (1H, m), 7.49 - 7.56 (1H, m), 7.58 - 7.66 (2H, m), 7.85 (1H, d, J = 8.4 Hz), 8.25 (1H, d, J = 5.2 Hz), 9.99 (1H, br s).

### Example 88

### 4-[2-(2,3-difluorophenyl)pyrimidin-4-yl]-N-(3-ethyl-4-methylisoxazol-5-yl)piperazine-1-carboxamide

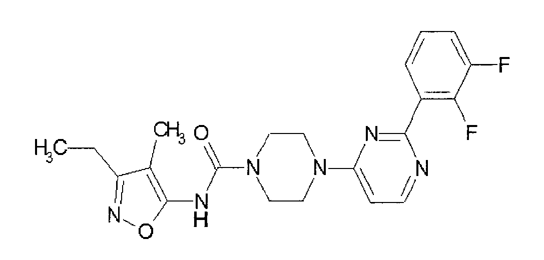

### (1) 2,2,2-trichloroethyl (3-ethyl-4-methylisoxazol-5-yl)carbamate

To a solution of 3-ethyl-4-methyl-5-aminoisoxazol (1.93 g, 9.12 mmol) and pyridine (0.75 g, 9.51 mmol) in acetonitrile (10 ml) under ice-colding was added dropwise ethyl 2,2,2-trichloroformate and the reaction was stirred at room temperature for 1 hour. The reaction was diluted with ethyl acetate, added 0.5N hydrochloride (25 ml), and extracted. The extract was washed with water, dried over anhydrous sodium sulfate and the solvent was distilled off under reduced pressure to obtain the title compound (2.4 g,
100%) as colorless oil.
¹H-NMR (CDCl₃) δ: 1.28 (3H, t, J = 7.5 Hz), 1. 94 (3H, s), 2.62 (2H, q, J = 7.5 Hz), 4.83 (2H, s), 6.83 (1H, br s).

### (2) 4-[2-(2,3-difluorophenyl)pyrimidin-4-yl]-N-(3-ethyl-4-methylisoxazol-5-yl)piperazine-1-carboxamide

To a suspension of 2,2,2-trichloroethyl (3-ethyl-4-methylisoxazol-5-yl)carbamate (0.36 g, 1.20 mmol), 2-(2,3-difluorophenyl)-4-piperazin-1-ylpyrimidine dihydrochloride (0.35 g, 1.00 mmol) was added dropwise triethylamine (0.40 g, 4.00 mmol) at room temperature and the reaction was stirred at 40°C for 2 hours. To the reaction was added dropwise water (4 ml), and the mixture was stirred at room temperature for 1 hour. The resulting crystal was filtered, washed with water to obtain a crude crystal as a solid. The resulting crude crystal was recrystallized from ethyl acetate to obtain the title compound (230 mg, 54%) as a white crystal. Melting point: 188-189°C
¹H-NMR (DMSO-d₆) δ: 1.17 (3H, t, J = 7.5 Hz), 1.78 (3H, s), 2.56 (2H, q, J = 7.5 Hz), 3.55-3.59 (4H, m), 3.73-3.80 (4H, m), 6.89 (1H, d, J = 6.6 Hz), 7.27-7.34 (1H, m), 7.50-7.56 (1H, m), 7.80-7.85 (1H, m), 8.38 (1H, d, J = 6.6 Hz), 9.24 (1H, s).

### Example 89

### 4-[2-(2,3-difluorophenyl)pyrimidin-4-yl]-N-(4-ethyl-3-methylisoxazol-5-yl)piperadine-1-carboxamide

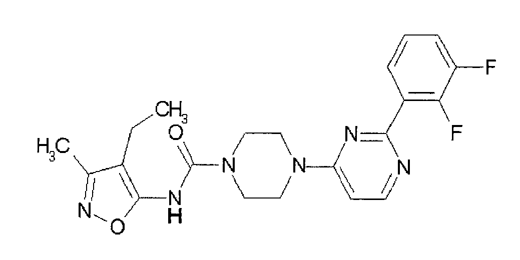

### (1) 2,2,2-trichloroethyl (4-ethyl-3-methylisoxazol-5-yl)carbamate

To a solution of 4-ethyl-3-methyl-5-aminoisoxazol (1.93 g, 9.12 mmol) and pyridine (0.75 g, 9.51 mmol) in acetonitrile (10 ml) under ice-colding was added dropwise ethyl 2,2,2-trichloroformate (1.93 g, 9.12 mmol) and the reaction was stirred at room temperature for 1 hour. The reaction was diluted with ethyl acetate, added 0.5N hydrochloride (25 ml) and extracted. The extract was washed with water, dried over anhydrous sodium sulfate and the solvent was distilled off under reduced pressure to obtain the title compound (2.1 g, 89%) as a pale brown oily material.
¹H-NMR (CDCl₃) δ: 1.14 (3H, t, J = 7.5 Hz), 2.26 (3H, s), 2.39 (2H, q, J = 7.5 Hz), 4.83 (2H, s), 6.85 (1H, br s).

### (2) 4-[2-(2,3-difluorophenyl)pyrimidin-4-yl]-N-(4-ethyl-3-methylisoxazol-5-yl)piperadine-1-carboxamide

The title compound (310 mg, 72%) as a white crystal was prepared from 2,2,2-trichloroethyl (4-ethyl-3-methylisoxazol-5-yl)carbamate and 2-(2,3-difluorophenyl)-4-piperazin-1-ylpyrimidine dihydrochloride in a manner similar to that of Example 88. Melting point: 176-177°C (ethyl acetate).
¹H-NMR (DMSO-d₆) δ: 1.03 (3H, t, J = 7.5 Hz), 2.17 (3H, s), 2.24 (2H, q, J = 7.5 Hz), 3.56-3.59 (4H, m), 3.76 (4H, br s), 6.90 (1H, d, J = 6.3 Hz), 7.28-7.33 (1H, m), 7.94-7.59 (1H, m), 7.80-7.85 (1H, m), 8.38 (1H, d, J = 6.3 Hz), 9.16 (1H, s).

### Example 90

### N-(3-cyclopropyl-4-isoxazol-5-yl)-4-[2-(2,3-difluorophenyl)pyrimidin-4-yl]piperadine-1-carboxamide

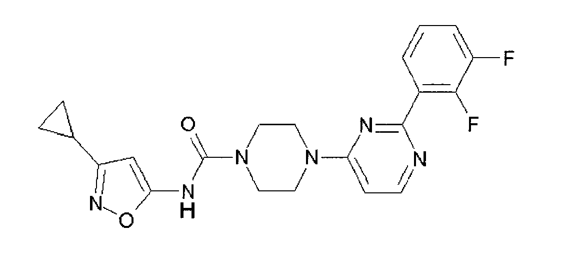

### (1) 2,2,2-trichloroethyl (3-cyclopropylisoxazol-5-yl)carbamate

To a solution of 3-cyclopropyl-5-aminoisoxazole (1.93 g, 9.12 mmol) and pyridine (0.75 g, 9.51 mmol) in acetonitrile (10 ml) under ice-colding was added dropwise ethyl 2,2,2-trichloroformate (1.93 g, 9.12 mmol) and the reaction was stirred at room temperature for 1 hour. The reaction was diluted with ethyl acetate, added 0.5N hydrochloride (25 ml) and extracted. The extract was washed with water, dried over anhydrous sodium sulfate, and the solvent was distilled off to obtain the title compound (1.60 g, 67%) as a yellow solid.
¹H-NMR (CDCl₃) δ: 0.81-0.86 (2H, m), 1.01-1.05 (2H, m), 1.92-2.00 (1H, m), 4.85 (2H, s), 5.84 (1H, s), 7.74 (1H, br s).

### (2) N-(3-cyclopropyl-4-isoxazol-5-yl)-4-[2-(2,3-difluorophenyl)pyrimidin-4-yl]piperadine-1-carboxamide

The desired product (180 mg, 42%) as a white crystal was prepared from 2,2,2-trichloroethyl (3-cyclopropylisoxazol-5-yl)carbamate and 2-(2,3-difluorophenyl)-4-piperazine-1-ylpyrimidine dihydrochloride in a manner similar to that of Example 88. Melting point: 180-181°C (ethyl acetate).
¹H-NMR (DMSO-d₆) δ: 0.69-0.75 (2H, m), 0.94-1.00 (2H, m), 1.86-1.95 (1H, m), 3.61 (4H, br s), 3.81 (4H, br s), 7.03 (1H, d, J = 6.6 Hz), 7.33-7.40 (1H, m), 7.60-7.68 (1H, m), 7.79-7.83 (1H, m), 8.42 (1H, d, J = 6.6 Hz), 10.35 (1H, s).

### Example 91

### 4-[2-(2,3-difluorophenyl)pyrimidin-4-yl]-N-(6-fluoropyridin-3-yl)piperazine-1-carboxamide

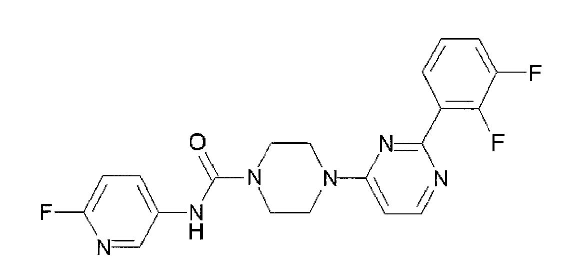

### (1) 2,2,2-trichloroethyl (6-fluoropyridin-3-yl)carbamate

To a solution of 6-fluoropydine-3-amine (1.0 g, 8.92 mmol) and pyridine (0.85 g, 10.7 mmol) in acetnitrile (10 ml) under ice-colding was added dropwise ethyl 2,2,2-trichloroformate (2.18 g, 10.26 mmol) and the reaction was stirred at room temperature for 1 hour. The reaction was diluted with ethyl acetate, added 0.5N hydrochloride (25 ml) and extracted. The extract was wished with water, dried over anhydrous sodium sulfate, and the solvent was distilled off under reduced pressure to obtain the title compound (2.14 g, 83%) as a purple solid.
¹H-NMR (CDCl₃) δ: 4.84 (2H, s), 6.94 (1H, d, J = 3.3 Hz), 6.97 (1H, d, J = 3.3 Hz), 8.05-8.10 (1H, m), 8.17-8.19 (1H, m).

### (2) 4-[2-(2,3-difluorophenyl)pyrimidin-4-yl]-N-(6-fluoropyridin-3-yl)piperazine-1-carboxamide

The title compound (310 mg, 75%) as a white crystal was prepared from 2,2,2-trichloroethyl (6-fluoropyridin-3-yl)carbamate and 2-(2,3-difluorophenyl)-4-piperazin-1-ylpyrimidine dihydrochloride in a manner similar to that of Example 88. Melting point: 192-193°C (ethyl acetate).
¹H-NMR (DMSO-d₆) δ: 3.61-3.65 (4H, m), 3.83 (4H, br s), 6.99 (1H, d, J = 6.0 Hz), 7.08-7.12 (1H, m), 7.31-7.38 (1H, m), 7.56-7.65 (1H, m), 7.80-7.85 (1H, m), 8.03-8.08 (1H, m), 8.30 (1H, s), 8.41 (1H, d, J = 6.0 Hz), 8.94 (1H, s).

### Example 92

### N-(6-chloro-5-isopropylpyridazin-3-yl)-4-[2-(2,3-difluorophenyl)pyrimidin-4-yl]piperazine-1-carboxamide

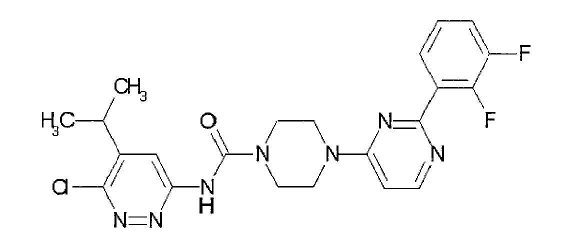

### (1) 2,2,2-trichloroethyl (6-chloro-5-isopropylpyridazin-3-yl)carbamate

To a solution of 6-chloro-5-isopropyl-3-aminopyridazine (1.53 g, 8.92 mmol) and pyridine (0.85 g, 10.7 mmol) in acetonitrile (70 ml) under ice-colding was added dropwise ethyl 2,2,2-trichloroformate (2.18 g, 10.26 mmol) and the reaction was stirred at room temperature for 168 hours. The precipitate was separated by filtration to obtain the title compound (640 mg, 21%) as a white solid.
¹H-NMR (CDCl₃) δ: 1.33 (6H, d, J = 6.9 Hz), 3.22-3.35 (1H, m), 4.87 (2H, s), 8.19 (1H, s), 8.44 (1H, br s).

### (2) N-(6-chloro-5-isopropylpyridazin-3-yl)-4-[2-(2,3-difluorophenyl)pyrimidin-4-yl]piperazine-1-carboxamide

The title compound (320 mg, 67%) as a white crystal was prepared from 2,2,2-trichloroethyl (6-chloro-5-isopropylpyridazin-3-yl)carbamate and 2-(2,3-difluorophenyl)-4-piperazin-1-ylpyrimidine dihydrochloride in a manner similar to that of Example 88. Melting point: 192-193°C (ethyl acetate).
¹H-NMR (DMSO-d₆) δ: 1.23 (6H, d, J = 6.6 Hz), 3.09-3.18 (1H, m), 3.60-3.80 (8H, m), 6.90 (1H, d, J = 6.3 Hz), 7.27-7.34 (1H, m), 7.50-7.59 (1H, m), 7.56-7.65 (1H, m), 7.83 (1H, dd, J = 6.6 Hz, 8.1 Hz), 8.09 (1H, s), 8.38 (1H, d, J = 6.3 Hz), 8.94 (1H, s), 10.15 (1H, s).

### Example 93

### 4-[4-(2-fluorophenyl)pyrimidin-2-yl]-N-pyridin-3-ylpiperazine-1-carboxamide

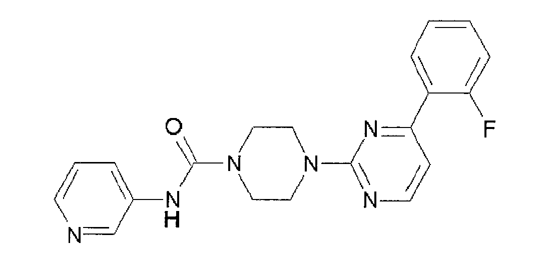

### (1) 4-(2-fluorophenyl)-2-piperazin-1-ylpyrimidine dihydrochloride

To a solution of tert-butyl 4-(4-chloropyrimidin-2-yl)piperazine-1-carboxylate (10 g, 33.5 mmol), 2-fluorophenylboronic acid (7.02 g, 50.2 mmol), and 2N aqueous sodium carbonate solution (45 ml) in 1,2-dimethoxyethane (300 ml) was added tetrakistriphenylphosphine palladium (4.64 g, 40.2 mmol) at room temperature under nitrogen atmosphere, and the mixture was stirred at 95°C overnight. The reaction was poured into the saturated saline, and extracted with ethyl acetate. The extract was dried over anhydrous magnesium sulfate, and the solvent was distilled off under reduced pressure to obtain tert-butyl 4-[4-(2-fluorophenyl)pyrimidin-2-yl]piperazine-1-carboxylate. The obtained tert-butyl 4-[4-(2-fluorophenyl)pyrimidin-2-yl]piperazine-1-carboxylate was dissolved in ethyl acetate (70 ml) and methanol (100 ml), and to the solution was added 4N hydrogen chloride-ethyl acetate solution (42 ml, 167 mmol), stirred at room temperature overnight, and the reaction was distilled off under reduced pressure. To the residue was added ethyl acetate (300 ml) and methanol (60 ml), stirred at room temperature for 2 hours, followed by the crystal was filtered to obtain the title compound (7.90 g, 95%) as a solid.
¹H-NMR (DMSO-d₆) δ: 3.15 - 3.22 (4H, m), 4.02 - 4.08 (4H, m), 7.14 - 7.17 (1H, m), 7.33 - 7.40 (2H, m), 7.55 - 7.61 (1H, m), 8.05 - 8.10 (1H, m), 8.52 - 8.55 (1H, m).

### (2) 4-[4-(2-fluorophenyl)pyrimidin-2-yl]-N-pyridin-3-ylpiperazine-1-carboxamide

The title compound (134 mg, 59%) as a solid was prepared from 2,2,2-trichloroethyl pyridin-3-ylcarbamate and 4-(2-fluorophenyl)-2-piperazin-1-ylpyrimidine dihydrochloride in a manner similar to that of Example 88.
Melting point: 200-201°C (tetrahydrofuran-hexane).
¹H-NMR (DMSO-d₆) 3.52 - 3.68 (4H, m), 3.79 - 3.94 (4H, m), 7.06 - 7.11 (1H, m), 7.22 - 7.44 (3H, m), 7.51 - 7.63 (1H, m), 7.85 - 7.95 (1H, m), 8.02 - 8.12 (1H, m), 8.13 - 8.21 (1H, m), 8.50 (1H, d, J = 5.3 Hz), 8.66 (1H, d, J = 2.3 Hz), 8.81 (1H, s).

### Example 94

### N-(3,4-dimethylisoxazol-5-yl)-4-[4-(2-fluorophenyl)pyrimidin-2-yl]piperazine-1-carboxamide

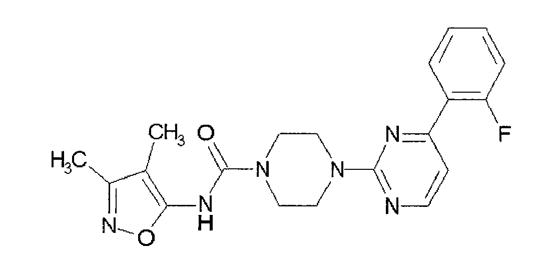

The title compound (185 mg, 77%) as a solid was prepared from 2,2,2-trichloroethyl (3,4-dimethylisoxazol-5-yl)carbamate and 4-(2-fluorophenyl)-2-piperazin-1-ylpyrimidine dihydrochloride in a manner similar to that of Example 88. Melting point: 194-195°C (tetrahydrofuran-hexane).
¹H-NMR (DMSO-d₆) δ: 1.77 (3H, s), 2.13 (3H, s), 3.46 - 3.66 (4H, m), 3.73 - 3.95 (4H, m), 7.06 - 7.11 (1H, m), 7.31 - 7.41 (2H, m), 7.52 - 7.62 (1H, m), 8.03 - 8.11 (1H, m), 8.50 (1H, d, J = 4.9 Hz), 9.25 (1H, s).

### Example 95

### 4-[4-(2-fluorophenyl)pyrimidin-2-yl]-N-pyridazin-3-ylpiperazine-1-carboxamide

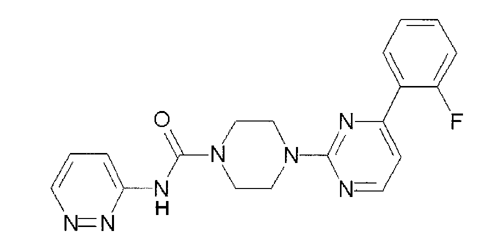

The title compound (183 mg, 80%) was prepared from 2,2,2-trichloroethyl pyridazin-3-ylcarbamate and 4-(2-fluorophenyl)-2-piperazin-1-ylpyrimidine dihydrochloride in a manner similar to that of Example 88. Melting point: 228-229°C (tetrahydrofuran-hexane).
¹H-NMR (DMSO-d₆) δ: 3.54 - 3.72 (4H, m), 3.77 - 3.98 (4H, m), 7.03 - 7.13 (1H, m), 7.29 - 7.43 (2H, m), 7.51 - 7.63 (2H, m), 7.95 - 8.14 (2H, m), 8.50 (1H, d, J = 4.9 Hz), 8.79 - 8.90 (1H, m), 9.96 (1H, s).

### Example 96

### 4-[4-(3-fluorophenyl)pyrimidin-2-yl]-N-pyridin-3-ylpiperazine-1-carboxamide

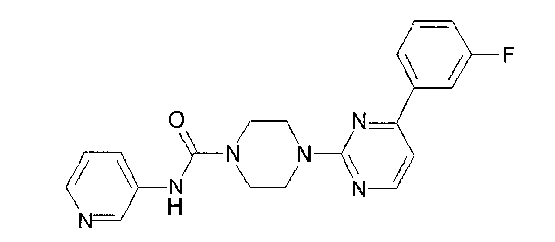

### (1) 4-(3-fluorophenyl)-2-piperazin-1-ylpyrimidine dihydrochloride

The title compound as a solid was prepared from tert-butyl 4-(4-chloropyrimidin-2-yl)piperazine-1-carboxylate and 3-fluorophenylboronic acid in a manner similar to that of Example 93. Yield 97%.
¹H-NMR (DMSO-d₆) δ: 3.17 - 3.23 (4H, m), 4.04 - 4.10 (4H, m), 7.36 - 7.42 (2H, m), 7.54 - 7.61 (1H, m), 7.97 - 8.04 (2H, m), 8.53 - 8.56 (1H, m).

### (2) 4-[4-(3-fluorophenyl)pyrimidin-2-yl]-N-pyridin-3-ylpiperazine-1-carboxamide

The title compound (63.7 mg, 28%) as a solid was prepared from 2,2,2-trichloroethyl pyridin-3-ylcarbamate and 4-(3-fluorophenyl)-2-piperazin-1-ylpyrimidine dihydrochloride in a manner similar to that of Example 88. Melting point: 210-211°C (tetrahydrofuran-hexane).
¹H-NMR (DMSO-d₆) δ: 3.54 - 3.69 (4H, m), 3.83 - 3.97 (4H, m), 7.24 - 7.33 (2H, m), 7.33 - 7.42 (1H, m), 7.52 - 7.63 (1H, m), 7.86 - 7.94 (1H, m), 7.94 - 8.05 (2H, m), 8.13 - 8.19 (1H, m), 8.51 (1H, d, J = 5.3 Hz), 8.67 (1H, d, J = 2.7 Hz), 8.82 (1H, s).

### Example 97

### N-(3,4-dimethylisoxazol-5-yl)-4-[4-(3-fluorophenyl)pyrimidin-2-yl]piperazine-1-carboxamide

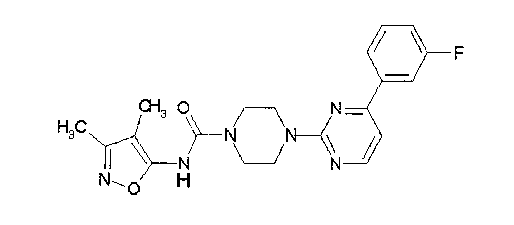

The title compound (144 mg, 60%) as a solid was prepared from 2,2,2-trichloroethyl (3,4-dimethylisoxazol-5-yl)carbamate and 4-(3-fluorophenyl)-2-piperazin-1-ylpyrimidine dihydrochloride in a manner similar to that of Example 88. Melting point: 161-162°C (tetrahydrofuran-hexane).
¹H-NMR (DMSO-d₆) δ: 1.77 (3H, s), 2.13 (3H, s), 3.45 - 3.68 (4H, m), 3.75 - 4.00 (4H, m), 7.31 (1H, d, J = 5.3 Hz), 7.33 - 7.44 (1H, m), 7.51 - 7.65 (1H, m), 7.91 - 8.06 (2H, m), 8.51 (1H, d, J = 5.3 Hz), 9.26 (1H, s).

### Example 98

### 4-[4-(3-fluorophenyl)pyrimidin-2-yl]-N-pyridazin-3-ylpiperazine-1-carboxamide

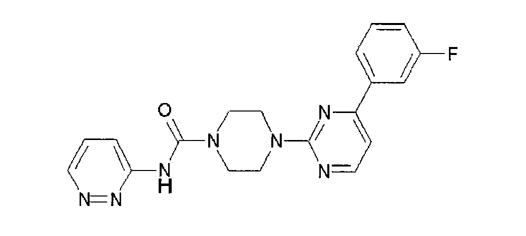

The title compound (183 mg, 80%) as a solid was prepared from 2,2,2-trichloroethyl pyridazin-3-ylcarbamate and 4-(3-fluorophenyl)-2-piperazin-1-ylpyrimidine dihydrochloride in a manner similar to that of Example 88.
Melting point: 238-239°C (tetrahydrofuran-hexane).
¹H-NMR (DMSO-d₆) δ: 3.59 - 3.71 (4H, m), 3.82 - 3.99 (4H, m), 7.30 (1H, d, J = 5.1 Hz), 7.33 - 7.43 (1H, m), 7.51 - 7.64 (2H, m), 7.91 - 8.07 (3H, m), 8.51 (1H, d, J = 5.1 Hz), 8.81 - 8.89 (1H, m), 9.98 (1H, s).

### Example 99

### 4-[4-(4-fluorophenyl)pyrimidin-2-yl]-N-pyridin-3-ylpiperazine-1-carboxamide

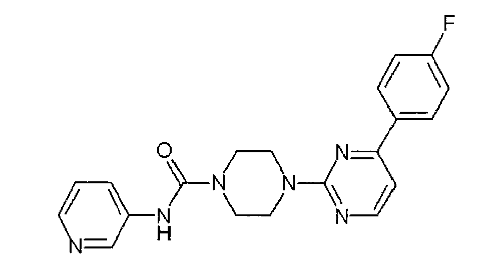

### (1) 4-(4-fluorophenyl)-2-piperazin-1-ylpyrimidine dihydrochloride

The title compound as a solid was prepared from tert-butyl 4-(4-chloropyrimidin-2-yl)piperazine-1-carboxylate and 4-fluorophenylboronic acid in a manner similar to that of Example 93. Yield 92%.
¹H-NMR (DMSO-d₆) δ: 3.14 - 3.23 (4H, m), 4.04 - 4.12 (4H, m), 7.34 - 7.40 (3H, m), 8.21 - 8.28 (2H, m), 8.49 - 8.53 (1H, m).

### (2) 4-[4-(4-fluorophenyl)pyrimidin-2-yl]-N-pyridin-3-ylpiperazine-1-carboxamide

The title compound (142 mg, 62%) as a solid was prepared from 2,2,2-trichloroethyl pyridin-3-ylcarbamate and 4-(4-fluorophenyl)-2-piperazin-1-ylpyrimidine dihydrochloride in a manner similar to that of Example 88.
Melting point: 233-234°C (tetrahydrofuran-hexane).
¹H-NMR (DMSO-d₆) δ: 3.53 - 3.68 (4H, m), 3.82 - 3.97 (4H, m), 7.21 - 7.41 (4H, m), 7.85 - 7.94 (1H, m), 8.16 (1H, dd, J = 4.5, 1.5 Hz), 8.19 - 8.28 (2H, m), 8.48 (1H, d, J = 4.9 Hz), 8.66 (1H, d, J = 2.3 Hz), 8.81 (1H, s).

### Example 100

### N-(3,4-dimethylisoxazol-5-yl)-4-[4-(4-fluorophenyl)pyrimidin-2-yl]piperazine-1-carboxamide

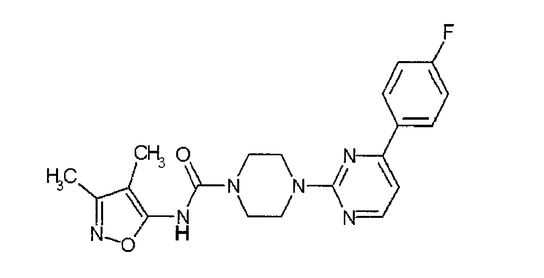

The title compound (171 mg, 71%) as a solid was prepared from 2,2,2-trichloroethyl (3,4-dimethylisoxazol-5-yl)carbamate and 4-(4-fluorophenyl)-2-piperazin-1-ylpyrimidine dihydrochloride in a manner similar to that of Example 88. Melting point: 226-227°C (tetrahydrofuran-hexane).
¹H-NMR (DMSO-d₆) δ: 1.77 (3H, s), 2.13 (3H, s), 3.49 - 3.64 (4H, m), 3.80 - 3.94 (4H, m), 7.26 (1H, d, J = 5.3 Hz), 7.30 - 7.40 (2H, m), 8.17 - 8.27 (2H, m), 8.47 (1H, d, J = 5.3 Hz), 9.25 (1H, s).

### Example 101

### 4-[4-(4-fluorophenyl)pyrimidin-2-yl]-N-pyridazin-3-ylpiperazine-1-carboxamide

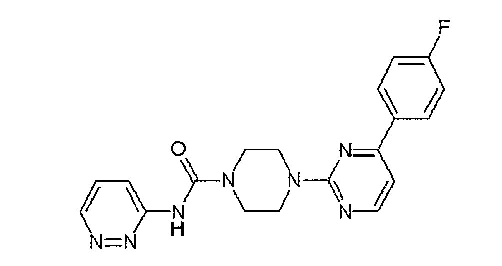

The title compound (176 mg, 77%) as a solid was prepared from 2,2,2-trichloroethyl pyridazin-3-ylcarbamate and 4-(4-fluorophenyl)-2-piperazin-1-ylpyrimidine dihydrochloride in a manner similar to that of Example 88.
Melting point: 168-169°C (tetrahydrofuran-hexane).
¹H-NMR (DMSO-d₆) δ: 3.53 - 3.72 (4H, m), 3.80 - 3.98 (4H, m), 7.25 (1H, d, J = 5.3 Hz), 7.30 - 7.41 (2H, m), 7.58 (1H, dd, J = 9.1, 4.9 Hz), 8.02 (1H, d, J = 9.1 Hz), 8.16 - 8.28 (2H, m), 8.47 (1H, d, J = 5.3 Hz), 8.85 (1H, d, J = 3.4 Hz), 9.96 (1H, s).

### Example 102

### 4-[4-(2,3-difluorophenyl)pyrimidin-2-yl]-N-pyridin-3-ylpiperazine-1-carboxamide

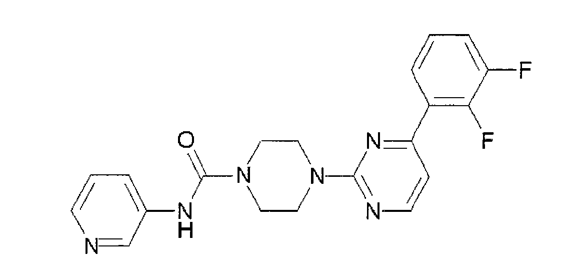

### (1) 4-(2,3-difluorophenyl)-2-piperazin-1-ylpyrimidine dihydrochloride

The title compound as a solid was prepared from tert-butyl 4-(4-chloropyrimidin-2-yl)piperazine-1-carboxylate and 2,3-difluorophenylboronic acid in a manner similar to that of Example 93. Yield 94%.
¹H-NMR (DMSO-d₆) δ: 3.15 - 3.23 (4H, m), 4.01 - 4.08 (4H, m), 7.15 - 7.18 (1H, m), 7.34 - 7.41 (1H, m), 7.58 - 7.65 (1H, m), 7.81 - 7.86 (1H, m), 8.57 - 8.59 (1H, m).

### (2) 4-[4-(2,3-difluorophenyl)pyrimidin-2-yl]-N-pyridin-3-ylpiperazine-1-carboxamide

The title compound (57.1 mg, 25%) as a solid was prepared from 2,2,2-trichloroethyl pyridin-3-ylcarbamate and 4-(2,3-difluorophenyl)-2-piperazin-1-ylpyrimidine dihydrochloride in a manner similar to that of Example 88.
Melting point: 232-233°C (tetrahydrofuran-hexane).
¹H-NMR (DMSO-d₆) δ: 3.48 - 3.69 (4H, m), 3.75 - 3.99 (4H, m), 7.09 (1H, dd, J = 5.1, 2.5 Hz ), 7.28 (1H, dd, J = 8.3, 4.5 Hz), 7.32 - 7.43 (1H, m), 7.54 - 7.66 (1H, m), 7.79 - 7.95 (2H, m), 8.16 (1H, dd, J = 4.5, 1.5 Hz), 8.16 (1H, dd, J = 4.5, 1.5 Hz), 8.54 (1H, d, J = 5.1 Hz), 8.66 (1H, d, J = 2.5 Hz), 8.81 (1H, s).

### Example 103

### N-(3,4-dimethylisoxazol-5-yl)-4-[4-(2,3-difluorophenyl)pyrimidin-2-yl]piperazine-1-carboxamide

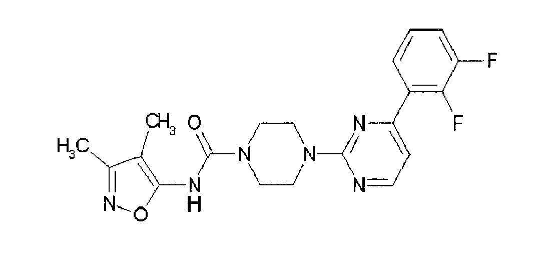

The title compound (137 mg, 58%) as a solid was prepared from 2,2,2-trichloroethyl (3,4-dimethylisoxazol-5-yl)carbamate and 4-(2,3-difluorophenyl)-2-piperazin-1-ylpyrimidine dihydrochloride in a manner similar to that of Example 88. Melting point: 193-194°C (tetrahydrofuran-hexane).
¹H-NMR (DMSO-d₆) δ: 1.76 (3H, s), 2.13 (3H, s), 3.51 - 3.64 (4H, m), 3.78 - 3.92 (4H, m), 7.04 - 7.13 (1H, m), 7.31 - 7.43 (1H, m), 7.53 - 7.67 (1H, m), 7.79 - 7.90 (1H, m), 8.53 (1H, d, J = 4.9 Hz), 9.24 (1H, s).

### Example 104

### 4-[4-(2,3-difluorophenyl)pyrimidin-2-yl]-N-pyridazin-3-ylpiperazine-1-carboxamide

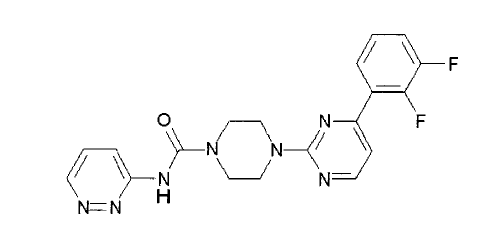

The title compound (206 mg, 91%) as a solid was prepared from 2,2,2-trichloroethyl pyridazin-3-ylcarbamate and 4-(2,3-difluorophenyl)-2-piperazin-1-ylpyrimidine dihydrochloride in a manner similar to that of Example 88.
Melting point: 248-249°C (tetrahydrofuran-hexane).
¹H-NMR (DMSO-d₆) δ: 3.54 - 3.72 (4H, m), 3.77 - 3.98 (4H, m), 7.09 (1H, dd, J = 5.0, 2.5 Hz), 7.32 - 7.43 (1H, m), 7.52 - 7.67 (2H, m), 7.79 - 7.91 (1H, m), 8.02 (1H, dd, J = 9.0, 1.3 Hz), 8.54 (1H, d, J = 5.0 Hz), 8.85 (1H, dd, J = 4.5, 1.3 Hz), 9.97 (1H, s).

### Example 105

### 4-[4-(2,5-difluorophenyl)pyrimidin-2-yl]-N-pyridin-3-ylpiperazine-1-carboxamide

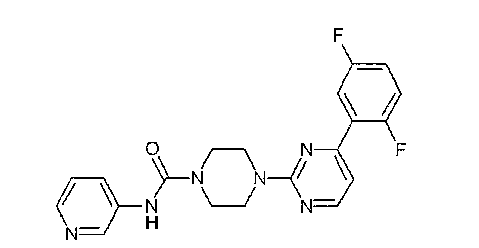

### (1) 4-(2,5-difluorophenyl)-2-piperazin-1-ylpyrimidine dihydrochloride

The title compound as a solid was prepared from tert-butyl 4-(4-chloropyrimidin-2-yl)piperazine-1-carboxylate and 2,5-difluorophenylboronic acid in a manner similar to that of Example 93. Yield 95%.
¹H-NMR (DMSO-d₆) δ: 3.13 - 3.23 (4H, m), 4.01 - 4.08 (4H, m), 7.17 - 7.20 (1H, m), 7.42 - 7.48 (2H, m), 7.87 - 7.93 (1H, m), 7.81 - 7.86 (1H, m), 8.57 - 8.59 (1H, m).

### (2) 4-[4-(2,5-difluorophenyl)pyrimidin-2-yl]-N-pyridin-3-ylpiperazine-1-carboxamide

The title compound (95.5 mg, 42%) as a solid was prepared from 2,2,2-trichloroethyl pyridin-3-ylcarbamate and 4-(2,5-difluorophenyl)-2-piperazin-1-ylpyrimidine dihydrochloride in a manner similar to that of Example 88. Melting point: 270-271°C (tetrahydrofuran-hexane).
¹H-NMR (DMSO-d₆) δ: 3.51 - 3.69 (4H, m), 3.80 - 3.94 (4H, m), 7.11 (1H, dd, J = 4.9, 2.7 Hz), 7.28 (1H, dd, J = 8.3, 4.5 Hz ), 7.38 - 7.49 (2H, m), 7.82 - 7.94 (2H, m), 8.16 (1H, dd, J = 4.5, 1.5 Hz), 8.53 (1H, d, J = 4.9 Hz), 8.67 (1H, d, J = 2.7 Hz), 8.81 (1H, s).

### Example 106

### N-(3,4-dimethylisoxazol-5-yl)-4-[4-(2,5-difluorophenyl)pyrimidin-2-yl]piperazine-1-carboxamide

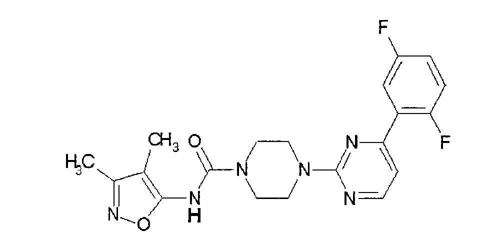

The title compound (204 mg, 86%) as a solid was prepared from 2,2,2-trichloroethyl (3,4-dimethylisoxazol-5-yl)carbamate and 4-(2,5-difluorophenyl)-2-piperazin-1-ylpyrimidine dihydrochloride in a manner similar to that of Example 88. Melting point: 218-219°C (tetrahydrofuran-hexane).
¹H-NMR (DMSO-d₆) δ: 1.77 (3H, s), 2.13 (3H, s), 3.48 - 3.65 (4H, m), 3.77 - 3.93 (4H, m), 7.11 (1H, dd, J = 5.1, 2.5 Hz), 7.37 - 7.49 (2H, m), 7.82 - 7.93 (1H, m), 8.53 (1H, d, J = 5.1 Hz), 9.24 (1H, s).

### Example 107

### 4-[4-(2,5-difluorophenyl)pyrimidin-2-yl]-N-pyridazin-3-ylpiperazine-1-carboxamide

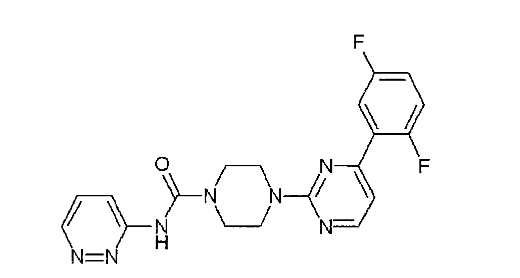

The title compound (101 mg, 44%) as a solid was prepared from 2,2,2-trichloroethyl pyridazin-3-ylcarbamate and 4-(2,5-difluorophenyl)-2-piperazin-1-ylpyrimidine dihydrochloride in a manner similar to that of Example 88.
Melting point: 271-272°C (tetrahydrofuran-hexane).
¹H-NMR (DMSO-d₆) δ: 3.55 - 3.72 (4H, m), 3.79 - 3.96 (4H, m), 7.11 (1H, dd, J = 5.1, 2.4 Hz), 7.38 - 7.50 (2H, m), 7.58 (1H, dd, d, J = 9.0, 4.7 Hz), 7.82 - 7.93 (1H, m), 8.02 (1H, dd, d, J = 9.0, 1.3 Hz), 8.53 (1H, d, J = 5.1 Hz), 8.85 (1H, dd, J = 4.7, 1.3 Hz), 9. 97 (1H, s).

### Example 108

### 4-[4-(3,4-difluorophenyl)pyrimidin-2-yl]-N-pyridin-3-ylpiperazine-1-carboxamide

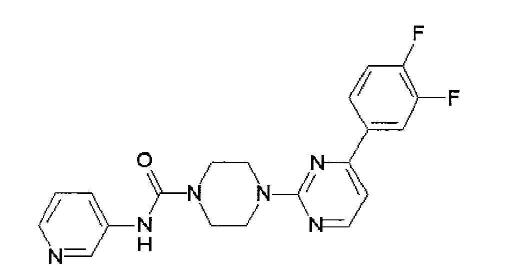

### (1) 4-(3,4-difluorophenyl)-2-piperazin-1-ylpyrimidine dihydrochloride

The title compound as a solid was prepared from tert-butyl 4-(4-chloropyrimidin-2-yl)piperazine-1-carboxylate and 3,4-difluorophenylboronic acid in a manner similar to that of Example 93. Yield 98%.
¹H-NMR (DMSO-d₆) δ: 3.14 - 3.23 (4H, m), 4.03 - 4.11 (4H, m), 7.37 - 7.40 (1H, m), 7.56 - 7.64 (1H, m), 8.04 - 8.10 (1H, m), 8.22 - 8.30 (1H, m), 8.53 - 8.56 (1H, m).

### (2) 4-[4-(3,4-difluorophenyl)pyrimidin-2-yl]-N-pyridin-3-ylpiperazine-1-carboxamide

The title compound (47.8 mg, 21%) as a solid was prepared from 2,2,2-trichloroethyl pyridin-3-ylcarbamate and 4-(3,4-difluorophenyl)-2-piperazin-1-ylpyrimidine dihydrochloride in a manner similar to that of Example 88. Melting point: 231-232°C (tetrahydrofuran-hexane).
¹H-NMR (DMSO-d₆) 5: 3.48 - 3.70 (4H, m), 3.78 - 3.99 (4H, m), 7.23 - 7.33 (2H, m), 7.53 - 7.65 (1H, m), 7.86 - 7.94 (1H, m), 8.01 - 8.11 (1H, m), 8.16 (1H, dd, J = 4.5, 1.5 Hz), 8.19 - 8.29 (1H, m), 8.51 (1H, d, J = 5.3 Hz), 8.66 (1H, d, J = 2.7 Hz), 8.82 (1H, s).

### Example 109

### N-(3,4-dimethylisoxazol-5-yl)-4-[4-(3,4-difluorophenyl)pyrimidin-2-yl]piperazine-1-carboxamide

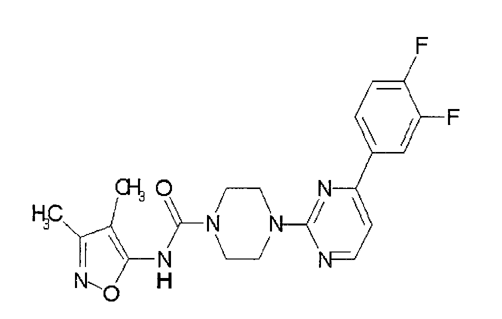

The title compound (162 mg, 68%) as a solid was prepared from 2,2,2-trichloroethyl (3,4-dimethylisoxazol-5-yl)carbamate and 4-(3,4-difluorophenyl)-2-piperazin-1-ylpyrimidine dihydrochloride in a manner similar to that of Example 88. Melting point: 214-215°C (tetrahydrofuran-hexane).
¹H-NMR (DMSO-d₆) 5: 1.77 (3H, s), 2.13 (3H, s), 3.50 - 3.63 (4H, m), 3.80 - 3.96 (4H, m), 7.31 (1H, d, J = 5.3 Hz), 7.52 - 7.66 (1H, m), 8.01 - 8.11 (1H, m), 8.17 - 8.30 (1H, m), 8.50 (1H, d, J = 5.3 Hz), 9.26 (1H, s).

### Example 110

### 4-[4-(3,4-difluorophenyl)pyrimidin-2-yl]-N-pyridazin-3-ylpiperazin-1-carboxamide

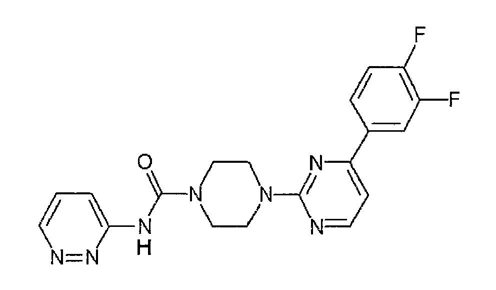

The title compound (188 mg, 83%) as a solid was prepared from 2,2,2-trichloroethyl pyridazin-3-ylcarbamate and 4-(3,4-difluorophenyl)-2-piperazin-1-ylpyrimidine dihydrochloride in a manner similar to that of Example 88. Melting point: 218-219°C (tetrahydrofuran-hexane).
¹H-NMR (DMSO-d₆) δ: 3.56 - 3.73 (4H, m), 3.80 - 3.99 (4H, m), 7.30 (1H, d, J = 5.3 Hz), 7.51 - 7.66 (2H, m), 7.96 - 8.11 (2H, m), 8.50 (1H, d, J = 5.3 Hz), 8.85 (1H, d, J = 3.4 Hz), 9.97 (1H, s).

### Example 111

### 4-[4-(3,5-difluorophenyl)pyrimidin-2-yl]-N-pyridin-3-ylpiperazine-1-carboxamide

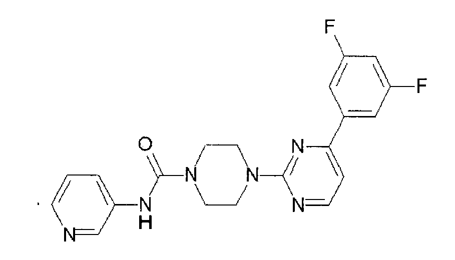

### (1) 4-(3,5-difluorophenyl)-2-piperazin-1-ylpyrimidine dihydrochloride

The title compound as a solid was prepared from tert-butyl 4-(4-chloropyrimidin-2-yl)piperazine-1-carboxylate and 3,5-difluorophenylboronic acid in a manner similar to that of Example 93. Yield 95%.
¹H-NMR (DMSO-d₆) δ: 3.14 - 3.23 (4H, m), 4.04 - 4.10 (4H, m), 7.41 - 7.49 (2H, m), 7.88 - 7.96 (2H, m), 8.55 - 8.59 (1H, m).

### (2) 4-[4-(3,5-difluorophenyl)pyrimidin-2-yl]-N-pyridin-3-ylpiperazine-1-carboxamide

The title compound (146 mg, 64%) as a solid was prepared from 2,2,2-trichloroethyl pyridin-3-ylcarbamate and 4-(3,5-difluorophenyl)-2-piperazin-1-ylpyrimidine dihydrochloride in a manner similar to that of Example 88.
Melting point: 231-232°C (tetrahydrofuran-hexane).
¹H-NMR (DMSO-d₆) δ: 3.51 - 3.70 (4H, m), 3.78 - 4.00 (4H, m), 7.28 (1H, dd, J = 8.3, 4.9 Hz), 7.35 (1H, d, J = 4.9 Hz), 7.38 - 7.48 (1H, m), 7.85 - 7.96 (3H, m), 8.16 (1H, dd, J = 4.9, 1.5 Hz), 8.54 (1H, d, J = 4.9 Hz), 8.66 (1H, d, J = 2.7 Hz), 8.82 (1H, s).

### Example 112

### N-(3,4-dimethylisoxazol-5-yl)-4-[4-(3,5-difluorophenyl)pyrimidin-2-yl]piperazine-1-carboxamide

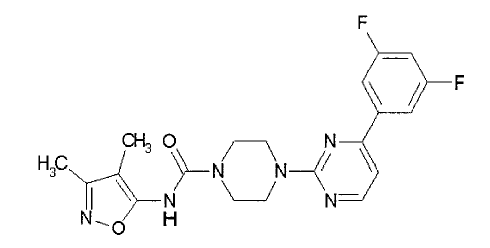

The title compound (188 mg, 79%) as a solid was prepared from 2,2,2-trichloroethyl (3,4-dimethylisoxazol-5-yl)carbamate and 4-(3,5-difluorophenyl)-2-piperazin-1-ylpyrimidine dihydrochloride in a manner similar to that of Example 88. Melting point: 241-242°C (tetrahydrofuran-hexane).
¹H-NMR (DMSO-d₆) δ: 1.77 (3H, s), 2.13 (3H, s), 3.46 - 3.66 (4H, m), 3.76 - 4.00 (4H, m), 7.35 (1H, d, J = 5.3 Hz), 7.38 - 7.49 (1H, m), 7.83 - 7.96 (2H, m), 8.53 (1H, d, J = 5.3 Hz), 9.25 (1H, s).

### Example 113

### 4-[4-(3,5-difluorophenyl)pyrimidin-2-yl]-N-pyridazin-3-ylpiperazine-1-carboxamide

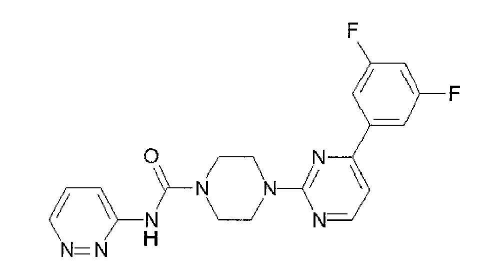

The title compound (89.0 mg, 39%) as a solid was prepared from 2,2,2-trichloroethyl pyridazin-3-ylcarbamate and 4-(3,5-difluorophenyl)-2-piperazin-1-ylpyrimidine dihydrochloride in a manner similar to that of Example 88.
Melting point: 270-271°C (tetrahydrofuran-hexane).
¹H-NMR (DMSO-d₆) δ: 3.53 - 3.75 (4H, m), 3.78 - 4.00 (4H, m), 7.35 (1H, d, J = 5.3 Hz), 7.38 - 7.49 (1H, m), 7.58 (1H, dd, J = 9.1, 4.5 Hz), 7.82 - 7.95 (2H, m), 8.02 (1H, dd, J = 9.1, 1.5 Hz), 8.54 (1H, d, J = 5.3 Hz), 8.85 (1H, d, J = 3.4 Hz), 9.97 (1H, s).

### Example 114

### 4-[4-(2,6-difluorophenyl)pyrimidin-2-yl]-N-pyridin-3-ylpiperazine-1-carboxamide

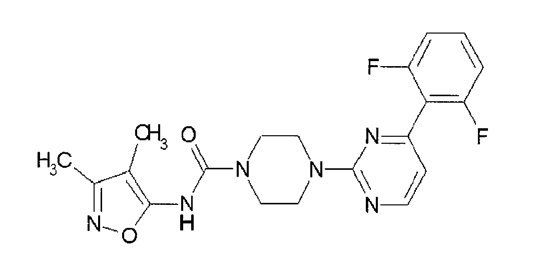

### (1) Tert-butyl 4-[4-(2,6-difluorophenyl)pyrimidin-2-yl]piperazine-1-carboxylate

To a solution of tert-butyl 4-(4-chloropyrimidin-2-yl)piperazine-1-carboxylate (6.00 g, 20.1 mmol), 2,6-difluorophenylboronic acid (3.80 g, 24.1 mmol), potassium fluoride (3.5 g, 60.3 mmol) in tetrahydrofuran-water (10:1)(66 ml) was added trisdibenzylideneacetone dipalladium (1.80 g, 2.01 mmol) and tri-tert-butylphosphine (10 wt% in hexane) (8.4 g, 4.02 mmol) at room temperature under nitrogen atmosphere, and the mixture was stirred at 60°C for 2 hours. The reaction was cooled to room temperature, and concentrated. The residue was poured into water, and extracted with ethyl acetate. The extract was dried over anhydrous magnesium sulfate, and the solvent was distilled off under reduced pressure. The residue was purified by a column chromatography on silica gel (ethyl acetate:hexane = 1:5) to obtain the title compound (6.60 g, 87%) as a solid.
¹H-NMR (CDCl₃) δ: 1.42 (9H, s), 3.32 - 3.42 (4H, m), 3.72 - 3.75 (4H, m), 6.84 - 6.85 (1H, m), 7.22 - 7.26 (2H, m), 7.54 - 7.62 (1H, m), 8.51 (1H, d, J = 4.9 Hz).

### (2) 4-(2,6-difluorophenyl)-2-piperazin-1-ylpyrimidine dihydrochloride

The title compound as a solid was prepared from tert-butyl 4-[4-(2,6-difluorophenyl)pyrimidin-2-yl]piperazine-1-carboxylate in a manner similar to that of Example 93.
Yield 87%.
¹H-NMR (DMSO-d₆) δ: 3.11 - 3.21 (4H, m), 3.98 - 4.02 (4H, m), 6.93 - 6.96 (1H, m), 7.23 - 7.29 (2H, m), 7.55 - 7.63 (1H, m), 8.55 - 8.59 (1H, m).

### (3) 4-[4-(2,6-difluorophenyl)pyrimidin-2-yl]-N-pyridin-3-ylpiperazine-1-carboxamide

The title compound (158 mg, 69%) as a solid was prepared from 2,2,2-trichloroethyl pyridin-3-ylcarbamate and 4-(2,6-difluorophenyl)-2-piperazin-1-ylpyrimidine dihydrochloride in a manner similar to that of Example 88.
Melting point: 185-186°C (tetrahydrofuran-hexane).
¹H-NMR (DMSO-d₆) δ: 3.51 - 3.65 (4H, m), 3.73 - 3.90 (4H, m), 6.86 (1H, d, J = 4.9 Hz), 7.19 - 7.33 (3H, m), 7.51 - 7.66 (1H, m), 7.84 - 7.94 (1H, m), 8.16 (1H, dd, J = 4.5, 1.5 Hz), 8.53 (1H, d, J = 4.9 Hz), 8.65 (1H, d, J = 2.3 Hz), 8.80 (1H, s).

### Example 115

### N-(3,4-dimethylisoxazol-5-yl)-4-[4-(2,6-difluorophenyl)pyrimidin-2-yl]piperazine-1-carboxamide

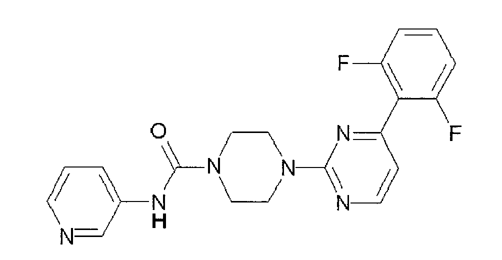

The title compound (166 mg, 67%) as a solid was prepared from 2,2,2-trichloroethyl (3,4-dimethylisoxazol-5-yl)carbamate and 4-(2,6-difluorophenyl)-2-piperazin-1-ylpyrimidine dihydrochloride in a manner similar to that of Example 88. Melting point: 189-190°C (tetrahydrofuran-hexane).
¹H-NMR (DMSO-d₆) δ: 1.76 (3H, s), 2.13 (3H, s), 3.43 - 3.64 (4H, m), 3.71 - 3.89 (4H, m), 6.86 (1H, d, J = 4.9 Hz), 7.18 - 7.32 (2H, m), 7.51 - 7.65 (1H, m), 8.53 (1H, d, J = 4.9 Hz), 9.23 (1H, s).

### Example 116

### 4-[4-(2,6-difluorophenyl)pyrimidin-2-yl]-N-pyridazin-3-ylpiperazine-1-carboxamide

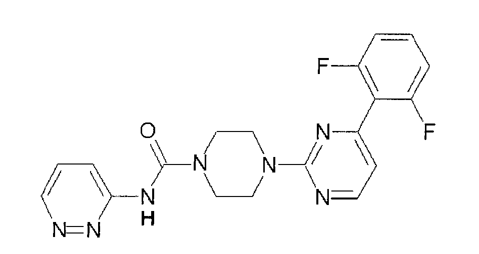

The title compound (105 mg, 46%) as a solid was prepared from 2,2,2-trichloroethyl pyridazin-3-ylcarbamate and 4-(2,6-difluorophenyl)-2-piperazin-1-ylpyrimidine dihydrochloride in a manner similar to that of Example 88.
Melting point: 228-229°C (tetrahydrofuran-hexane).
¹H-NMR (DMSO-d₆) δ: 3.54 - 3.69 (4H, m), 3.74 - 3.90 (4H, m), 6.86 (1H, d, J = 4.9 Hz), 7.18 - 7.32 (2H, m), 7.52 - 7.66 (2H, m), 8.01 (1H, d, J = 9.1 Hz), 8.53 (1H, d, J = 4.9 Hz), 8.84 (1H, d, J = 3.8 Hz), 9.94 (1H, s).

### Formulation Example 1

| | |
|---|---|
| (1) Compound of Example 1 | 10 mg |
| (2) Lactose | 60 mg |
| (3) Corn starch | 35 mg |
| (4) Hydroxypropylmethyl cellulose | 3 mg |
| (5) Magnesium stearate | 2 mg |

A mixture of 10 mg of the compound obtained in Example 1, 60 mg of lactose and 35 mg of corn starch is granulated using 0.03 mL of 10wt% aqueous hydroxypropylmethyl cellulose solution (3 mg in terms of hydroxypropylmethyl cellulose), dried at 40°C and sieved. The resulting granule is mixed with 2 mg of magnesium stearate and compressed. The resulting uncoated tablets are sugar-coated with a suspension of sucrose, titanium dioxide, talc and gum Arabic in water, and the coated tablets are lusetred with beeswax to obtain coated tablets.

### Experimental Example 1

### Measurement of FAAH inhibitory activity

### (1) Preparation of enzyme fraction

The FAAH gene was cloned by PCR. That is, an amplified fragment was obtained by carrying out 45 cycles the reaction carried out at 95°C for 30 sec, at 55°C for 30 sec , and at 72°C for 2 min in one cycle using a human brain library as cDNA library, 5'-AAAAGAATTCGCCACCATGGTGCAGTACGAGCTGTG-3' [SEQ ID NO:1] and 5'-TTTTGTCGACTCAGGATGACTGCTTTT-3' [SEQ ID NO:2] as primer set, and KOD DNA polymerase (Toyobo Co., Ltd.) as DNA polymerase. The amplified fragment was cleaved with restriction enzymes EcoRI and SalI and collected, and then was inserted into pMSRα vector which was cleaved with the restriction enzymes EcoRI and SalI and collected similarily, thereby to obtain pMSRα-human FAAH. A cell line CHO-Kl/human FAAH was prepared, in which human FAAH was stably expressed in the cell line CHO-K1 by a method known per se, using the resulting plasmid. CHO-K1/human FAAH was incubated using a medium (in which fetal bovine serum (FBS) and G418 were added to Ham's F-12 medium to final concentrations of 10% and 800 µg/ml, respectively) in a CO₂ incubator at 37°C and the cells were harvested. Cells were washed with PBS, suspended in a buffer (10 mM Tris, 1 mM EDTA and 10 mM MgCl₂ as each final concentration), and disrupted with a Polytron homogenizer. After centrifugation at 900g, the supernatant was collected and further centrifuged at 10000g. A pellet obtained therefrom was suspended in M-PER (Catalog No. 78501; Pierce Biotechnology, Inc.) to give an enzyme fraction.

### (2) Enzymatic reaction

Using a 96-well plate (Costar Corp.), various concentration of test compounds, the enzymatic fraction (final concentration of 150 ng) and AMC arachidonyl amide substrate (AMCAA: manufactured by CAYMAN CHEMICAL: final concentration of 3 uM) were reacted in a 50uL reaction buffer (125 mM Tris-HCl (pH 9.0), 1 mM EDTA, 0.4 mM HEPES, 0.2% glycerol and 0.02% Triton X-100 as each final concentration) at 37°C for 90 minutes. After the reaction, the fluorescent count of the plate was measured by an ARVO SX 1420 MULTILABEL COUNTER (manufactured by WALLAC) under excitation at 355 nm and emission at 460 nm. The count of a sample containing solvent insted of the test compound was taken as 100%, and the count at zero time was taken as 0%, to calculate the inhibitory activity of the compound.

**Table 1**

| Example numbers that gave compounds whose human FAAH inhibitory ratio (%) was 90% or more at a concentration of 1 µM. |
|---|
| 1-11, 15-33, 34, 37-40, 42-47, 57, 59-61, 68-82, 85, 88-116 |

It can be seen from the results of Table 1 that the compound of the present invention has excellent FAAH inhibitory activity.

### Experimental Example 2

### Analgesic effect in acetic acid writhing test in mice

A suspension of the test compound (10 mg/kg) was orally administered to a mouse. At 60 minutes after the administration, 0.6% aqueous acetic acid solution was intraperitoneally administered in an amount of 10 ml/kg body weight. The mouse was accommodated in a special cage from immediately after the administration until 20 minutes later, and the writhing response was counted. A test of mean difference between two groups (Student's t-test) was performed compared to control group and the analgesic effect was evaluated.

**Table 2**

| Example No. | Writhing count (%) |
|---|---|
| 5 | 42.2 |
| 14 | 67.2 |
| 15 | 66.7 |
| 17 | 56.9 |
| 19 | 51.9 |
| 77 | 46.8 |
| 79 | 38.9 |
| 110 | 34.7 |

It can be seen from the results of Table 2 that the compound of the present invention has excellent analgesic effect.

### Experimental Example 3

### Effect on inflammatory pain (formalin test)

Formalin test was performed according to the method by Dudhgaonkar et al (Eur. J. Pharmacol., 492, 117-122, 2004). Formalin was injected subcutaneously (20µL/site) in the subplantar region of mouse right hind paw, and the effect of a test compound for pseudo-escape response in 5 minutes from immediately after the administration and between 10 minutes and 30 minutes after injection was examined.

### Experimental Example 4

### Effect on inflammatory pain (yeast-induced hyperalgesia)

Randall-Selitto method was performed according to the method by Randall, L.D. and Selitto, J.J (Arch. Int. Pharmacodyn. Ther., 111(4), 409-419, 1957). Yeast was injected subcutaneously in the subplantar region of rat right hind paw and thus induced inflammation, and the effect of a test compound for pain response to a pressure stimulus was examined.

### Experimental Example 5

### Effect on inflammatory pain (Adjuvant arthritis pain method)

Adjuvant arthritis method was performed according to the method by Newbould,B.B. (Br. J Pharmacol. Chemother., 21, 127-136, 1963). Complete Freund's adjuvant was injected in the subplantar region of rat left hind paw and thus induced polyarthritis. After a certain period of time, the abnormal phonation by flexural stimulus in tarsal-tibia joints of opposite hind paw is regarded as a pain response, and the effect of a test compound was examined.

### Experimental Example 6

### Anti-inflammatory effect (carrageenin edema acute inflammation model)

Carrageenin edema test was performed according to the method by Winter et. al (Proc. Soc. Exp. Biol. Med. 111, 544-547, 1962). A carrageenin solution was injected subcutaneously in the footpad site of rat right hind paw and thus induced edema. After the administration of a test compound, the volume of the footpad site of the right hind paw was measured over time and the effect for edema was examined.

### Experimental Example 7

### Effect on inflammatory pain (hyperalgesia by TNFα)

Randall & Selitto modified method was performed according to the method by Randall, L.D. and Selitto, J.J. (Arch. Int. Pharmacodyn. Ther., 111(4), 409-419, 1957). TNFα was injected subcutaneously in the footpad site of rat right hind paw and thus induced inflammation, and the effect of a test compound to pain response for pressure stimulus was examined.

### Effect on neuropathic pain (SNI model)

SNI ( spared nerve injury ) modeling was performed according to the method by Decosterd I. and Woolf C.J.Pain (Pain, 87, 149-58, 2000). After a certain period of time, the effect of a test compound to pain response for tactile stimulus was examined.

### Experimental Example 8

### Effect on neuropathic pain (ZDF model)

Using ZDF (Zucker Diabetic Rat) , the effect of a test compound to pain response for tactile stimulus was examined.

### Experimental Example 9

### Effect on inflammatory pain (hyperalgesia by TNFα)

Randall & Selitto modified method was performed according to the method by Randall, L.D. and Selitto, J.J. (Arch. Int. Pharmacodyn. Ther., 111(4), 409-419, 1957). TNFα was injected subcutaneously in the footpad of rat right hind paw and thus induced inflammation, and the effect of a test compound to pain response for pressure stimulus was examined.

From Experimental Example 3-8, the compound of the present invention indicated excellent analgesic effect on pain. In addition, from Experimental Example 9, the effect on inflammatory pain can be tested.

### Industrial Applicability

According to the present invention, there can be provided a novel fused-ring compound which has a FAAH inhibitory effect and is useful as an analgesic.

## Claims

1. A compound represented by formula (I): 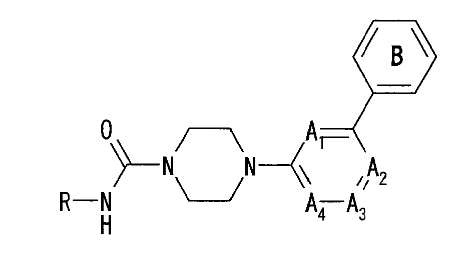 wherein R is an aromatic hydrocarbon or aromatic heterocyclic group each of which may be substituted by one or more substituents (excluding C₁₋₆ alkoxy, phenoxy, carboxyl and tetrazolyl);
A₁, A₂, A₃ and A₄ are each independently CH or N;
ring B is a phenyl group which may be substituted by one or more halogen atoms;
provided that when the moiety represented by formula: 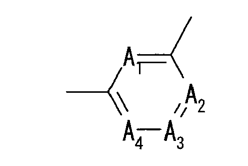 is 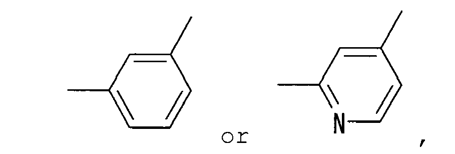 ring B is a phenyl group substituted by one or more halogen atoms, or a salt thereof.

2. The compound according to claim 1, wherein R is an aromatic hydrocarbon or aromatic heterocyclic group which may be substituted by one or more substituents selected from halogen and a C₁₋₆ alkyl group which may be halogenated.

3. The compound according to claim 1, wherein R is a phenyl or 5- to 10-membered aromatic heterocyclic group each of which may be substituted by one or more C₁₋₆ alkyl groups.

4. The compound according to claim 1, wherein the moiety represented by the formula: 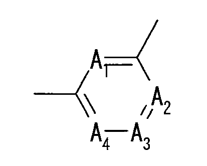 in formula (I) is

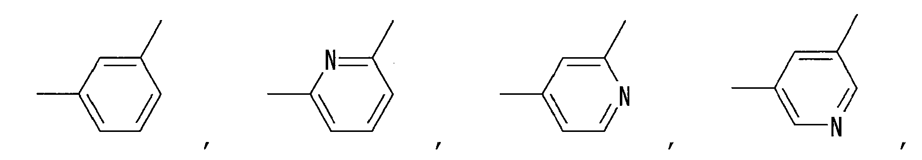

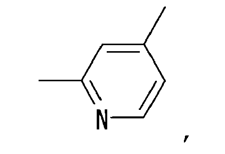

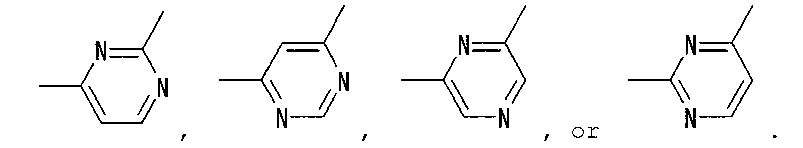

5. The compound according to claim 1, wherein ring B is a phenyl group substituted by one or more halogen atoms.

6. The compound according to claim 1, wherein R is a phenyl or 5- to 10-membered aromatic heterocyclic group each of which may be substituted by one or more C₁₋₆ alkyl groups,
the moiety represented by the formula: 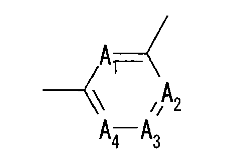 in formula (I) is 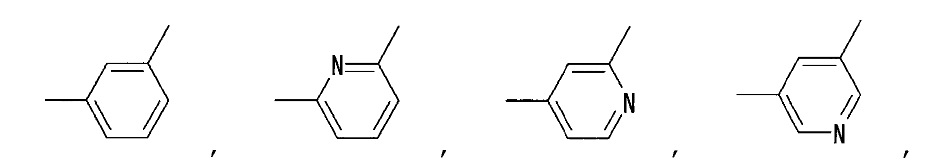 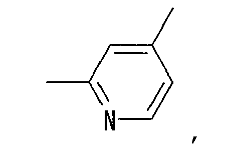 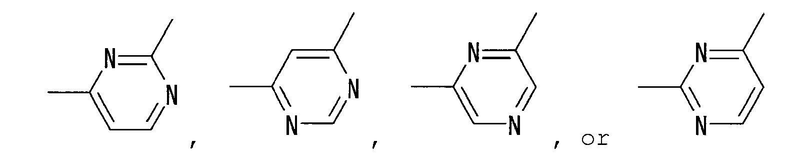 and ring B is a phenyl group substituted by one or more halogen atoms.

7. The compound according to claim 1, wherein R is an isoxazolyl, pyridazinyl, pyridinyl, or phenyl group each of which may be substituted by one or more methyl groups,
the moiety represented by the formula: 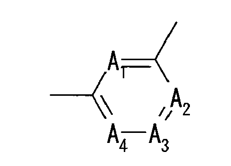 in formula (I) is 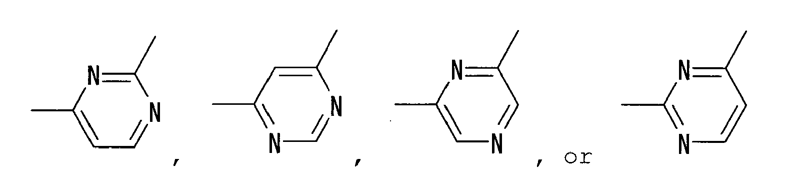 and ring B is a phenyl group substituted by one or more fluorine atoms.

8. The compound according to claim 1, wherein R is an isoxazolyl group which may be substituted by one or more methyl groups,
the moiety represented by the formula: 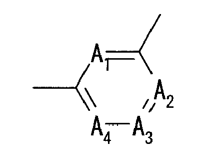 in formula (I) is 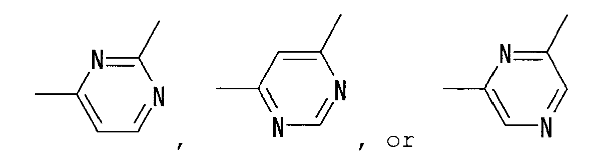 and ring B is a phenyl group substituted by two fluorine atoms.

9. The compound according to claim 1, wherein the moiety represented by the formula: 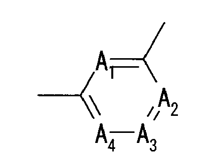 in formula (I) is 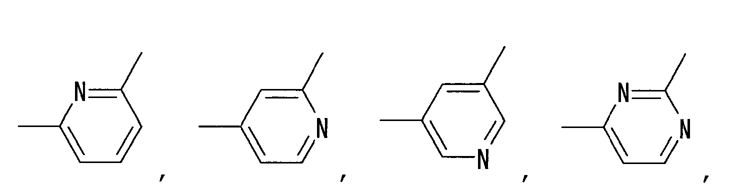 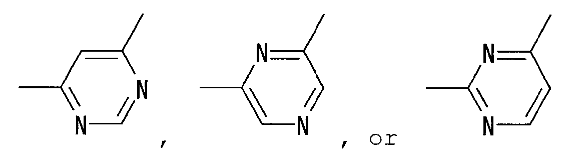 and ring B is a non-substituted phenyl group.

10. The compound according to claim 1, wherein R is a phenyl or 5- to 10-membered aromatic heterocyclic group each of which may be substituted by one or more C₁₋₆ alkyl groups,
the moiey represented by the formula: 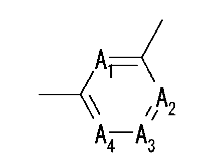 in formula (I) is 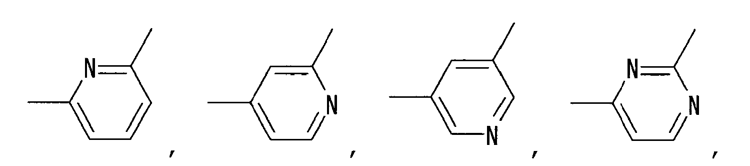 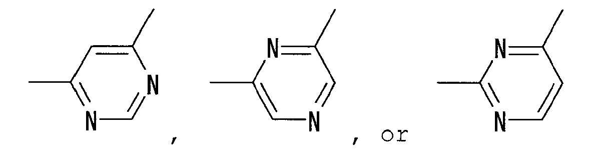 and ring B is a non-substituted phenyl group.

11. The compound according to claim 1, wherein R is an isoxazolyl, pyridazinyl, pyridinyl, or phenyl group each of which may be substituted by one or more methyl groups,
the moiety represented by the formula: 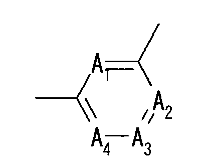 in formula (I) is 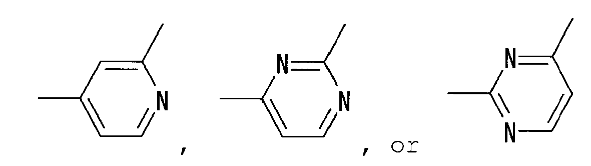 and ring B is a non-substituted phenyl group.

12. The compound according to claim 1, wherein R is a pyridazinyl or pyridinyl group,
the moiety represented by the formula: 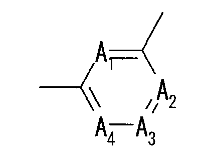 in formula (I) is 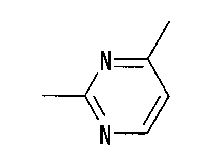 and ring B is a non-substituted phenyl group.

13. 4-[2-(2,3-difluorophenyl)pyrimidin-4-yl]-N-(3,4-dimethylisoxazol-5-yl)piperazine-1-carboxamide or a salt thereof.

14. 4-[6-(2,4-difluorophenyl)pyrazin-2-yl]-N-(3,4-dimethylisoxazol-5-yl)piperazine-1-carboxamide or a salt thereof.

15. 4-[4-(2,4-difluorophenyl)pyrimidin-2-yl]-N-pyridin-3-ylpiperazine-1-carboxamide or a salt thereof.

16. 4-[4-(2,4-difluorophenyl)pyrimidin-2-yl]-N-pyridazin-3-ylpiperazine-1-carboxamide or a salt thereof.

17. 4-[6-(2,4-difluorophenyl)pyrimidin-4-yl]-N-(3,4-dimethylisoxazol-5-yl)piperazine-1-carboxamide or a salt thereof.

18. 4-(4-phenylpyrimidin-2-yl)-N-pyridin-3-ylpiperazine-1-carboxamide or a salt thereof.

19. 4-(4-phenylpyrimidin-2-yl)-N-pyridazin-3-ylpiperazine-1-carboxamide or a salt thereof.

20. A prodrug of the compound according to claim 1.

21. A medicine comprising the compound according to claim 1 or the prodrug according to claim 20.

22. The medicine according to claim 21, which is a FAAH inhibitor.

23. The medicine according to claim 21, which is a prophylatic or therapeutic agent for anxiety or depression, or an analgesic.

24. The medicine according to claim 21, which is a prophylactic or therapeutic agent for inflammatory pain or neuropathic pain.

25. A FAAH inhibitory method **characterized by** administering to a mammal the effective amount of compound according to claim 1, or a prodrug thereof.

26. A method of prophylaxis or treatment for anxiety, or depression, or of pain relief **characterized by** administering to a mammal the effective amount of compound according to claim 1, or a prodrug thereof.

27. A method of prophylaxis or treatment for inflammatory pain or neuropathic pain **characterized by** administering to a mammal the effective amount of compound according to claim 1, or a prodrug thereof.

28. Use of the compound according to claim 1, or a prodrug thereof, for the manufacture of a FAAH inhibitor.

29. Use of the compound according to claim 1, or a prodrug thereof, for the manufacture of a prophylactic or therapeutic agent for anxiety or depression, or an analgesic.

30. Use of the compound according to claim 1, or a prodrug thereof, for the manufacture of a prophylactic or therapeutic agent for inflammatory pain or neuropathic pain.


SEQUENCE LISTING

<110> Takeda Pharmaceutical Company Limited
<120> Amide Compound
<130> 668505
<150> JP 2007-264381
<151> 2007-10-10
<160> 2
<210> 1
<211> 36
<212> DNA
<213> Artificial Sequence
<220>
<223> primer
<400> 1
aaaagaattc gccaccatgg tgcagtacga gctgtg 36

<210> 2
<211> 27
<212> DNA
<213> Artificial Sequence
<220>
<223> primer
<400> 2
ttttgtcgac tcaggatgac tgctttt 27